(19) Europäisches Patentamt
European Patent Office
Office européen des brevets

(11) EP 0 991 624 B1

(12) **EUROPÄISCHE PATENTSCHRIFT**

(45) Veröffentlichungstag und Bekanntmachung des Hinweises auf die Patenterteilung:
**19.11.2003 Patentblatt 2003/47**

(21) Anmeldenummer: **98938621.4**

(22) Anmeldetag: **22.06.1998**

(51) Int Cl.[7]: **C07D 215/48**, A61K 31/395, C07D 215/38, C07D 209/08, C07D 223/16, C07D 401/12, C07D 215/22, C07D 401/06, C07D 215/14, C07D 409/12, C07D 401/04, C07D 215/36, C07D 215/18, C07D 217/06

(86) Internationale Anmeldenummer:
**PCT/EP98/03800**

(87) Internationale Veröffentlichungsnummer:
**WO 99/000371 (07.01.1999 Gazette 1999/01)**

(54) **PHENYLALKYLDERIVATE MIT THROMBINHEMMENDER WIRKUNG**
PHENYLALKYL DERIVATIVES WITH THROMBIN-INHIBITING EFFECT
DERIVES PHENYLALKYLES A EFFET INHIBITEUR DE THROMBINE

(84) Benannte Vertragsstaaten:
**AT BE CH DE DK ES FI FR GB GR IE IT LI LU MC NL PT SE**

(30) Priorität: **26.06.1997 DE 19727117**

(43) Veröffentlichungstag der Anmeldung:
**12.04.2000 Patentblatt 2000/15**

(73) Patentinhaber: **Boehringer Ingelheim Pharma GmbH & Co.KG**
**55218 Ingelheim am Rhein (DE)**

(72) Erfinder:
- **HECKEL, Armin**
  **D-88400 Biberach (DE)**
- **SOYKA, Rainer**
  **D-88400 Biberach (DE)**
- **GRELL, Wolfgang**
  **D-88400 Biberach (DE)**
- **HAAKSMA, Eric**
  **D-88400 Biberach (DE)**
- **BINDER, Klaus**
  **D-65187 Wiesbaden (DE)**
- **ZIMMERMANN, Rainer**
  **D-88441 Mittelbiberach (DE)**

(74) Vertreter: **Laudien, Dieter, Dr.**
**c/o Boehringer Ingelheim GmbH**
**55216 Ingelheim (DE)**

(56) Entgegenhaltungen:
**DE-A- 4 306 506** **US-A- 5 373 019**

- **CHEMICAL ABSTRACTS, vol. 129, no. 5, 3. August 1998 Columbus, Ohio, US; abstract no. 54291w, ITO IYOTAKA ET AL.: "Preparation of carbamoylindolines as 5-hydroxytryptamine antagonists" XP002081134 & JP 10 158241 A (FUJISAWA PHARMACEUTICAL CO.,LTD.)**

EP 0 991 624 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Gegenstand der vorliegenden Erfindung sind Phenylalkylderivate der allgemeinen Formel

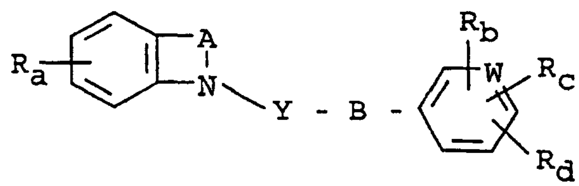

, (I)

deren Tautomere, deren Stereoisomere, deren Gemische und deren Salze, insbesondere deren physiologisch verträgliche Salze mit anorganischen oder organischen Säuren oder Basen, welche wertvolle Eigenschaften aufweisen.

Dokument US 5 373 019 A beschreibt Chinolinon und Indolinonderivate mit erythrozyten - und thrombozytenaggregationshemmender Wirkung. Dokument DE 43 06 506 A offenbart 4-Alkylaminopyridine, die die durch Thrombin induzierte Gerinnung von Fibrinogen im Blut und die durch Thrombin induzierte Aggregation der Blutplättchen hemmen.

Die Verbindungen der obigen allgemeinen Formel I, in denen $R_b$ eine Cyanogruppe darstellt, stellen wertvolle Zwischenprodukte zur Herstellung der übrigen Verbindungen der allgemeinen Formel I dar, und die Verbindungen der obigen allgemeinen Formel I, in denen $R_b$ eine der nachstehend erwähnten gegebenenfalls substituierten Aminomethyl- oder Amidinogruppen darstellt, sowie deren Tautomere und deren Stereoisomere weisen wertvolle pharmakologische Eigenschaften auf, insbesondere eine thrombinhemmende Wirkung, die Thrombinzeit verlängernde Wirkung und eine thrombozytenaggregationshemmende Wirkung.

Gegenstand der vorliegenden Anmeldung sind somit die Verbindungen der obigen allgemeinen Formel I sowie deren Herstellung, die die pharmakologisch wirksamen Verbindungen enthaltende Arzneimittel und deren Verwendung.

In der obigen allgemeinen Formel I bedeuten

$R_a$ ein Wasserstoffatom, eine Carboxy-, $C_{1-3}$-Alkoxycarbonyl-, Benzoyl-, Phenylsulfonyl-, Nitro-, $R_1NR_2$-, $R_1NR_2$-X- oder $(R_3X)NR_1$- Gruppe, in denen

$R_1$ ein Wasserstoffatom, eine $C_{1-5}$-Alkylgruppe, welche durch eine Phenyl-, Carboxy-, $C_{1-4}$-Alkoxycarbonyl- oder Aminocarbonylgruppe substituiert sein kann, wobei die Aminogruppe der Aminocarbonylgruppe zusätzlich durch $C_{1-4}$-Alkyl-, Phenyl-$C_{1-3}$-alkyl-, Phenyl-, Carboxy-$C_{1-3}$-alkyl- oder $C_{1-3}$-Alkoxycarbonyl-$C_{1-3}$-alkylgruppen mono- oder disubstituiert und die Substituenten gleich oder verschieden sein können, oder eine geradkettige $C_{2-3}$-Alkylgruppe, die endständig durch Amino-, $C_{1-3}$-Alkylamino-, Di-($C_{1-3}$-Alkyl)amino-, $C_{1-4}$-Alkanoylamino-, Phenylamino-, N-Benzyloxycarbonylphenylamino-, Pyrrolidino-, Piperidino- oder Morpholinogruppe substituiert ist,

$R_2$ ein Wasserstoffatom, eine gegebenenfalls durch eine oder zwei Phenylgruppen oder durch eine Naphthylgruppe substituierte $C_{1-3}$-Alkylgruppe oder eine Phenylgruppe, die durch ein Fluor-, Chlor- oder Bromatom oder durch eine geradkettige $C_{2-3}$-Alkylgruppe, die endständig durch eine Amino-, $C_{1-3}$-Alkylamino-, $C_{1-3}$-Alkanoylamino-, Di-($C_{1-3}$-Alkyl)amino-, Pyrrolidino-, Piperidino- oder Morpholinogruppe substituiert ist, substituiert sein kann,

$R_1$ und $R_2$ zusammen mit dem dazwischen liegenden Stickstoffatom eine gegebenenfalls durch eine $C_{1-3}$-Alkyl-, Carboxyoder $C_{1-3}$-Alkoxycarbonylgruppe substituierte Pyrrolidinooder Piperidinogruppe, eine durch zwei $C_{1-3}$-Alkylgruppen substituierte Pyrrolidino- oder Piperidinogruppe oder eine Morpholinogruppe,

$R_3$ eine geradkettige oder verzweigte $C_{1-7}$-Alkylgruppe, die in 1-, 2- oder 3-Stellung durch eine Phenylgruppe oder in 2-bis 7-Stellung durch ein Fluor-, Chlor- oder Bromatom, durch eine Carboxy- oder $C_{1-3}$-Alkoxycarbonylgruppe substituiert sein kann, eine Trifluormethylgruppe, eine Phenyl-, Naphthyl- oder Chromanylgruppe, die jeweils durch ein Fluor-, Chlor- oder Bromatom, durch eine Trifluormethyl-, $C_{1-3}$-Alkyl-, $C_{1-3}$-Alkoxy-, Amino-, $C_{1-3}$-Alkylamino-, Di-($C_{1-3}$-alkyl)amino- oder Aminocarbonylgruppe substituiert sein können, wobei die vorstehend erwähnten Phenyl-, Naphthyl- oder Chromanylgruppen zusätzlich durch ein bis drei Methylgruppen substituiert sein können, eine durch zwei Chlor- oder Bromatome substituierte Phenyl- oder Aminophenylgruppe, eine gegebenenfalls durch ein Chlor- oder Bromatom oder durch eine Methylgruppe substituierte Thienylgruppe, eine $C_{3-8}$-Cycloalkyl-, $C_{8-12}$-Bicycloalkanon-, Chinolyl-, Isochinolyl- oder Benzimidazolylgruppe oder

$R_1$ und $R_3$ zusammen eine n-Alkylengruppe mit 3 bis 5 Kohlenstoffatomen, in der eine mit der $SO_2$- oder CO-Gruppe verknüpfte Ethylengruppe durch eine 1,2-Phenylengruppe ersetzt sein kann, und

X eine Carbonyl- oder Sulfonylgruppe darstellen,

oder $R_a$ auch eine $C_{2-3}$-Alkanoylgruppe, die im Alkylteil durch eine Carboxy-$C_{1-3}$-alkyl- oder $C_{1-3}$-Alkoxycarbonyl-$C_{1-3}$-alkylgruppe und eine Benzoyl-, Naphthoyl-, Phenylsulfonyl- oder Naphthylsulfonylgruppe substituiert ist,

$R_b$ eine gegebenenfalls durch eine $C_{1-10}$-Alkoxycarbonyl- oder Phenyl-$C_{1-3}$-alkoxycarbonylgruppe substituierte Ami-

dinogruppe, eine Cyano- oder Aminomethylgruppe,
$R_c$ und $R_d$, die gleich oder verschieden sein können, jeweils ein Wasserstoff-, Fluor-, Chlor-, Brom- oder Jodatom, eine Methyl-, Methoxy-, Nitro-, Amino- oder Aminocarbonylgruppe oder eine gegebenenfalls durch eine geradkettige $C_{2-4}$-Alkanoylgruppe substituierte Aminogruppe, in der der Alkanoylteil endständig durch eine Carboxy- oder $C_{1-3}$-Alkoxycarbonylgruppe substituiert sein kann,
A eine gegebenenfalls durch eine oder zwei $C_{1-3}$-Alkylgruppen substituierte Ethylen-, Ethenylen-, n-Propylen- oder n-Butylengruppe, wobei eine Methylengruppe einer gegebenenfalls durch eine oder zwei $C_{1-3}$-Alkylgruppen substituierten Ethylenoder n-Propylengruppe, die

(i) mit dem Stickstofftom verknüpft ist, durch eine Carbonylgruppe, oder

(ii) mit dem Phenylkern verknüpft ist, durch ein Sauerstoffoder Schwefelatom, durch eine Sulfinyl- oder Sulfonylgruppe oder durch eine gegebenenfalls durch eine $C_{1-3}$-Alkylgruppe substituierte Iminogruppe ersetzt sein kann,

B eine Bindung, eine gegebenenfalls durch eine oder zwei $C_{1-3}$-Alkylgruppen substituierte Methylen-, Ethylen-, Ethenylen- oder n-Propylengruppe, wobei

(iii) in den vorstehend erwähnten Methylen-, Ethylen- oder n-Propylengruppen eine Methylengruppe, wenn Y eine Carbonyloder Thiocarbonylgruppe darstellt, durch ein Sauerstoffatom oder durch eine gegebenenfalls durch eine $C_{1-3}$-Alkylgruppe substituierte Iminogruppe oder

(iv) in den vorstehend erwähnten Ethylen- oder n-Propylengruppen, wenn Y eine Methylengruppe darstellt, eine in 3-oder 4-Stellung befindliche Methylengruppe bezogen auf das Stickstoffatom durch ein Sauerstoffatom oder durch eine gegebenenfalls durch eine $C_{1-3}$-Alkylgruppe substituierte Iminogruppe ersetzt sein kann,

W eine Methingruppe oder ein Stickstoffatom und
Y eine Methylen-, Carbonyl- oder Thiocarbonylgruppe.

Bevorzugte Verbindungen der obigen allgemeinen Formel I sind jedoch diejenigen, in denen
$R_a$, $R_c$, $R_d$, A, B, W und Y wie vorstehend erwähnt definiert sind und
$R_b$ eine gegebenenfalls durch eine $C_{1-10}$-Alkoxycarbonyl- oder Phenyl-$C_{1-3}$-alkoxycarbonylgruppe substituierte Amidinogruppe darstellt,
deren optische Antipoden und deren Salze.

Besonders bevorzugte Verbindungen der obigen allgemeinen Formel I sind jedoch diejenigen, in denen
$R_a$ eine $R_1NR_2$-, $R_1'NR_2'$-X- oder $(R_3X)NR_1$-Gruppe, in denen Gruppe, in denen

$R_1$ ein Wasserstoffatom, eine $C_{1-4}$-Alkylgruppe, welche durch eine Phenyl-, Carboxy-, $C_{1-2}$-Alkoxycarbonyl- oder Aminocarbonylgruppe substituiert sein kann, wobei die Aminogruppe der Aminocarbonylgruppe zusätzlich durch $C_{1-4}$-Alkyl-, Phenyl-, Benzyl-, Carboxy-$C_{1-2}$-alkyl- oder $C_{1-2}$-Alkoxycarbonyl-$C_{1-2}$-alkylgruppen mono- oder disubstituiert und die Substituenten gleich oder verschieden sein können, oder eine Ethylgruppe, die endständig durch Amino-, Acetylamino-, Morpholino-, Phenylamino- oder N-Benzyloxycarbonyl-phenylaminogruppe substituiert ist,

$R_2$ ein Wasserstoffatom, eine gegebenenfalls durch eine oder zwei Phenylgruppen oder durch eine Naphthylgruppe substituierte $C_{1-3}$-Alkylgruppe, eine Cyclohexylgruppe, eine gegebenenfalls durch ein Chloratom, durch eine 2-Aminoethyloder 2-Acetylaminogruppe substituierte Phenylgruppe,

$R_1'$ und $R_2'$ die für $R_1$ und $R_2$ vorstehend erwähnten Bedeutungen besitzen oder zusammen mit dem dazwischen liegenden Stickstoffatom eine gegebenenfalls durch eine Methyl-, Carboxy- oder $C_{1-2}$-Alkoxycarbonylgruppe substituierte Pyrrolidino- oder Piperidinogruppe, eine durch zwei Methylgruppen substituierte Pyrrolidino- oder Piperidinogruppe oder eine Morpholinogruppe,

$R_3$ eine geradkettige oder verzweigte $C_{1-5}$-Alkylgruppe, die in 1-, 2- oder 3-Stellung durch eine Phenyl-, Carboxy- oder $C_{1-3}$-Alkoxycarbonylgruppe oder in 2- oder 3-Stellung durch ein Chloratom substituiert sein kann, eine Trifluormethylgruppe, eine Phenyl- oder Naphthylgruppe, die jeweils durch ein Fluor-, Chlor- oder Bromatom, durch eine Trifluormethyl-, $C_{1-3}$-Alkyl-, $C_{1-3}$-Alkoxy-, Amino-, $C_{1-3}$-Alkylamino-, Di-($C_{1-3}$-alkyl)amino- oder Aminocarbonylgruppe substituiert sein können, wobei die vorstehend erwähnten Phenylgruppen zusätzlich durch ein bis drei Methylgruppen substituiert sein können, eine durch zwei Chlor- oder Bromatome substituierte Phenyl- oder Aminophenylgruppe, eine durch ein Chlor- oder Bromatom substituierte Thienylgruppe, eine $C_{3-7}$-Cycloalkyl-, Chinolyl-, Isochinolyl- oder Benzimidazolylgruppe oder

$R_1$ und $R_3$ zusammen eine n-Alkylengruppe mit 3 bis 5 Kohlenstoffatomen, in der eine mit der $SO_2$- oder CO-Gruppe verknüpfte Ethylengruppe durch eine 1,2-Phenylengruppe ersetzt sein kann, und

X eine Carbonyl- oder Sulfonylgruppe darstellen,

oder $R_a$ auch eine $C_{2-3}$-Alkanoylgruppe, die durch eine Carboxy-$C_{1-3}$-alkyl- oder $C_{1-3}$-Alkoxycarbonyl-$C_{1-3}$-alkylgruppe

und eine Benzoyl-, Naphthoyl-, Phenylsulfonyl- oder Naphthylsulfonylgruppe substituiert ist,
$R_b$ eine gegebenenfalls durch eine $C_{1-10}$-Alkoxycarbonyl- oder Phenyl-$C_{1-3}$-alkoxycarbonylgruppe substituierte Amidinogruppe,
$R_c$ ein Wasserstoff-, Fluor-, Chlor-, Brom- oder Jodatom, eine Methyl-, Methoxy-, Aminocarbonyl-, Amino- oder Nitrogruppe oder eine gegebenenfalls durch eine geradkettige $C_{2-4}$-Alkanoylgruppe substituierte Aminogruppe, in der der Alkanoylteil endständig durch eine Carboxy- oder $C_{1-3}$-Alkoxycarbonylgruppe substituiert sein kann,
$R_d$ ein Wasserstoffatom,
A eine gegebenenfalls durch eine oder zwei Methylgruppen substituierte Ethylen-, n-Propylen- oder n-Butylengruppe, wobei eine Methylengruppe einer gegebenenfalls durch eine oder zwei Methylgruppen substituierten Ethylen- oder n-Propylengruppe, die

(i) mit dem Stickstofftom verknüpft ist, durch eine Carbonylgruppe ersetzt sein kann,

B eine Bindung, eine gegebenenfalls durch eine oder zwei Methylgruppen substituierte Methylen-, Ethylen-, Ethenylen- oder n-Propylengruppe, wobei

(iii) in den vorstehend erwähnten Methylen-, Ethylen- oder n-Propylengruppen eine Methylengruppe, wenn Y eine Carbonyloder Thiocarbonylgruppe darstellt, durch ein Sauerstoffatom oder durch eine gegebenenfalls durch eine Methylgruppe substituierte Iminogruppe oder

(iv) in den vorstehend erwähnten Ethylen- oder n-Propylengruppen, wenn Y eine Methylengruppe darstellt, eine in 3-oder 4-Stellung befindliche Methylengruppe bezogen auf das Stickstoffatom durch ein Sauerstoffatom oder durch eine gegebenenfalls durch eine Methylgruppe substituierte Iminogruppe ersetzt sein kann,

W eine Methingruppe und
Y eine Methylen-, Carbonyl- oder Thiocarbonylgruppe bedeuten,
insbesondere die vorstehend erwähnten Verbindungen, in denen
$R_a$ eine $(R_3SO_2)NR_1$-Gruppe darstellt,
deren optische Antipoden und deren Salze.

Ganz besonders bevorzugte Verbindungen sind diejenigen, in denen
$R_a$ eine $(R_3SO_2)NR_1$-Gruppe, wobei $R_1$ und $R_3$ wie vorstehend erwähnt definiert sind,
$R_b$ eine gegebenenfalls durch eine $C_{1-10}$-Alkoxycarbonyl- oder Phenyl-$C_{1-3}$-alkoxycarbonylgruppe substituierte Amidinogruppe,
$R_c$ und $R_d$ jeweils ein Wasserstoffatom,
A eine gegebenenfalls durch eine Methylgruppe substituierte n-Propylengruppe,
B eine Ethylengruppe,
W eine Methingruppe und
Y eine Carbonylgruppe bedeuten,
deren optische Antipoden und deren Salze.

Beispielsweise seien folgende besonders bevorzugte Verbindungen erwähnt:

(a) 1- [3- (4-Amidino-phenyl)propionyl] -6- (4-fluor-phenylsulfonamido)-1,2,3,4-tetrahydro-chinolin,

(b) 1-[3-(4-Amidino-phenyl)propionyl]-6-butylsulfonamido-1,2,3,4-tetrahydro-chinolin,

(c) 1-[3-(4-Amidino-phenyl)propionyl]-5-phenylsulfonamido-1,2,3,4-tetrahydro-chinolin,

(d) 1- [3- (4-Amidino-phenyl)propionyl] -3-methyl-6-phenylsulfonamido-1,2,3,4-tetrahydro-chinolin,

(e) 1-[3-(4-Amidino-phenyl)propionyl]-6-(5-chlor-thien-2-ylsulfonamido)-1,2,3,4-tetrahydro-chinolin,

(f) 1-[3-(4-Amidino-phenyl)propionyl]-6-phenylsulfonamido-1,2,3,4-tetrahydro-chinolin,

(g) 1-[3-(4-Amidino-phenyl)propionyl]-6-(N-methyl-phenylsulfonamido)-1,2,3,4-tetrahydro-chinolin,

(h) 1-[3-(4-Amidino-phenyl)propionyl]-6-(N-ethoxycarbonylmethyl-phenylsulfonamido)-1,2,3,4-tetrahydro-chinolin,

(i) 1-[3-(4-Amidino-phenyl)propionyl]-6-(N-carboxymethyl-phenylsulfonamido)-1,2,3,4-tetrahydro-chinolin,

(j) 1-[3-(4-Aminomethyl-phenyl)propionyl]-6-phenylsulfonamido-1,2,3,4-tetrahydro-chinolin,

(k) 1-[3-(4-Amidino-phenyl)propyl]-6-phenylsulfonamido-1,2,3,4-tetrahydro-chinolin,

(1) 1-[3-(4-Methyloxycarbonyl-amidino-phenyl)propionyl]-6-phenylsulfonamido-1,2,3,4-tetrahydro-chinolin,

(m) 1-[3-(4-Amidino-phenyl)-propionyl]-6-(N-phenyl-methylaminocarbony)-1,2,3,4-tetrahydro-chinolin,

(n) 1-[(4-Amidino-phenoxy)-acetyl]-6-[N-(1-naphthylsulfonyl)-hydroxycarbonylmethylamino]-1,2,3,4-tetrahydro-chinolin,

(o) 1-[3-(4-Amidino-phenyl)-propionyl]-6-diethylaminocarbonyl-1,2,3,4-tetrahydro-chinolin,

(p) 1- [3- (4-Amidino-phenyl) -propionyl] -6- (N-benzoyl-methylamino)-1,2,3,4-tetrahydro-chinolin,

(q) 1-[3-(4-Amidino-phenyl)-propionyl]-6-(N-benzoyl-methylamino)-1,2,3,4-tetrahydro-chinolin,

(r) 1-[3-(4-Amidino-phenyl)-propionyl]-6-[N-(naphth-1-yl-sulfonyl)-hydroxycarbonylmethylamino]-1,2,3,4-tetrahydro-chinolin,

(s) 1- [3- (4-Amidino-phenyl) -propionyl] -6- [N- (1-naphthoyl) -hydroxycarbonylmethylamino]-1,2,3,4-tetrahydro-chinolin,

(t) 1-[3-(4-Amidino-phenyl)-propionyl]-6-(N-benzoyl-hydroxycarbonylmethylamino]-1,2,3,4-tetrahydro-chinolin,

(u) 1- [3- (4-Amidino-phenyl) -propionyl] -6- [N- (chinolin-8-sulfonyl)-hydroxycarbonylmethylamino]-1,2,3,4-tetrahydro-chinolin und

(v) 1-[3-(4-Amidino-phenyl)-propionyl]-6-[N-(n-butylsulfonyl)-hydroxycarbonylmethylamino]-1,2,3,4-tetrahydro-chinolin,

deren optische Antipoden und deren Salze.

Erfindungsgemäß erhält man die neuen Verbindungen der allgemeinen Formel I beispielsweise nach folgenden Verfahren:

a) Zur Herstellung einer Verbindung der allgemeinen Formel I, in der $R_b$ eine Cyanogruppe und Y eine Methylengruppe darstellen:

Umsetzung einer Verbindung der allgemeinen Formel

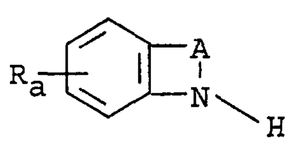

, (II)

in der

A und $R_a$ wie eingangs definiert sind, mit einer Verbindung der allgemeinen Formel

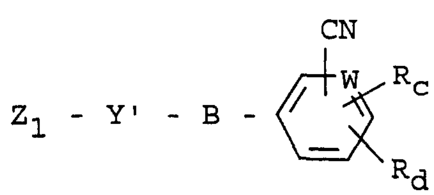

, (III)

in der
B, W, $R_c$ und $R_d$ wie eingangs definiert sind,
Y' eine Methylengruppe und
$Z_1$ eine Austrittsgruppe wie ein Halogenatom, einen Sulfonsäurerest, z.B. ein Chlor-, Brom- oder Jodatom, eine Methansulfonyloxy- oder p-Toluolsulfonyloxygruppe darstellen.

Die Umsetzung wird zweckmäßigerweise in einem Lösungsmittel oder Lösungsmittelgemisch wie Methylenchlorid, Chloroform, Ether, Tetrahydrofuran, Dioxan oder Dimethylformamid gegebenenfalls in Gegenwart einer anorganischen oder organischen Base, wie Natriumhydroxid, Kaliumcarbonat, Triethylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

b) Zur Herstellung einer Verbindung der allgemeinen Formel I, in der $R_b$ eine Cyanogruppe und Y eine Carbonylgruppe darstellen:

Umsetzung einer Verbindung der allgemeinen Formel

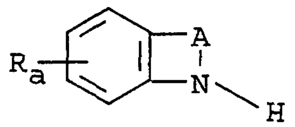

, (II)

in der
A und $R_a$ wie eingangs definiert sind, mit einer Verbindung der allgemeinen Formel

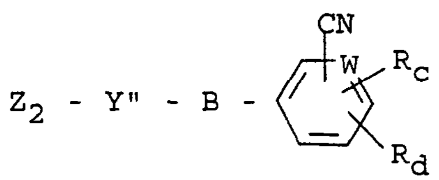

, (IV)

in der
B, W, $R_c$ und $R_d$ wie eingangs definiert sind,
Y" eine Carbonylgruppe und
$Z_2$ eine Hydroxygruppe oder eine Austrittsgruppe wie ein Halogenatom, z. B. ein Chlor- oder Bromatom, darstellen.

Die Umsetzung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, und gegebenenfalls in Gegenwart einer anorganischen Base wie Natriumcarbonat oder einer organischen Base wie Triethylamin oder Pyridin, welche gleichzeitig als Lösungsmittel dienen können, bei Temperaturen zwischen -25°C und 250°C, vorzugsweise jedoch bei Temperaturen zwischen -10°C und der Siedetemperatur des verwendeten Lösungsmittels, durchgeführt.

c) Zur Herstellung einer Verbindung der allgemeinen Formel I, in der $R_a$ eine $R_1N(XR_3)$-Gruppe und $R_b$ eine Cyanogruppe darstellen:

Umsetzung einer Verbindung der allgemeinen Formel

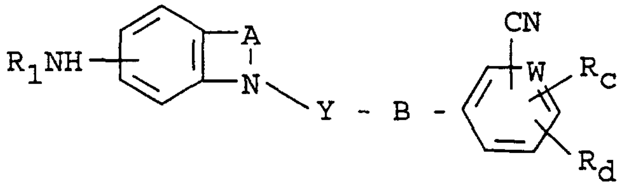

, (V)

in der
A, B, W, Y, $R_c$, $R_d$ und $R_1$ wie eingangs definiert sind, mit einer Verbindung der allgemeinen Formel

$$Z_3\text{-}X\text{-}R_3 \qquad ,(VI)$$

in der
X und $R_3$ wie eingangs definiert sind und
$Z_3$ eine Hydroxygruppe oder eine Austrittsgruppe wie ein Halogenatom, z. B. ein Chlor- oder Bromatom, darstellt.

Die Umsetzung wird zweckmäßigerweise in einem Lösungsmittel oder Lösungsmittelgemisch wie Wasser, Methylenchlorid, Chloroform, Ether, Tetrahydrofuran, Dioxan oder Dimethylformamid gegebenenfalls in Gegenwart einer anorganischen oder organischen Base, wie Natriumhydroxid, Kaliumcarbonat, Triethylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

Bedeutet $Z_3$ eine Hydroxygruppe und X eine Carbonylgruppe, so wird die Umsetzung vorzugsweise in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, und gegebenenfalls in Gegenwart einer anorganischen Base wie Natriumcarbonat oder einer organischen Base wie Triethylamin oder Pyridin, welche gleichzeitig als Lösungsmittel dienen können, bei Temperaturen zwischen -25°C und 150°C, vorzugsweise jedoch bei Temperaturen zwischen -10°C und der Siedetemperatur des verwendeten Lösungsmittels, durchgeführt.

d) Zur Herstellung einer Verbindung der allgemeinen Formel I, in der $R_a$ eine $R_1NR_2$- oder $R_1N(XR_3)$-Gruppe, in der $R_1$ mit Ausnahme des Wasserstoffatoms wie eingangs definiert ist, und $R_b$ eine Cyanogruppe oder eine durch eine $C_{1-10}$-Alkoxycarbonylgruppe oder Phenyl-$C_{1-3}$-alkoxycarbonylgruppe substituierte Amidinogruppe darstellen:

Umsetzung einer Verbindung der allgemeinen Formel

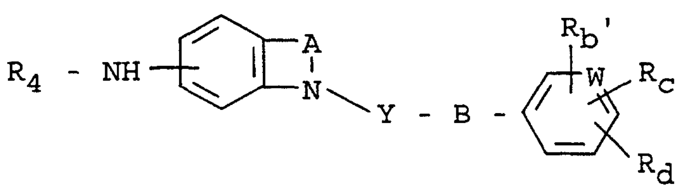

, (VII)

in der
A, B, W, Y, $R_c$ und $R_d$ wie eingangs definiert sind,
$R_4$ die für $R_2$ eingangs erwähnten Bedeutungen aufweist oder eine $R_3$-X-Gruppe darstellt, wobei $R_3$ und X wie eingangs erwähnt definiert sind, und
$R_b$' eine Cyanogruppe oder eine durch eine $C_{1-10}$-Alkoxycarbonylgruppe oder Phenyl-$C_{1-3}$-alkoxycarbonylgruppe substituierte Amidinogruppe darstellt, mit einer Verbindung der allgemeinen Formel

$$Z_4\text{-}R_1' \qquad ,(VIII),$$

in der
$R_1$' eine $C_{1-5}$-Alkylgruppe, welche durch eine Phenyl-, Carboxy-, $C_{1-4}$-Alkoxycarbonyl- oder Aminocarbonylgruppe

substituiert sein kann, wobei die Aminogruppe der Aminocarbonylgruppe zusätzlich durch $C_{1-4}$-Alkyl-, Phenyl-$C_{1-3}$-alkyl-, Phenyl-, Carboxy-$C_{1-3}$-alkyl- oder $C_{1-3}$-Alkoxycarbonyl-$C_{1-3}$-alkylgruppen mono- oder disubstituiert und die Substituenten gleich oder verschieden sein können, oder eine geradkettige $C_{2-3}$-Alkylgruppe, die endständig durch eine Di-($C_{1-3}$-Alkyl)amino-, Pyrrolidino-, Piperidino- oder Morpholinogruppe substituiert ist, und

$Z_4$ eine Austrittsgruppe wie ein Halogenatom, z. B. ein Chloroder Bromatom, darstellen.

Die Umsetzung wird zweckmäßigerweise in einem Lösungsmittel oder Lösungsmittelgemisch wie Wasser, Methylenchlorid, Chloroform, Ether, Tetrahydrofuran, Dioxan oder Dimethylformamid gegebenenfalls in Gegenwart einer anorganischen oder organischen Base, wie Natriumhydroxid, Kaliumcarbonat, Triethylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

e) Zur Herstellung einer Verbindung der allgemeinen Formel I, in der $R_a$ eine Nitrogruppe und $R_b$ eine Cyanogruppe darstellen:

Nitrierung einer Verbindung der allgemeinen Formel

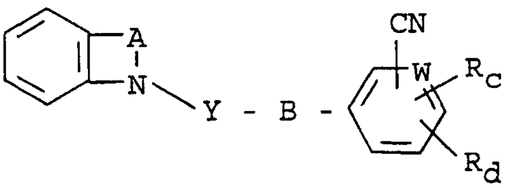

, (IX)

in der

A, B, W, Y, $R_c$ und $R_d$ wie eingangs definiert sind.

Die Nitrierung wird vorzugsweise in einem Lösungsmittel wie Eisessig oder Tetrahydrofuran in Gegenwart eines Nitrierungsmittels wie verdünnte oder konzentrierte Salpetersäure oder Salpetersäure/Schwefelsäure bei Temperaturen zwischen 0 und 50°C, vorzugsweise bei Raumtemperatur, durchgeführt. Die Nitrierung kann auch ohne Lösungsmittel durchgeführt werden. Desweiteren kann ein gegebenenfalls erhaltenes Stellungsisomerengemisch mittels üblichen Methoden, z. B. mittels Chromatographie, in die einzelnen Isomeren aufgetrennt werden.

f) Zur Herstellung einer Verbindung der allgemeinen Formel I, in der $R_a$ eine Aminogruppe und $R_b$ eine Cyanogruppe darstellen:

Reduktion einer Verbindung der allgemeinen Formel

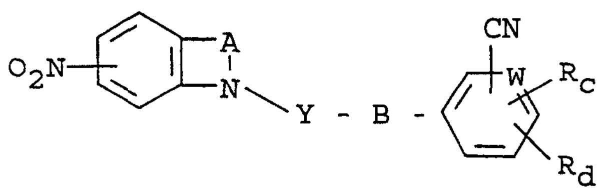

, (X)

in der

A, B, W, Y, $R_c$ und $R_d$ wie eingangs definiert sind.

Die Reduktion wird vorzugsweise in einem Lösungsmittel wie Wasser, Wasser/Ethanol, Methanol, Eisessig, Essigsäurethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit, oder mit Hydrazin in Gegenwart von Raney-Nickel bei Temperaturen zwischen 0 und 80°C, vorzugsweise jedoch bei Temperaturen zwischen 20 und 40°C, durchgeführt.

g) Zur Herstellung einer Verbindung der allgemeinen Formel I, in der $R_b$ eine Amidinogruppe darstellt:

Umsetzung einer gegebenenfalls im Reaktionsgemisch gebildeten Verbindung der allgemeinen Formel

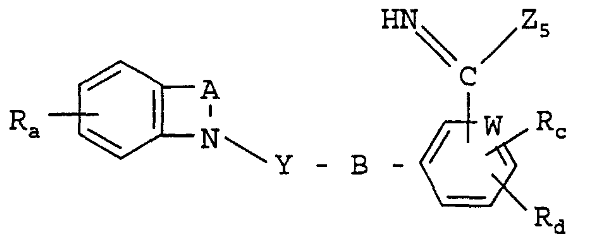

, (XI)

in der
A, B, W, Y, $R_a$, $R_c$ und $R_d$ wie eingangs definiert sind und
$Z_5$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit Ammoniak oder mit dessen Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen -10 und 150°C, vorzugsweise bei Temperaturen zwischen 0 und 120°C, mit einem entsprechenden freien Amin oder mit einem entsprechenden Säureadditionssalz wie beispielsweise den entsprechenden Ammoniumcarbonaten, -acetaten oder -chloriden durchgeführt.

Eine Verbindung der allgemeinen Formel XI erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50°C, vorzugsweise jedoch bei Temperaturen zwischen 0 und 30°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid oder durch Umsetzung eines entsprechenden Nitrils mit einem Alkoholat wie Natriummethylat in einem Lösungsmittel wie Dioxan oder Tetrahydrofuran, vorzugsweise jedoch in dem entsprechenden Alkohol. Bei den Umsetzungen mit einem Alkohol kann gleichzeitig eine vorhandene Estergruppe umgeestert werden.

h) Zur Herstellung einer Verbindung der allgemeinen Formel I, in der $R_b$ eine durch eine $C_{1-10}$-Alkoxycarbonylgruppe oder Phenyl-$C_{1-3}$-alkoxycarbonylgruppe substituierte Amidinogruppe darstellt:

Umsetzung einer Verbindung der allgemeinen Formel

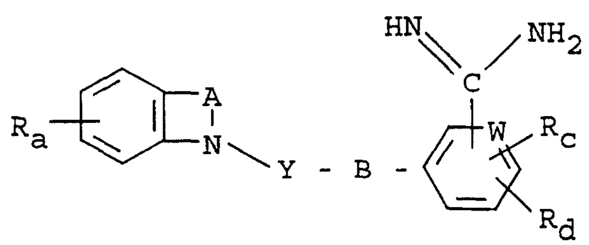

, (XII)

in der
A, B, W, Y, $R_a$, $R_c$ und $R_d$ wie eingangs definiert sind, mit einer Verbindung der allgemeinen Formel

$$Z_6\text{-CO-OR}_4 \quad ,(XIII)$$

in der
$R_4$ eine $C_{1-10}$-Alkyl- oder Phenyl-$C_{1-3}$-alkylgruppe und
$Z_6$ eine Austrittsgruppe wie ein Halogenatom, z.B. ein Chloroder Bromatom, darstellen.

Die Umsetzung wird zweckmäßigerweise in einem Lösungsmittel wie Tetrahydrofuran, Methylenchlorid, Chloroform, Dimethylformamid, Wasser oder Gemischen aus diesen Lösungsmitteln gegebenenfalls in Gegenwart einer Base wie Natriumcarbonat, Kaliumcarbonat oder Natronlauge oder in Gegenwart einer organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 60°C, durchgeführt.

i) Zur Herstellung einer Verbindung der allgemeinen Formel I, in der $R_b$ eine Aminomethylgruppe darstellt:
Reduktion einer Verbindung der allgemeinen Formel

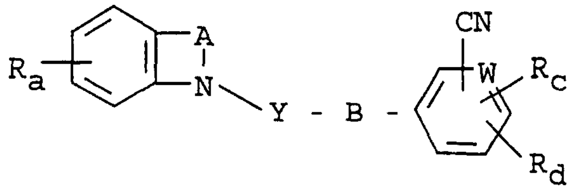
, (XIV)

in der
A, B, W, Y, $R_a$, $R_c$ und $R_d$ wie eingangs definiert sind.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100°C, vorzugsweise bei Temperaturen zwischen 20 und 80°C, durchgeführt.

j) Zur Herstellung einer Verbindung der allgemeinen Formel I, in der $R_a$ eine $R_1NR_2$-Gruppe, in der $R_1$ mit Ausnahme des Wasserstoffatoms wie eingangs definiert ist, und $R_b$ eine Cyanogruppe oder eine durch eine $C_{1-10}$-Alkoxycarbonylgruppe oder Phenyl-$C_{1-3}$-alkoxycarbonylgruppe substituierte Amidinogruppe darstellen:
Umsetzung einer Verbindung der allgemeinen Formel

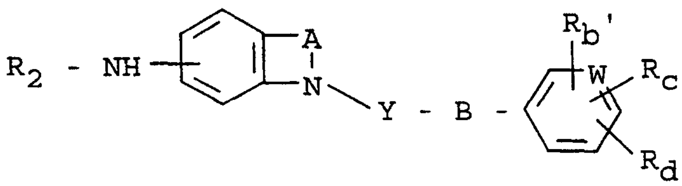
, (XV)

in der
A, B, W, Y, $R_2$, $R_c$ und $R_d$ wie eingangs definiert sind und
$R_b$' eine Cyanogruppe oder eine durch eine $C_{1-10}$-Alkoxycarbonylgruppe oder Phenyl-$C_{1-3}$-alkoxycarbonylgruppe substituierte Amidinogruppe darstellt, mit einer Verbindung der allgemeinen Formel

$$Z_7\text{-}R_1' \qquad ,(XVI),$$

in der
$R_1$' wie eingangs erwähnt definiert ist und
$Z_7$ eine Austrittsgruppe wie ein Halogenatom, z. B. ein Chloroder Bromatom, oder zusammen mit einem Wasserstoffatom des benachbarten Kohlenstoffatoms ein Sauerstoffatom darstellen.

Die Umsetzung wird zweckmäßigerweise in einem Lösungsmittel oder Lösungsmittelgemisch wie Wasser, Methylenchlorid, Chloroform, Ether, Tetrahydrofuran, Dioxan oder Dimethylformamid gegebenenfalls in Gegenwart einer anorganischen oder organischen Base, wie Natriumhydroxid, Kaliumcarbonat, Triethylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

Die Umsetzung mit einer Carbonylverbindung der allgemeinen Formel XVI wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/-Kohle, oder in Gegenwart eines Metallhydrids wie Natriumborhydrid, Lithiumborhydrid oder Lithiumaluminiumhydrid bei Temperaturen zwischen 0 und 100°C, vorzugsweise bei Temperaturen zwischen 20 und 80°C, durchgeführt.

k) Zur Herstellung einer Verbindung der allgemeinen Formel I, in der $R_b$ eine Cyanogruppe und Y eine Carbonylgruppe darstellen:

Umsetzung einer Verbindung der allgemeinen Formel

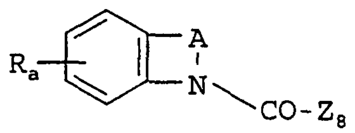

, (XVII)

in der

A und $R_a$ wie eingangs definiert sind und

$Z_8$ eine Austrittsgruppe wie ein Halogenatom, z.B. ein Chlor-, Brom- oder Jodatom, darstellt, mit einer Verbindung der allgemeinen Formel

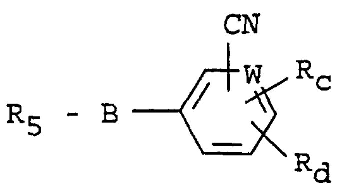

, (XVIII)

in der

B, W, $R_c$ und $R_d$ wie eingangs definiert sind und

$R_5$ eine gegenbenenfalls durch eine $C_{1-3}$-Alkylgruppe substituierte Aminogruppe darstellt.

Die Umsetzung wird zweckmäßigerweise in einem Lösungsmittel oder Lösungsmittelgemisch wie Wasser, Methylenchlorid, Chloroform, Ether, Tetrahydrofuran, Dioxan oder Dimethylformamid gegebenenfalls in Gegenwart einer anorganischen oder organischen Base, wie Natriumhydroxid, Kaliumcarbonat, Triethylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

Erhält man erfindungsgemäß eine Verbindung der allgemeinen Formel I, in der X eine Carbonylgruppe darstellt, so kann diese mittels eines schwefeleinführenden Mittels in eine entsprechende Thiocarbonylverbindung übergeführt werden oder

erhält man erfindungsgemäß eine Verbindung der allgemeinen Formel I, in der $R_a$ einen Acylrest enthält, so kann diese mittels Hydrolyse in eine Verbindung der allgemeinen Formel I, in der $R_a$ eine $R_1$NH-Gruppe darstellt oder in der $R_a$ eine Carboxygruppe enthält, übergeführt werden oder

erhält man erfindungsgemäß eine Verbindung der allgemeinen Formel I, in der $R_a$ eine Carboxy- oder Sulfonsäuregruppe darstellt oder enthält, so kann diese mittels Amidierung in eine entsprechende Amidverbindung der allgemeinen Formel I übergeführt werden.

Die Umsetzung wird mit einem schwefeleinführenden Mittel wie Phosphorpentasulfid oder 2,4-Bis(4-methoxyphenyl)-1,3-di-thia-2,4-diphosphetan-2,4-disulfid zweckmäßigerweise in einem Lösungsmittel wie Toluol oder Xylol bei Temperaturen zwischen 50 und 150°C, z.B. bei der Siedetemperatur des Reaktionsgemisches, durchgeführt.

Die nachträgliche Hydrolye erfolgt vorzugsweise hydrolytisch in einem wässerigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches.

Die nachträgliche Amidierung wird gegebenenfalls in einem Lösungsmittel oder Lösungsmittelgemisch wie

Methylenchlorid, Dimethylformamid, Benzol, Toluol, Chlorbenzol, Tetrahydrofuran, Benzol/Tetrahydrofuran oder Dioxan mit einem entsprechenden Amin gegebenenfalls in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/-N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methylmorpholin oder Triethylamin zweckmäßigerweise bei Temperaturen zwischen 0 und 150°C, vorzugsweise bei Temperaturen zwischen 0 und 100°C, durchgeführt.

Die Umsetzung einer entsprechenden reaktionsfähigen Carbonoder Sulfonsäue wie deren Ester, Imidazolide oder Halogeniden mit einem entsprechenden Amin wird vorzugsweise in einem entsprechenden Amin als Lösungsmittel gegebenenfalls in Gegenwart eines weiteren Lösungsmittels wie Methylenchlorid oder Ether und vorzugsweise in Gegenwart einer tertiären organische Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Ferner können die erhaltenen Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure oder Methansulfonsäure in Betracht.

Außerdem lassen sich die so erhaltenen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsstoffe verwendeten Verbindungen der allgemeinen Formeln II bis XVIII erhält man nach literaturbekannten Verfahren bzw. sind literaturbekannt.

So erhält man beispielsweise eine Verbindung der allgemeinen Formel II durch Hydrierung einer entsprechenden ungesättigten Verbindung und eine Ausgangsverbindung der allgemeinen Formeln V, VII, IX, X, XI und XIV durch Alkylierung oder Acylierung einer so erhaltenen Verbindung der allgemeinen Formel II.

Wie bereits eingangs erwähnt, weisen die Verbindungen der allgemeinen Formel I und deren Salze wertvolle Eigenschaften auf. So stellen die Verbindungen der allgemeinen Formel I, in der $R_b$ ein Wasserstoffatom, eine Nitro- oder Cyanogruppe darstellt, wertvolle Zwischenprodukte zur Herstellung der übrigen Verbindungen der allgemeinen Formel I dar, und die Verbindungen der obigen allgemeinen Formel I, in denen $R_b$ eine der vorstehend erwähnten gegebenenfalls substituierten Aminomethyl- oder Amidinogruppen darstellt, sowie deren physiologisch verträglichen Salze wertvolle pharmakologische Eigenschaften auf, insbesondere eine thrombinhemmende und thrombozytenaggregationshemmende Wirkung, eine die Thrombinzeit verlängernde Wirkung und eine Hemmwirkung auf verwandte Serinproteasen wie z. B. Trypsin, Plasmin, Urokinase Faktor VIIa, Faktor Xa, Faktor IX, Faktor XI und Faktor XII.

Beispielsweise wurden die Verbindungen

A = 1-[3-(4-Amidino-phenyl)propionyl]-6-butylsulfonamido-1,2,3,4-tetrahydro-chinolin-hydrochlorid,

B = 1-[3-(4-Amidino-phenyl)-propionyl]-1,2,3,4-tetrahydrochinolin-6-carbonsäure-methyl-N-phenyl-amid,

C = 1-[3-(4-Amidino-phenyl)-propionyl]-1,2,3,4-tetrahydrochinolin-6-carbonsäure-diethylamid,

D = N-Benzyl-N-fl-[3-(4-amidino-phenyl)-propionyl]-1,2,3,4-tetrahydro-chinolin-6-yl}-acetamid,

E = ({1-[3-(4-Amidino-phenyl)-propionylJ-1,2,3,4-tetrahydrochinolin-6-carbonyl}-phenyl-amino)-essigsäure,

F = 1-[3-(4-Amidino-phenyl)propionyll-6-phenylsulfonamido-1,2,3,4-tetrahydro-chinolin-hydrochlorid,

G = (i) 1- [3- (4-Amidino-phenyl) propionyl] -6- (N-carboxymethylphenylsulfonamido)-1,2,3,4-tetrahydro-chinolin,

H = [{1-[3-(4-Amidino-phenyl)-propionyl]-1,2,3,4-tetrahydrochinolin-6-yl}-(naphthalin-1-sulfonyl)-amino]-essigsäure und

I = [{1-[3-(4-Amidino-phenyl)-propionyl]-1,2,3,4-tetrahydrochinolin-6-yl}-(chinolin-8-sulfonyl)-amino]-essigsäure

auf ihre Wirkung auf die Thrombinzeit wie folgt untersucht:

Material: Plasma, aus humanem Citratblut.
Test-Thrombin (Rind), 30 U/ml, Behring Werke, Marburg
Diethylbarbituratacetat-Puffer, ORWH 60/61, Behring Werke, Marburg
Biomatic B10 Koagulometer, Sarstedt

Durchführung:

Die Bestimmung der Thrombinzeit erfolgte mit einem Biomatic B10-Koagulometer der Firma Sarstedt.

Die Testsubstanz wurde in die vom Hersteller vorgeschriebenen Testgefäßen mit 0,1 ml humanem Citrat-Plasma und 0,1 ml Diethylbarbiturat-Puffer (DBA-Puffer) gegeben. Der Ansatz wurde für eine Minute bei 37°C inkubiert. Durch Zugabe von 0,3 U Test-Thrombin in 0,1 ml DBA-Puffer wurde die Gerinnungsreaktion gestartet. Gerätebedingt

erfolgt mit der Eingabe von Thrombin die Messung der Zeit bis zur Gerinnung des Ansatzes. Als Kontrolle dienten Ansätze bei denen 0,1 ml DBA-Puffer zugegeben wurden.

Gemäß der Definition wurde über eine Dosis-Wirkungskurve die effective Substanzkonzentration ermittelt, bei der die Thrombinzeit gegenüber der Kontrolle verdoppelt wurde.

Die nachfolgende Tabelle enthält die gefundenen Werte:

| Substanz | Thrombinzeit ($ED_{200}$ in μM) |
|---|---|
| A | 0,08 |
| B | 0,02 |
| C | 0,10 |
| D | 0,05 |
| E | 0,04 |
| F | 0,04 |
| G | 0,03 |
| H | 0,02 |
| I | 0,03 |

Desweiteren konnten an Ratten bei der Applikation der vorstehenden Verbindungen bis zu einer Dosis von 20 mg/kg i.v. oder 100 mg/kg p.o. keine toxischen Nebenwirkungen beobachtet werden. Die Verbindungen sind demnach gut verträglich.

Aufgrund ihrer pharmakologischen Eigenschaften eignen sich die Verbindungen und deren physiologisch verträglichen Salze zur Vorbeugung und Behandlung venöser und arterieller thrombotischer Erkrankungen, wie zum Beispiel der Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen wie Lungenembolie, der disseminierten intravaskulären Gerinnung, der Prophylaxe der Koronarthrombose, der Prophylaxe des Schlaganfalls und der Verhinderung der Occlusion von Shunts. Zusätzlich sind die erfindungsgemäßen Verbindungen zur antithrombotischen Unterstützung bei einer thrombolytischen Behandlung, wie zum Beispiel mit rt-PA oder Streptokinase, zur Verhinderung der Langzeitrestenose nach PT(C)A, zur Verhinderung der Metastasierung und des Wachstums von koagulationsabhängigen Tumoren und von fibrinabhängigen Entzündungsprozessen geeignet.

Die zur Erzielung einer entsprechenden Wirkung erforderliche Dosierung beträgt zweckmäßigerweise bei intravenöser Gabe 0,1 bis 50 mg/kg, vorzugsweise 0,5 bis 30 mg/kg, und bei oraler Gabe 1 bis 100 mg/kg, vorzugsweise 0,5 bis 50 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Herstellung der Ausgangsverbindungene:

Beispiel I

3-(4-Cyano-phenyl)propionsäure

100,1 g (0,578 Mol) 4-Cyano-zimtsäure werden in 1400 ml 1N Kaliumcarbonat-Lösung aufgenommen und über Palladium-Kohle bei 5 bar während 2,5 Stunden hydriert. Anschließend stellt man die Lösung leicht sauer und saugt den Niederschlag ab, der dann im Umlufttrockenschrank getrocknet wird.
Ausbeute: 90,8 g (89,6% der Theorie),
Schmelzpunkt: 137-139°C

Beispiel II

6-Phenylsulfonamido-chinolin

61,5 g (0,426 Mol) 6-Amino-chinolin werden in 210 ml Pyridin gelöst und unter Eiskühlung mit 82,8 g (0,469 Mol) Benzolsulfonsäurechlorid versetzt. Anschließend erwärmt man die Lösung auf 100°C und läßt nach 20 Minuten langsam auf 45°C abkühlen. Dann gibt man 85 ml 6N Natronlauge zu und engt zur Trockene ein. Der verbleibende Niederschlag wird erst mit Wasser, dann mit Ethanol gewaschen und anschließend getrocknet.
Ausbeute: 106,6 g (87,9% der Theorie),
Schmelzpunkt: 219-221°C
Analog werden hergestellt:

(1) 6-(2-Naphthyl-sulfonamido)-chinolin Schmelzpunkt: 152°C

(2) 6-(1-Naphthyl-sulfonamido)-chinolin Schmelzpunkt: 248°C

(3) 6-(4-Fluor-phenylsulfonamido)-chinolin Schmelzpunkt: 220-221°C

(4) 6-Butylsulfonamido-chinolin Schmelzpunkt: 82-84°C

(5) 5-Phenylsulfonamido-chinolin Schmelzpunkt: 112°C

(6) 7-Phenylsulfonamido-chinolin Schmelzpunkt: 185-187°C

(7) 7-Benzylsulfonamido-chinolin $R_f$-Wert: 0,73 (Kieselgel; Methylenchlorid/Methanol = 9:1)

(8) 6-Benzylcarboxamido-chinolin Schmelzpunkt: 146-149°C

(9) 6-Phenylsulfonamido-2-methyl-chinolin $R_f$-Wert: 0,61 (Kieselgel; Essigester)

(10) 6-Benzylsulfonamido-chinolin Schmelzpunkt: 179-181°C

(11) 6-Benzoylamino-chinolin Schmelzpunkt: 155-158°C

(12) 6-(4-Chlor-phenylsulfonamido)-chinolin

(13) 6-(4-Brom-phenylsulfonamido)-chinolin

(14) 6-(3-Chlor-phenylsulfonamido)-chinolin

(15) 6-(3-Brom-phenylsulfonamido)-chinolin

(16) 6-(4-Methyl-phenylsulfonamido)-chinolin

Beispiel III

6-(N-Methyl-phenylsulfonamido)-chinolin

4,0 g 6-Phenylsulfonamido-chinolin werden in 50 ml Dimethylsulfoxid gelöst und bei Raumtemperatur mit 1,83 g Kaliumtert.butylat versetzt. Dann tropft man 2,13 g Methyljodid zu und läßt über Nacht weiterrühren. Anschließend wird auf 300 ml Eiswasser gegossen und mit Essigsäureethylester extrahiert. Die organische Phase wird getrocknet und eingeengt.
Ausbeute: 3,0 g (71,8% der Theorie),
Schmelzpunkt: 101-103°C
Analog werden hergestellt:

(1) 6- [N- (2-Phenylethyl) -phenylsulfonamido] chinolin

(2) 6-[N-(Ethoxycarbonylmethyl)-phenylsulfonamido]chinolin

Beispiel IV

6-Phenylsulfonamido-1,2,3,4-tetrahydro-chinolin

106,6 g (0,375 Mol) 6-Phenylsulfonamido-chinolin werden in 1400 ml Eisessig gelöst und über 17 g Platinoxid bei 3 bar während 70 Minuten hydriert. Anschließend wird vom Katalysator abgesaugt, eingeengt und der Rückstand mit wenig Ethanol gewaschen und getrocknet.
Ausbeute: 98,4 g (91,0% der Theorie),
Schmelzpunkt: 160-162°C

Analog werden hergestellt:

(1) 6-(2-Naphthyl-sulfonamido)-1,2,3,4-tetrahydro-chinolin
Schmelzpunkt: 152-154°C

(2) 6-(1-Naphthyl-sulfonamido)-1,2,3,4-tetrahydro-chinolin
Schmelzpunkt: 175-176°C

(3) 6-(4-Fluor-phenylsulfonamido)-1,2,3,4-tetrahydro-chinolin
Schmelzpunkt: 85°C

(4) 6-Butylsulfonamido-1,2,3,4-tetrahydro-chinolin

| $C_{13}H_{20}N_2O_2S$ (268,36) | | | |
|---|---|---|---|
| Ber. | C 58,18 | H 7,51 | N 10,43 |
| Gef. | 57,95 | 7,70 | 10,22 |

(5) 6-(N-Methyl-phenylsulfonamido)-1,2,3,4-tetrahydro-chinolin

(6) 7-Benzylsulfonamido-1,2,3,4-tetrahydro-chinolin
$R_f$-Wert: 0,72 (Kieselgel; Essigester)

(7) 5-Phenylsulfonamido-1,2,3,4-tetrahydro-chinolin
$R_f$-Wert: 0,82 (Kieselgel; Essigester)

(8) 7-Phenylsulfonamido-1,2,3,4-tetrahydro-chinolin
$R_f$-Wert: 0,83 (Kieselgel; Essigester)

(9) 6-Phenylacetylamino-1,2,3,4-tetrahydro-chinolin
Schmelzpunkt: 116-118°C

(10) 2-Methyl-6-phenylsulfonamido-1,2,3,4-tetrahydro-chinolin
$R_f$-Wert: 0,66 (Kieselgel; Toluol/Essigester = 6:4)

(11) 6-Benzylsulfonamido-1,2,3,4-tetrahydro-chinolin

(12) 6-Benzoylamino-1,2,3,4-tetrahydro-chinolin
Schmelzpunkt: 150-153°C

(13) 6-(4-Fluor-phenylsulfonamido)-1,2,3,4-tetrahydro-chinolin
Schmelzpunkt: 85°C

(14) 6-n-Butylsulfonamido-1,2,3,4-tetrahydro-chinolin

| $C_{13}H_{20}N_2O_2S$ (268,38) | | | |
|---|---|---|---|
| Ber. | C 58,18 | H 7,57 | N 10,43 |
| Gef. | 57,95 | 7,70 | 10,22 |

(15) 6-[N-(2-Phenylethyl)-phenylsulfonamido]-1,2,3,4-tetrahydro-chinolin
$R_f$-Wert: 0,60 (Kieselgel; Essigester/Petrolether = 1:1)

(16) 6-[N-(Ethoxycarbonylmethyl)-phenylsulfonamido]-1,2,3,4-tetrahydro-chinolin

(17) 6-(4-Chlor-phenylsulfonamido)-1,2,3,4-tetrahydro-chinolin

(18) 6-(4-Brom-phenylsulfonamido)-1,2,3,4-tetrahydro-chinolin

(19) 6-(3-Chlor-phenylsulfonamido)-1,2,3,4-tetrahydro-chinolin

(20) 6-(3-Brom-phenylsulfonamido)-1,2,3,4-tetrahydro-chinolin

(21) 6-(4-Methyl-phenylsulfonamido)-1,2,3,4-tetrahydro-chinolin

(22) 6-Morpholinocarbonyl-1,2,3,4-tetrahydrochinolin Hergestellt aus der gemäß Beispiel VII(6) hergestellten Verbindung
Ausbeute: 96% der Theorie,
$R_f$-Wert: 0,64 (Kieselgel; Essigsäureethylester)

(23) 6-Piperidinocarbonyl-1,2,3,4-tetrahydrochinolin Hergestellt aus der gemäß Beispiel VII hergestellten Verbindung
Ausbeute: 36% der Theorie,
$R_f$-Wert: 0,57 (Kieselgel; Essigsäureethylester)

(24) 6-Benzylaminocarbonyl-1,2,3,4-tetrahydrochinolin Hergestellt aus der gemäß Beispiel VII(1) hergestellten Verbindung
Ausbeute: 50% der Theorie,
$R_f$-Wert: 0,89 (Kieselgel; Essigsäureethylester)

(25) 6-(N-Methyl-phenylaminocarbonyl)-1,2,3,4-tetrahydrochinolin
Hergestellt aus der gemäß Beispiel VII(2) hergestellten Verbindung
Ausbeute: 62% der Theorie,
$R_f$-Wert: 0,84 (Kieselgel; Essigsäureethylester)

(26) 6-Diethylaminocarbonyl-1,2,3,4-tetrahydrochinolin Ausbeute: 98% der Theorie,
Hergestellt aus der gemäß Beispiel VII(3) hergestellten Verbindung
$R_f$-Wert: 0,60 (Kieselgel; Essigsäureethylester)

(27) 6-(3',5'-Dimethyl-piperidinocarbonyl)-1,2,3,4-tetrahydrochinolin
Hergestellt aus der gemäß Beispiel VII(4) hergestellten Verbindung
Ausbeute: 97% der Theorie,
$R_f$-Wert: 0,67 (Kieselgel; Essigsäureethylester)

(28) 6-Methoxycarbonyl-1,2,3,4-tetrahydrochinolin
Hergestellt aus Chinolin-6-carbonsäuremethylester (Hergestellt analog J.Amer.Chem.Soc.68, 2721 (1946))
Ausbeute: 60% der Theorie

(29) 6-(4'-Methylpiperidinocarbonyl)-1,2,3,4-tetrahydrochinolin
Hergestellt aus der gemäß Beispiel VII(5) hergestellten Verbindung
Ausbeute: 96% der Theorie,
$R_f$-Wert: 0,67 (Kieselgel; Essigsäureethylester)

Beispiel V

6-Trifluoracetylamino-chinolin

7,2 g (0,05 Mol) 6-Amino-chinolin und 14,2 g (0,11 Mol) N,N-Diisopropyl-ethylamin werden in 100 ml Methylenchlorid gelöst. Anschließend tropft man bei ca. O°C 11,55 g (0,055 Mol) Trifluoressigsäureanhydrid zu und rührt bei dieser Temperatur 1 Stunde. Der ausgefallene Niederschlag wird abgesaugt und mit Methylenchlorid und Wasser gewaschen und dann getrocknet. Die Mutterlauge wird dreimal mit Methylenchlorid extrahiert, dann wird die organische Phase abgetrennt, über Natriumsulfat getrocknet, einrotiert und mit dem oben erhaltenen Niederschlag vereinigt.
Ausbeute: 10,69 g (89,0% der Theorie),
Schmelzpunkt: 183-185°C

Beispiel VI

6-Trifluoracetylamino-1,2,3,4-tetrahydro-chinolin

2,4 g (0,01 Mol) 6-Trifluoracetylamino-chinolin werden in 20 ml Eisessig gelöst und mit 0,6 g Platinoxid bei 3 bar während 1 Stunde hydriert. Anschließend wird der Katalysator abgesaugt und die Lösung eingeengt. Der Rückstand wird mit wenig Natriumhydrogencarbonat-Lösung gewaschen und getrocknet.
Ausbeute: 1,79 g (74% der Theorie)
Schmelzpunkt: 95-97°C

Beispiel VII

6-Piperidinocarbonyl-chinolin

3,0 g Chinolin-6-carbonsäurechlorid (Hergestellt analog J. Med. Chem. 38. 3094-3105 (1995)) werden in 70 ml Pyridin mit 4,25 ml Piperidin bei Raumtemperatur versetzt und 20 Minuten gerührt. Anschließend wird eingeengt, der Rückstand in wenig Wasser aufgenommen und mit Methylenchlorid extrahiert. Die organische Phase wird eingeengt und über eine Kieselgelsäule mit Essigsäureethylester filtriert.
Ausbeute: 2,1 g (52% der Theorie),
$R_f$-Wert: 0,24 (Kieselgel; Essigsäureethylester)

Analog werden hergestellt:

(1) 6-Benzylaminocarbonyl-chinolin
Ausbeute: 73% der Theorie,
Rf-Wert: 0,45 (Kieselgel; Essigsäureethylester)

(2) 6-(N-Methyl-phenylaminocarbonyl)-chinolin
Ausbeute: 83% der Theorie,
$R_f$-Wert: 0,49 (Kieselgel; Essigsäureethylester)

(3) 6-Diethylaminocarbonyl-chinolin
Ausbeute: 72% der Theorie,
$R_f$-Wert: 0,24 (Kieselgel; Essigsäureethylester)

(4) 6-(3',5'-Dimethyl-piperidinocarbonyl)-chinolin Ausbeute: 33% der Theorie,
$R_f$-Wert: 0,25 (Kieselgel; Essigsäureethylester)

(5) 6-(4'-Methylpiperidinocarbonyl)-chinolin
Ausbeute: 50% der Theorie,
$R_f$-Wert: 0,21 (Kieselgel; Essigsäureethylester)

(6) 6-Morpholinocarbonyl-chinolin
Ausbeute: 69% der Theorie,
$R_f$-Wert: 0,22 (Kieselgel; Essigsäureethylester)

Herstellung der Verbindungen der allgemeinen Formel I:

Beispiel 1

1-[3-(4-Cyano-phenyl)propionyl)-5-nitro-2,3-dihydro-indol

1,6 g 5-Nitro-2,3-dihydro-indol werden in 40 ml Methylenchlorid gelöst, anschließend mit 1,5 ml Triethylamin und dann mit 1,93 g 3-(4-Cyano-phenyl)propionsäurechlorid in 4 ml Methylenchlorid versetzt. Nachdem man über Nacht gerührt hat, wird zur Trockene eingeengt. Das Produkt (3 g, 93 % der Theorie) wird ohne weitere Reinigung verarbeitet.

Analog werden hergestellt:

(1) 1-[3-(4-Cyano-phenyl)propionyl]-3-methyl-6-phenylsulfonamido-1,2,3,4-tetrahydro-chinolin
Ausbeute: 94 % der Theorie,
$R_f$-Wert: 0,67 (Kieselgel; Toluol/Essigester = 6:4)

(2) 1-[3-(4-Cyano-phenyl)propionyl]-2,3,4,5-tetrahydro-benzo-[b] azepin
Ausbeute: 82 % der Theorie,
$R_f$-Wert: 0,55 (Kieselgel; Toluol/Essigester = 7:3)

(3) 1-[3-(4-Cyano-phenyl)propionyl]-4-methyl-6-nitro-tetrahydro-chinolin
Hergestellt aus 3-(4-Cyano-phenyl)propionylchlorid und 4-Methyl-6-nitro-tetrahydro-chinolin
Ausbeute: 55 % der Theorie,
$R_f$-Wert: 0,45 (Kieselgel; Toluol/Essigester = 9:1)

Beispiel 2

5-Amino-1-[3-(4-cyano-phenyl)propionyl]-2,3-dihydro-indol

2 g 1-[3-(4-Cyano-phenyl)propionyl]-5-nitro-2,3-dihydro-indol werden in 100 ml Methanol/Methylenchlorid gelöst und bei 3 bar über Palladiumkohle hydriert. Dann wird eingeengt.
Ausbeute: 2 g (74 % der Theorie),
$R_f$-Wert: 0,20 (Kieselgel; Toluol/Essigester = 6:4)

Analog werden hergestellt:

(1) 6-Amino-1-[3-(4-cyano-phenyl)propionyl]-3-methyl-1,2,3,4-tetrahydro-chinolin
Hergestellt aus der gemäß Beispiel 3(1) hergestellten Verbindung
Ausbeute: 60 % der Theorie,
$R_f$-Wert: 0,35 (Kieselgel; Toluol/Essigester = 8:2)

(2) 7-Amino-1-[3-(4-cyano-phenyl)-propionyl]-2,3,4,5-tetrahydro-1H-benzo [b] azepin
Hergestellt aus der gemäß Beispiel 3 hergestellten Verbindung Ausbeute: 74 % der Theorie,
$R_f$-Wert: 0,13 (Kieselgel; Toluol/Essigester = 7:3)

(3) 6-Amino-1-[3-(4-cyano-phenyl)propionyl]-4-methyl-1,2,3,4-tetrahydro-chinolin
Ausbeute: 75 % der Theorie,
Schmelzpunkt: sintert ab 80°C

(4) 1-[3-(2-Amino-4-cyanophenyl)propionyl]-6-phenylsulfonylamino-1,2,3,4-tetrahydro-chinolin
Hergestellt aus der gemäß Beispiel 9(28) hergestellten Verbindung
Ausbeute: 67 % der Theorie,
$R_f$-Wert: 0,58 (Kieselgel; Methylenchlorid/Methanol = 4:1)

Beispiel 3

1-[3-(4-Cyano-phenyl)propionyl]-7-nitro-2,3,4,5-tetrahydro-1Hbenzo [b] azepin

3 g 1-[3-(4-Cyano-phenyl)propionyl]-2,3,4,5-tetrahydro-1H-benzo[b]azepin (siehe Beispiel 1(2)) werden in 17

ml Eisessig gelöst und mit 1 ml Salpetersäure in 3 ml Eisessig über Nacht bei Raumtemperatur gerührt. Danach wird eingeengt und der Rückstand in Wasser aufgenommen und 3 mal mit Methylenchlorid extrahiert. Die organische Phase wird getrocknet, eingeengt und der Rückstand mit Toluol/Essigester = 8:2 über eine Kieselgelsäule chromatographiert.
Ausbeute: 1,2 g (35 % der Theorie),
Schmelzpunkt: ab 127°C

Analog wird hergestellt:

(1) 1-[3-(4-Cyano-phenyl)propionyl]-3-methyl-6-nitro-1,2,3,4-tetrahydro-chinolin
Ausbeute: 85 % der Theorie,
$R_f$-Wert: 0,72 (Kieselgel; Toluol/Essigester = 8:2)

Beispiel 4

1-[3-(4-Cyano-phenyl)propionyl]-6-trifluoracetylamino-1,2,3,4-tetrahydro-chinolin

10 g (0,057 Mol) 3-(4-Cyano-phenyl)propionsäure und 6,6 g (0,065 Mol) N-Methyl-morpholin werden in 250 ml Tetrahydrofuran gelöst und auf -20°C abgekühlt. Dann werden 8,2 g (0,06 Mol) Chlorameisensäure-isobutylester zugetropft. Anschließend gibt man 13,9 g (0,057 Mol) 6-(Trifluoracetylamino)-1,2,3,4-tetrahydro-chinolin in 200 ml Tetrahydrofuran zu und läßt die Lösung über Nacht auf Raumtemperatur erwärmen. Dann wird mit 200 ml Essigester verdünnt und mit 2 x 80 ml 0,5 N Salzsäure und dann 100 ml Natriumhydrogencarbonatlösung gewaschen. Die organische Phase wird über Natriumsulfat getrocknet und im Vakuum eingeengt.
Ausbeute: 16 g (70 % der Theorie),
Schmelzpunkt: 151-152°C

| $C_{21}H_{18}F_3N_3O_2$ (401,39) | | | |
|---|---|---|---|
| Ber. | C 62,83 | H 4,51 | N 10,46 |
| Gef. | 62,45 | 4,55 | 10,44 |

Beispiel 5

6-Amino-1-[3-(4-cyano-phenyl)propionyl]-1,2,3,4-tetrahydrochinolin

16 g 1-[3-(4-Cyano-phenyl)propionyl]-6-trifluoracetylamino-1,2,3,4-tetrahydro-chinolin werden in 70 ml Methanol und 50 ml Dioxan gelöst und mit 200 ml 1N Natronlauge 2 Stunden bei 40°C gerührt. Dann wird mit Methylenchlorid extrahiert, die organische Phase über Natriumsulfat getrocknet und eingeengt. Ausbeute: 10,7 g (88 % der Theorie),
Schmelzpunkt: 180-200°C

| $C_{19}H_{19}N_3O$ (305,38) | | | |
|---|---|---|---|
| Ber. | C 74,72 | H 6,27 | N 13,75 |
| Gef. | 74,41 | 6,37 | 13,56 |

Reispiel 6

6-(N-Ethoxycarbonylmethyl-amino)-1-[3-(4-cyano-phenyl)-propionyl]-1,2,3,4-tetrahydro-chinolin

10,7 g (35 mMol) 6-Amino-1-[3-(4-cyano-phenyl)-propionyl]-1,2,3,4-tetrahydro-chinolin werden mit 9,2 ml (53 mMol) N-Ethyl-diisopropylamin in 70 ml Dimethylformamid gelöst und unter Eiskühlung mit 9 g (42 mMol) Iodessigesäureethylester versetzt. Die Lösung wird über Nacht gerührt und dann auf Wasser gegossen, mit Essigsäureethylester extrahiert und die organische Phase einrotiert.
Ausbeute: 12,7 g (92 % der Theorie),
Schmelzpunkt: 117-119°C

Analog werden hergestellt:

(1) 6-(N-Ethoxycarbonylmethyl-benzylamino)-1-[3-(4-cyano-phenyl)-propionyl]-1,2,3,4-tetrahydro-chinolin
Hergestellt aus 6-Benzylamino-1-[3-(4-cyano-phenyl)-propionyl]-1,2,3,4-tetrahydro-chinolin (siehe Beispiel 14)

und Jodessigsäureethylester
Ausbeute: 91 % der Theorie,
$R_f$-Wert: 0,36 (Kieselgel; Methylenchlorid/Essigester 19:1)

(2) 6-[N-Ethoxycarbonylmethyl-(naphthalin-2-ylmethyl)-amino]-1-[3-(4-cyano-phenyl)-propionyl]-1,2,3,4-tetrahydro-chinolin Hergestellt aus 6-[(Naphthalin-2-ylmethyl)-amino]-1-[3-(4-cyano-phenyl)-propionyl]-1,2,3,4-tetrahydro-chinolin (siehe Beispiel 14(2)) und Jodessigsäureethylester
Schmelzpunkt: Öl
Ausbeute: 93 % der Theorie,

(3) 6-[N-Ethoxycarbonylmethyl-(naphthalin-1-ylmethyl)-amino]-1-[3-(4-cyano-phenyl)-propionyl]-1,2,3,4-tetrahydro-chinolin Hergestellt aus 6-[(Naphthalin-1-ylmethyl)-amino]-1-[3-(4-cyano-phenyl)-propionyl]-1,2,3,4-tetrahydro-chinolin (siehe Beispiel 14(1)) und Jodessigsäureethylester
Schmelzpunkt: 80°C
Ausbeute: 96 % der Theorie,

(4) 1-[3-(4-Cyano-phenyl)-propionyl]-6-(N-ethoxycarbonylmethyl-2,2-diphenyl-ethylamino)-1,2,3,4-tetrahydro-chinolin Hergestellt aus 1-[3-(4-Cyano-phenyl)-propionyl]-6-(2,2-diphenyl-ethylamino)-1,2,3,4-tetrahydro-chinolin (siehe Beispiel 14(4)) und Jodessigsäureethylester
Ausbeute: 93 % der Theorie,
Schmelzpunkt: 156-158°C

Beispiel 7

1-[3-(4-Cyano-phenyl)-propionyl]-6-[N-ethoxycarbonylmethyl-(isochinolin-5-sulfonyl)-amino]-1,2,3,4-tetrahydro-chinolin

2,8 g 1-[3-(4-Cyano-phenyl)-propionyl]-6-(isochinolin-5-sulfonamido)-1,2,3,4-tetrahydro-chinolin (siehe Beispiel 10(14)) werden in 40 ml Dimethylsulfoxid gelöst, mit 670 mg Kaliumtert.butylat versetzt und 1 Stunde bei Raumtemperatur gerührt. Dann werden 1,0 g Bromessigsäureethylester zugegeben und die Lösung über Nacht gerührt. Anschließend wird auf Eiswasser gegossen, mit Essigester 3 x extrahiert und die organische Phase getrocknet und einrotiert.
Ausbeute: 2,1 g (64 % der Theorie),
Schmelzpunkt: 116-117°C
Analog werden hergestellt:

(1) 1-[3-(4-Cyano-phenyl)-propionyl]-6-(N-ethoxycarbonylmethyl-n-butylsulfonylamino)-1,2,3,4-tetrahydro-chinolin Hergestellt aus der gemäß Beispiel 9(4) hergestellten Verbindung
Ausbeute: 77 % der Theorie,
Schmelzpunkt: Öl

(2) 1-[(4-Cyano-phenoxy)-acetyl]-6-(N-ethoxycarbonylmethyl-1-naphthalinsulfonylamido)-1,2,3,4-tetrahydro-chinolin Hergestellt aus der gemäß Beispiel 9(27) hergestellten Verbindung
Ausbeute: 77 % der Theorie,

| $C_{32}H_{29}N_3O_6S$ (583,61) | | | |
|---|---|---|---|
| Ber. | C 65.85 | H 5,00 | N 7,19 |
| Gef: | 65,31 | 5,15 | 7,02 |

(3) 6-[N-(3-Methoxycarbonylpropyl)-naphth-1-yl-sulfonylamido]-1-[3-(4-benzyloxycarbonylamidino-phenyl)-propionyl]-1,2,3,4-tetrahydro-chinolin
Hergestellt aus 6-(Naphth-1-yl-sulfonylamido)-1-[3-(4-benzyloxycarbonylamidino-phenyl)-propionyl]-1,2,3,4-tetrahydro-chinolin (siehe Beispiel 32) und 4-Brombuttersäuremethylester Ausbeute: 79 % der Theorie,

(4) 6-[N-(2-Ethoxycarbonylethyl)-naphth-1-yl-sulfonylamido]-1-[3-(4-benzyloxycarbonylamidino-phenyl)-propionyl]-1,2,3,4-tetrahydro-chinolin
Hergestellt aus 6-(Naphth-1-yl-sulfonylamido)-1-[3-(4-benzyloxycarbonylamidino-phenyl)-propionyl]-1,2,3,4-tetrahydro-chinolin (siehe Beispiel 32) und 2-Brompropionsäureethylester Ausbeute: 72 % der Theorie,
$R_f$-Wert: 0,24 (Kieselgel; Methylenchlorid/Essigester = 8:2)

(5) 6- [N-Methyl- (naphth-1-yl-sulfonyl) -amido] -1- [3- (4-benzyloxycarbonylamidino-phenyl)-propionyl]-1,2,3,4-tetrahydro-chinolin
Hergestellt aus 6-[(Naphth-1-yl-sulfonyl)-amzdo]-1-[3-(4-benzyloxycarbonylamidino-phenyl)-propionyl)-1,2,3,4-tetrahydrochinolin und Methyliodid
Ausbeute: 68 % der Theorie,
$R_f$-Wert: 0,57 (Kieselgel; Methylenchlorid/Essigester = 7:3)

(6) 6-[N-Benzyl-(naphth-1-yl-sulfonyl)-amido]-1-[3-(4-benzyloxycarbonylamidino-phenyl)-propionyl]-1,2,3,4-tetrahydro-chinolin
Hergestellt aus 6-[(Naphth-1-yl-sulfonyl)-amido]-1-[3-(4-benzyloxycarbonylamidino-phenyl)-propionyl]-1,2,3,4-tetrahydrochinolin und Benzylbromid
Ausbeute: 72 % der Theorie,
$R_f$-Wert: 0,44 (Kieselgel; Methylenchlorid/Essigester = 7:3)

(7) 6-[N-Ethoxycarbonylmethyl-(naphth-1-yl-sulfonyl)-amido]-1-[3-(4-benzyloxycarbonylamidino-phenyl)-propionyl]-1,2,3,4-tetrahydro-chinolin
Hergestellt aus 6-[(Naphth-1-yl-sulfonyl)-amido]-1-[3-(4-benzyloxycarbonylamidino-phenyl)-propionyl]-1,2,3,4-tetrahydrochinolin und Bromessigsäureethylester
Ausbeute: 83 % der Theorie,

(8) 1-[3-(4-Benzyloxycarbonylamidino-phenyl)-propionyl]-6-[N-(naphth-1-yl-sulfonyl)-N,N-(di(methoxycarbonylmethyl)-aminocarbonylmethyl)-amino]-1,2,3,4-tetrahydro-chinolin
Hergestellt aus der gemäß Beispiel 32 hergestellten Verbindung und Bromacetylmethoxycarbonylmethylaminoessigsäuremethylester Ausbeute: 59 % der Theorie,
$R_f$-Wert: 0,27 (Kieselgel; Essigester/Petrolether = 4:1)

Beispiel 8

1-[3-(4-Cyanophenyl)-propionyl]-6-methylamino-1,2,3,4-tetrahydro-chinolin

10,5 g (34 mMol) 1-(3-Cyanophenyl-propionyl)-6-amino-1,2,3,4-tetrahydro-chinolin werden mit 40 ml Triethylorthoformiat und 1 ml Trifluoressigsäure 6 Stunden gekocht und dann einrotiert. Den Rückstand nimmt man in 50 ml Ethanol auf und gibt bei 0°C 1,45 g Natriumcyanborhydrid in Portionen zu. Diese Lösung läßt man bei Raumtemperatur über Nacht rühren und erhitzt dann 4 Stunden am Rückfluß. Schließlich wird mit Eiswasser verdünnt und mit Salzsäure sauer gestellt. Anschließend neutralisiert man mit Ammoniak, extrahiert mit Methylenchlorid, trocknet die organische Phase und dampft sie ein. Der Rückstand wird über Kieselgel mit Methylenchlorid/Essigester 8:2 chromatographiert.
Ausbeute: 5,4 g (49 % der Theorie),
Schmelzpunkt: 120°C

| $C_{20}H_{21}N_3O$ (319,40) | | | |
|---|---|---|---|
| Ber. | C 75,20 | H 6,62 | N 13,15 |
| Gef: | 75,02 | 6,73 | 12,98 |

Beispiel 9

1-[3-(4-Cyano-phenyl)propionyl]-6-phenylsulfonamido-1,2,3,4-tetrahydro-chinolin

4,6 g 3-(4-Cyano-phenyl)propionsäure werden mit 2,8 g N-Methyl-morpholin in 120 ml Tetrahydrofuran gelöst und auf -35°C abgekühlt. Zu dieser Lösung gibt man 3,6 ml Chlorameisensäureisobutylester, setzt das Rühren während einer halben Stunde fort und gibt dann bei -40°C 7,2 g 6-Phenylsulfonamido-1,2,3,4-tetrahydro-chinolin (siehe Beispiel IV) zu. Nach 2 Stunden läßt man die Lösung langsam auf Raumtemperatur erwärmen und rührt weiter über Nacht. Dann wird eingeengt und der Rückstand wird in Essigester und Wasser aufgenommen. Die organische Phase wird nochmals mit Wasser gewaschen, getrocknet und eingeengt. Das verbleibende Öl wird über eine Kieselgelsäule mit Essigester/Petrolether (7:3) chromatographiert. Ausbeute: 10,3 g (92,4 % der Theorie),

Schmelzpunkt: 87-89°C

| $C_{25}H_{23}N_3O_3S$ (445,54) | | | | |
|---|---|---|---|---|
| Ber. | C 67,40 | H 5,20 | N 9,43 | S 7,20 |
| Gef. | 67,47 | 5,61 | 9,09 | 7,38 |

Analog werden hergestellt:

(1) 1-[3-(4-Cyano-phenyl)propionyl]-6-(2-naphthyl-sulfonamido)-1,2,3,4-tetrahydro-chinolin
Hergestellt aus der gemäß Beispiel IV(1) hergestellten Verbindung
Ausbeute: 68 % der Theorie,
Schmelzpunkt: 70°C

| $C_{29}H_{25}N_3O_3S$ (495,60) | | | |
|---|---|---|---|
| Ber. | C 70,28 | H 5,08 | N 8,48 |
| Gef. | 70,42 | 5,30 | 8,21 |

(2) 1-[3-(4-Cyano-phenyl)propionyl]-6-(l-naphthyl-sulfonamido)-1,2,3,4-tetrahydro-chinolin
Hergestellt aus der gemäß Beispiel IV(2) hergestellten Verbindung
Ausbeute: 14 % der Theorie,
$R_f$-wert: 0,56 (Kieselgel; Toluol/Essigester = 1:1)

(3) 1-[3-(4-Cyano-phenyl)propionyl]-6-(4-fluor-phenylsulfonamido)-1,2,3,4-tetrahydro-chinolin
Hergestellt aus der gemäß Beispiel IV(3) hergestellten Verbindung
Ausbeute: 64 % der Theorie,
Schmelzpunkt: 62-64°C

| $C_{25}H_{22}FN_3O_3S$ (463,53) | | | |
|---|---|---|---|
| Ber. | C 64,78 | H 4,78 | N 9,07 |
| Gef. | 65,00 | 5,16 | 8,73 |

(4) 6-(n-Butylsulfonamido)-1-[3-(4-cyano-phenyl)propionyl]-1,2,3,4-tetrahydro-chinolin
Hergestellt aus der gemäß Beispiel IV(4) hergestellten Verbindung
Ausbeute: 53 % der Theorie,
Schmelzpunkt: 138-140°C

(5) 1-[3-(4-Cyano-phenyl)propionyl)-5-phenylsulfonamido-1,2,3,4-tetrahydro-chinolin
Hergestellt aus der gemäß Beispiel IV(7) hergestellten Verbindung
$R_f$-Wert: 0,32 (Kieselgel; Toluol/Essigester = 6:4)

(6) 1-[3-(4-Cyano-phenyl)propionyl]-7-phenylsulfonamido-1,2,3,4-tetrahydro-chinolin
Hergestellt aus der gemäß Beispiel IV(8) hergestellten Verbindung
Schmelzpunkt: 74-76°C

(7) 7-Benzylsulfonamido-1-[3-(4-cyano-phenyl)propionyl]-1,2,3,4-tetrahydro-chinolin
Hergestellt aus der gemäß Beispiel IV(6) hergestellten Verbindung
Schmelzpunkt: 177-180°C

(8) 1-[3-(4-Cyano-phenyl)propionyl]-6-(N-methyl-phenylsulfonamido)-1,2,3,4-tetrahydro-chinolin
Hergestellt aus der gemäß Beispiel IV(5) hergestellten Verbindung
Ausbeute: 74 % der Theorie,
Schmelzpunkt: 114-116°C

| $C_{26}H_{25}N_3O_3S$ (459,57) | | | | |
|---|---|---|---|---|
| Ber. | C 67,95 | H 5,48 | N 9,14 | S 6,98 |
| Gef. | 68,02 | 5,56 | 9,25 | 7,04 |

(9) 1-[3-(4-Cyano-phenyl)propionyl]-6-phenylacetylamino-1,2,3,4-tetrahydro-chinolin
Hergestellt aus der gemäß Beispiel IV(9) hergestellten Verbindung
Ausbeute: 81 % der Theorie,

(10) 1-[3-(4-Cyano-phenyl)propionyl]-6-[N-(ethoxycarbonylmethyl)-phenylsulfonamido]-1,2,3,4-tetrahydro-chinolin Hergestellt aus der gemäß Beispiel 6 hergestellten Verbindung Schmelzpunkt: 148-150°C

| $C_{29}H_{29}N_3O_5S$ (531,65) | | | | |
|---|---|---|---|---|
| Ber. | C 65,52 | H 5,50 | N 7,90 | S 6,03 |
| Gef. | 65,34 | 5,54 | 7,86 | 6,03 |

(11) l-[3-(4-Cyano-phenyl)propionyl]-6-[N-(2-phenylethyl)-phenylsulfonamido]-1,2,3,4-tetrahydro-chinolin Hergestellt aus der gemäß Beispiel IV(15) hergestellten Verbindung

(12) 6-Benzylsulfonamido-1-[3-(4-cyano-phenyl)propionyl]-1,2,3,4-tetrahydro-chinolin
Hergestellt aus der gemäß Beispiel IV(11) hergestellten Verbindung
Schmelzpunkt: 161-163°C

| $C_{26}H_{25}N_3O_3S$ (459,57) | | | |
|---|---|---|---|
| Ber. | C 67,95 | H 5,48 | N 9,14 |
| Gef. | 67,93 | 5,56 | 9,07 |

(13) 6-Benzoylamino-1-[3-(4-cyano-phenyl)propionyl]-1,2,3,4-tetrahydro-chinolin
Hergestellt aus der gemäß Beispiel IV(12) hergestellten Verbindung
Ausbeute: 83 % der Theorie,

| $C_{26}H_{23}N_3O_3$ (409,49) | | | |
|---|---|---|---|
| Ber. | C 76,26 | H 5,66 | N 10,26 |
| Gef. | 76,17 | 5,85 | 10,19 |

(14) 1-[3-(4-Cyano-phenyl)propionyl]-2-methyl-6-phenylsulfonamido-1,2,3,4-tetrahydro-chinolin
Hergestellt aus der gemäß Beispiel IV(10) hergestellten Verbindung
Ausbeute: 41 % der Theorie,

(15) 1-[3-(4-Cyano-phenyl)propionyl]-6-(4-chlor-phenylsulfonamido)-1,2,3,4-tetrahydro-chinolin
Hergestellt aus der gemäß Beispiel IV(17) hergestellten Verbindung

(16) l-[3-(4-Cyano-phenyl)propionyl]-6-(4-brom-phenylsulfonamido)-1,2,3,4-tetrahydro-chinolin
Hergestellt aus der gemäß Beispiel IV(18) hergestellten Verbindung

(17) 1-[3-(4-Cyano-phenyl)propionyl]-6-(3-chlor-phenylsulfonamido)-1,2,3,4-tetrahydro-chinolin
Hergestellt aus der gemäß Beispiel IV(19) hergestellten Verbindung

(18) 1-[3-(4-Cyano-phenyl)propionyl]-6-(3-brom-phenylsulfonamido)-1,2,3,4-tetrahydro-chinolin
Hergestellt aus der gemäß Beispiel IV(20) hergestellten Verbindung

(19) 1- [3- (4-Cyano-phenyl)propionyl] -6- (4-methyl-phenylsulfonamido)-1,2,3,4-tetrahydro-chinolin
Hergestellt aus der gemäß Beispiel IV(21) hergestellten Verbindung

(20) 1-[3-(4-Cyano-phenyl)propionyl]-6-(4-methoxy-phenylsulfonamido)-1,2,3,4-tetrahydro-chinolin
Hergestellt aus der gemäß Beispiel IV(22) hergestellten Verbindung

(21) 1- [3- (4-Cyano-phenyl) propionyl] -6- [N- (2-phenylethyl) -phenylsulfonamido]-1,2,3,4-tetrahydro-chinolin
Hergestellt aus der gemäß Beispiel IV(11) hergestellten Verbindung
Ausbeute: 69 % der Theorie,

(22) 1-[3-(4-Cyano-phenyl)-propionyl)-6-(4-methyl-piperidinocarbonyl)-1,2,3,4-tetrahydro-chinolin
Hergestellt aus der gemäß Beispiel IV(29) hergestellten Verbindung
Ausbeute: 67 % der Theorie
$R_f$-Wert: 0,73 (Kieselgel; Methylenchlorid/Essigsäureethylester = 8:2)

(23) 1-[3-(4-Cyano-phenyl)-propionyl]-6-(morpholinocarbonyl)-1,2,3,4-tetrahydro-chinolin
Hergestellt aus der gemäß Beispiel IV(22) hergestellten Verbindung
Ausbeute: 37 % der Theorie
$R_f$-Wert: 0,61 (Kieselgel; Methylenchlorid/Essigsäureethylester = 8:2)

(24) 1- [3- (3-Cyanophenyl)propionyl] -6-phenylsulfonamido-1,2,3,4-tetrahydrochinolin
Hergestellt aus der gemäß Beispiel IV hergestellten Verbindung Ausbeute: 78 % der Theorie
Schmelzpunkt: 130-133°C

(25) 1- [2- (4-Cyano-phenyloxy) -acetyl] -6-phenylsulfonamido-1,2,3,4-tetrahydro-chinolin
Hergestellt aus der gemäß Beispiel IV hergestellten Verbindung und 4-Cyano-phenoxyessigsäure
Ausbeute: 68 % der Theorie
Schmelzpunkt: 76-78°C

(26) 1-[2-((4-Cyano-phenyl)-methylamino)-acetyl]-6-benzolsulfonamido-1,2,3,4-tetrahydro-chinolin
Hergestellt aus der gemäß Beispiel IV hergestellten Verbindung Ausbeute: 82 % der Theorie
Schmelzpunkt: 193-194°C

(27) 1- [2- (4-Cyano-phenyloxy) -acetyl] -6- (1-naphthylsulfonamido)-1,2,3,4-tetrahydro-chinolin
Hergestellt aus der gemäß Beispiel IV(2) hergestellten Verbindung
Ausbeute: 41 % der Theorie

(28) 1- [3- (4-Cyano-2-nitro-phenyl) -propionyl] -6-phenylsulfonylamido-1,2,3,4-tetrahydro-chinolin
$R_f$-Wert: 0,56 (Kieselgel; Methylenchlorid/Methanol = 9:1)

(29) 1-(4-Cyano-benzoyl)-6-phenylsulfonamido-1,2,3,4-tetrahydro-chinolin
Hergestellt aus der gemäß Beispiel IV hergestellten Verbindung und 4-Cyano-benzosäure
Ausbeute: 71 % der Theorie,
Schmelzpunkt: 132-134°C

(30) 1-[3-(4-Cyano-3-methyl-phenyl)-propionyl]-6-phenylsulfonamido-1,2,3,4-tetrahydro-chinolin
Hergestellt aus der gemäß Beispiel IV hergestellten Verbindung und 4-Cyano-3-methyl-phenylpropionsäure
$R_f$-Wert: 0,40 (Kieselgel; Methylenchlorid/Ethanol = 40:1)

(31) 1-[3-(4-Cyano-3-fluor-phenyl)-propionyl]-6-phenylsulfonamido-1,2,3,4-tetrahydro-chinolin
Hergestellt aus der gemäß Beispiel IV hergestellten Verbindung und 4-Cyano-3-fluorphenylpropionsäure
Ausbeute: 36 % der Theorie,
Schmelzpunkt: 138-140°C

(32) 1- [3- (2-Cyano-pyridin-5-yl) -propionyl] -6-phenylsulfonamido-1,2,3,4-tetrahydro-chinolin
Hergestellt aus der gemäß Beispiel IV hergestellten Verbindung
Ausbeute: 73 % der Theorie,
$R_f$-Wert: 0,35 (Kieselgel; Methylenchlorid/Methanol = 40:1)

(33) 1-[3-(4-Cyano-phenyl)-acryloyl]-6-phenylsulfonamido-1,2,3,4-tetrahydro-chinolin
Ausbeute: 74 % der Theorie,

Schmelzpunkt: 175 -180°C

(34) 1-[3-(4-Cyano-phenyl)-propionyl]-6-piperidinocarbonyl-1,2,3,4-tetrahydro-chinolin
Hergestellt aus der gemäß Beispiel IV(23) hergestellten Verbindung
Ausbeute: 97 % der Theorie,
$R_f$-Wert: 0,38 (Kieselgel; Essigsäureethylester)

(35) 1-[3-(4-Cyano-phenyl)-propionyl]-6-benzylamidocarbonyl-1,2,3,4-tetrahydro-chinolin
Hergestellt aus der gemäß Beispiel IV(24) hergestellten Verbindung
Ausbeute: 60 % der Theorie,
$R_f$-Wert: 0,24 (Kieselgel; Toluol/Essigester = 4:6)

(36) 1- [3- (4-Cyano-phenyl) -propionyl] -6- (N-methyl-phenyl-aminocarbonyl)-1,2,3,4-tetrahydro-chinolin
Hergestellt aus der gemäß Beispiel IV(25) hergestellten Verbindung
Ausbeute: 82 % der Theorie,
$R_f$-Wert: 0,72 (Kieselgel; Essigsäureethylester)

(37) 1-[3-(4-Cyano-phenyl)-propionyl]-6-(diethylaminocarbonyl)-1,2,3,4-tetrahydro-chinolin
Hergestellt aus der gemäß Beispiel IV(26) hergestellten Verbindung
Ausbeute: 55 % der Theorie,
$R_f$-Wert: 0,21 (Kieselgel; Methylenchlorid/Essigsäureethylester = 8:2)

(38) 1-[3-(4-Cyano-phenyl)-propionyl]-6-(3,5-dimethyl-piperidinocarbonyl)-1,2,3,4-tetrahydro-chinolin
Hergestellt aus der gemäß Beispiel IV(27) hergestellten Verbindung
Ausbeute: 96 % der Theorie,
$R_f$-Wert: 0,14 (Kieselgel; Methylenchlorid/Essigsäureethylester = 8:2)

(39) 1-[3-(4-Cyano-phenyl)-propionyl]-6-methoxycarbonyl-1,2,3,4-tetrahydro-chinolin
Hergestellt aus 1,2,3,4-Tetrahydrochinolin-6-carbonsäuremethylester
Ausbeute: 65 % der Theorie

Beispiel 10

1-[3-(4-Cyano-phenyl)propionyl]-6-(4-amino-3,5-dichlor-phenylsulfonamido)-1,2,3,4-tetrahydro-chinolin

1,0 g 6-Amino-1-[3-(4-cyano-phenyl)propionyl]-1,2,3,4-tetrahydro-chinolin (siehe Beispiel 5) werden in 8 ml Pyridin gelöst, mit 1 g 4-Amino-3,5-dichlor-phenylsulfonsäurechlorid in Portionen versetzt und dann 40 Minuten bei 100°C erhitzt. Danach wird das Lösungsmittel abgezogen, der Rückstand mit 1N Salzsäure verrieben und mit Methylenchlorid extrahiert. Die organische Phase wird über Natriumsulfat getrocknet und eingeengt.
Ausbeute: 1,5 g (86 % der Theorie),
Schmelzpunkt: 183-184°C

| $C_{25}H_{22}N_4Cl_2O_3S$ (529,45) | | | |
|---|---|---|---|
| Ber. | C 56,72 | H 4,19 | N 10,58 |
| Gef. | 56,54 | 4,25 | 10,44 |

Analog werden hergestellt:

(1) 1- [3- (4-Cyano-phenyl) propionyl] -6- (5-dimethylamino-naphth-1-yl-sulfonamido)-1,2,3,4-tetrahydro-chinolin
Hergestellt aus der gemäß Beispiel 5 hergestellten Verbindung
Ausbeute: 95 % der Theorie,
Schmelzpunkt: 78-80°C

| $C_{31}H_{30}N_4O_3S$ (538,67) | | | |
|---|---|---|---|
| Ber. | C 69,12 | H 5,61 | N 10,43 |
| Gef. | 70,11 | 5,82 | 9,79 |

(2) 1-[3-(4-Cyano-phenyl)propionyl]-6-propylsulfonamido-1,2,3,4-tetrahydro-chinolin
Hergestellt aus der gemäß Beispiel 5 hergestellten Verbindung
Ausbeute: 95 % der Theorie,
Schmelzpunkt: 152-153°C

| $C_{22}H_{25}N_3O_3S$ (411,52) | | | |
|---|---|---|---|
| Ber. | C 64,21 | H 6,12 | N 10,21 |
| Gef. | 64,05 | 6,10 | 10,02 |

(3) 6-(5-Chlor-thien-2-yl-sulfonamido)-1-[3-(4-cyano-phenyl)-propionyl]-1,2,3,4-tetrahydro-chinolin
Hergestellt aus der gemäß Beispiel 5 hergestellten Verbindung
Ausbeute: 69 % der Theorie,
Schmelzpunkt: 153-154°C

| $C_{23}H_{20}ClN_3O_3S_2$ (486,01) | | | | |
|---|---|---|---|---|
| Ber. | C 56,84 | H 4,14 | N 8,64 | Cl 7,29 |
| Gef. | 56,88 | 4,24 | 8,58 | 7,08 |

(4) 1-[3-(4-Cyano-phenyl)propionyl]-6-isopropylsulfonamido-1,2,3,4-tetrahydro-chinolin
Hergestellt aus der gemäß Beispiel 5 hergestellten Verbindung
Ausbeute: 37 % der Theorie,
Schmelzpunkt: 151-152°C

| $C_{22}H_{25}N_3O_3S$ (411,52) | | | |
|---|---|---|---|
| Ber. | C 64,21 | H 6,12 | N 10,21 |
| Gef. | 64,70 | 6,25 | 9,89 |

(5) 6-(3-Chlor-propylsulfonamido)-1-[3-(4-cyano-phenyl)propionyl]-1,2,3,4-tetrahydro-chinolin
Hergestellt aus der gemäß Beispiel 5 hergestellten Verbindung
Ausbeute: 55 % der Theorie,

| $C_{22}H_{24}ClN_3O_3S$ (445,97) | | | | |
|---|---|---|---|---|
| Ber. | C 59,25 | H 5,42 | N 9,42 | Cl 7,95 |
| Gef. | 58,68 | 5,44 | 9,26 | 8,32 |

(6) 1-[3-(4-Cyano-phenyl)propionyl]-5-phenylsulfonamido-2,3-dihydro-indol
Hergestellt aus der gemäß Beispiel 2 hergestellten Verbindung
Ausbeute: 78 % der Theorie,
$R_f$-Wert: 0,46 (Kieselgel; Toluol/Essigester = 6:4)

(7) 1-[3-(4-Cyano-phenyl)propionyl]-7-phenylsulfonamido-2,3,4,5-tetrahydro-1H-benzo[b]azepin
Hergestellt aus der gemäß Beispiel 2(2) hergestellten Verbindung
Ausbeute: 57 % der Theorie,
Schmelzpunkt: ab 148°C
$R_f$-Wert: 0,30 (Kieselgel; Toluol/Essigester = 13:7)

(8) 1-[3-(4-Cyano-phenyl)propionyl]-3-methyl-6-phenylsulfonamido-1,2,3,4-tetrahydro-chinolin
Hergestellt aus der gemäß Beispiel 2(1) hergestellten Verbindung
Ausbeute: 79 % der Theorie,
$R_f$-Wert: 0,35 (Kieselgel; Toluol/Essigester = 7:3)

(9) 1-[3-(4-Cyano-phenyl)propionyl]-4-methyl-6-phenylsulfonamido-1,2,3,4-tetrahydro-chinolin
Hergestellt aus der gemäß Beispiel 2(3) hergestellten Verbindung
Ausbeute: 89 % der Theorie,

$R_f$-Wert: 0,21 (Kieselgel; Toluol/Essigester = 7:3)

(10) 1- [3- (4-Cyano-phenyl)propionyl] -6- (3-trifluoromethylphenylsulfonamido)-1,2,3,4-tetrahydro-chinolin
Hergestellt aus der gemäß Beispiel 5 hergestellten Verbindung
Ausbeute: 72 % der Theorie,
$R_f$-Wert: 0,55 (Kieselgel; Methylenchlorid/Ethanol = 4:1)

(11) 1-[3-(4-Cyano-phenyl)-propionyl]-6-(2,5-dichloro-phenylsulfonamido)-1,2,3,4-tetrahydro-chinolin
Ausbeute: 89 % der Theorie,
Schmelzpunkt: 219-220°C

| $C_{25}H_{21}Cl_2N_3O_3S$ (514,43) | | | |
|---|---|---|---|
| Ber. | C 58,37 | H 4,11 | N 8,17 |
| Gef:: | 58,10 | 4,33 | 8,05 |

(12) l-[3-(4-Cyano-phenyl)-propionyl]-6-(2,3,5,6-tetramethylphenylsulfonamido)-1,2,3,4-tetrahydro-chinolin
Hergestellt aus der gemäß Beispiel 5 hergestellten Verbindung
Ausbeute: 89 % der Theorie,
Schmelzpunkt: 228°C

(13) 1-[3-(4-Cyano-phenyl)-propionyl]-6-(2,4,6-trimethyl-phenylsulfonamido)-1,2,3,4-tetrahydro-chinolin
Hergestellt aus der gemäß Beispiel 5 hergestellten Verbindung
Ausbeute: 86 % der Theorie,
Schmelzpunkt: 182°C

| $C_{28}H_{29}N_3O_3S$ (501,65) | | | |
|---|---|---|---|
| Ber. | C 68,97 | H 5,99 | N 8,62 |
| Gef:: | 68,91 | 6,08 | 8,68 |

(14) 1- [3- (4-Cyano-phenyl) -propionyl] -6- (isochinolin-5-yl-sulfonamido)-1,2,3,4-tetrahydro-chinolin
Hergestellt aus der gemäß Beispiel 5 hergestellten Verbindung
Ausbeute: 89 % der Theorie,
Schmelzpunkt: 210-211°C
$R_f$-Wert: 0,46 (Kieselgel; Methylenchlorid/Ethanol = 20:1)

(15) 1- [3- (4-Cyano-phenyl) -propionyl] -6- (cyclopropylsulfonamido)-1,2,3,4-tetrahydro-chinolin
Hergestellt aus der gemäß Beispiel 5 hergestellten Verbindung
Ausbeute: 74 % der Theorie,
Schmelzpunkt: 138-139°C

(16) 1-[3-(4-Cyano-phenyl)-propionyl]-6-(benzimidazol-5-ylsulfonamido)-1,2,3,4-tetrahydro-chinolin
Hergestellt aus der gemäß Beispiel 5 hergestellten Verbindung
Ausbeute: 36 % der Theorie,
$R_f$-Wert: 0,29 (Kieselgel; Methylenchlorid/Ethanol = 20:1)

(17) 1-[3-(4-Cyano-phenyl)-propionyl]-6-(cyclohexylsulfonamido)-1,2,3,4-tetrahydro-chinolin
Hergestellt aus der gemäß Beispiel 5 hergestellten Verbindung
Ausbeute: 57 % der Theorie,
Schmelzpunkt: 159-163°C

(18) l-[3-(4-Cyano-phenyl)-propionyl]-6-(3-tolylsulfonamido)-1,2,3,4-tetrahydro-chinolin
Hergestellt aus der gemäß Beispiel 5 hergestellten Verbindung
Ausbeute: 78 % der Theorie,
Schmelzpunkt: 130°C

| $C_{26}H_{25}N_3O_3S$ (459,57) | | | |
|---|---|---|---|
| Ber. | C 67,95 | H 5,48 | N 9,14 |
| Gef:: | 67,68 | 5,54 | 8,89 |

(19) 1-[3-(4-Cyano-phenyl)-propionyl]-6-(4-methoxy-benzolsulfonamido)-1,2,3,4-tetrahydro-chinolin
Hergestellt aus der gemäß Beispiel 5 hergestellten Verbindung
Ausbeute: 87 % der Theorie,
Schmelzpunkt: Zersetzung ab 65°C

| $C_{26}H_{25}N_3O_4S$ (475,57) | | | |
|---|---|---|---|
| Ber. | C 65.66 | H 5,29 | N 8,83 |
| Gef:: | 65,75 | 5,61 | 8,64 |

(20) 1- [3- (4-Cyano-phenyl) -propionyl] -6- (N-ethoxycarbonylmethyl-chinolin-8-sulfonylamido)-1,2,3,4-tetrahydro-chinolin Hergestellt aus 1-[3-(4-Cyano-phenyl)-propionyl]-6-(N-ethoxycarbonylmethylamino)-1,2,3,4-tetrahydro-chinolin (siehe Beispiel 6) und Chinolinsulfonsäurechlorid
Ausbeute: 57 % der Theorie

(21) 1-[3-(4-Cyano-phenyl)-propionyl]-6-[N-ethoxycarbonylmethyl-(2-naphthylsulfonylamido]-1,2,3,4-tetrahydro-chinolin Hergestellt aus 1- [3- (4-Cyano-phenyl) -propionyl] -6-ethoxycarbonylamino-1,2,3,4-tetrahydro-chinolin (siehe Beispiel 6) und Naphthalin-2-sulfonsäurechlorid
Ausbeute: 69 % der Theorie

(22) 1-[3-(4-Cyano-phenyl)-propionyl]-6-(N-benzyl-methansulfonamido)-1,2,3,4-tetrahydro-chinolin
Hergestellt aus der gemäß Beispiel 14 hergestellten Verbindung und Methansulfonsäurechlorid
Ausbeute: 51 % der Theorie,
$R_f$-Wert: 0,34 (Kieselgel; Toluol/Essigsäureethylester = 1:1)

(23) 1-[3-(4-Cyano-phenyl)-propionyl]-6-[N-(ethoxycarbonylmethylamino)-phenylmethansulfonamido]-1,2,3,4-tetrahydro-chinolin
Hergestellt aus der gemäß Beispiel 6 hergestellten Verbindung und Phenylmethansulfonsäurechlorid
Ausbeute: 78 % der Theorie,

Beispiel 11

1-[3-(4-Cyano-phenyl)-propionyl]-6-(N-benzoyl-ethoxycarbonylmethylamino)-1,2,3,4-tetrahydro-chinolin

1,57 g 1-[3-(4-Cyano-phenyl)-propionyl]-6-(N-ethoxycarbonylmethyl-amino)-1,2,3,4-tetrahydro-chinolin (siehe Beispiel 6) und 1 g Triethylamin werden in 20 ml Methylenchlorid gelöst und 0,7 g Benzoylchlorid werden unter Eiskühlung langsam zugetropft. Anschließend läßt man die Lösung über Nacht bei Raumtemperatur rühren. Dann engt man die Lösung ein, nimmt den Rückstand in Wasser auf und extrahiert mit Essigester. Die organische Phase wird getrocknet und einrotiert. Der Rückstand wird über eine Kieselgelsäule filtriert.
Ausbeute: 1,7 g (85 % der Theorie),
**[0067]** Analog werden hergestellt:

(1) 1-[3-(4-Cyano-phenyl)-propionyl]-6-[N-ethoxycarbonylmethyl-(naphtho-1-yl)-amino]-1,2,3,4-tetrahydro-chinolin
Ausbeute: 82 % der Theorie

(2) 1-[3-(4-Cyano-phenyl)-propionyl]-6-(N-benzoyl-methylamino)-1,2,3,4-tetrahydro-chinolin
Hergestellt aus der gemäß Beispiel 8 hergestellten Verbindung und Benzoylchlorid
Ausbeute: 83 % der Theorie,
$R_f$-Wert: 0,37 (Kieselgel; Toluol/Essigsäureethylester = 1:1)

(3) 1-[3-(4-Cyano-phenyl)-propionyl]-6-[N-(4-chlor-benzoyl)-methylamino]-1,2,3,4-tetrahydro-chinolin

Hergestellt aus der gemäß Beispiel 8 hergestellten Verbindung und 4-Chlorbenzoylchlorid
Ausbeute: 91 % der Theorie,

| $C_{27}H_{24}N_3O_2S$ (475,57) | | | |
|---|---|---|---|
| Ber. | C 70,81 | H 5,28 | N 9,17 |
| Gef:: | 71,52 | 5,51 | 8,50 |

(4) 1-[3-(4-Cyano-phenyl)-propionyl]-6-(N-naphtho-1-yl-methylamino)-1,2,3,4-tetrahydro-chinolin
Hergestellt aus der gemäß Beispiel 8 hergestellten Verbindung und Naphthalin-1-carbonsäurechlorid
Ausbeute: 94 % der Theorie,

| $C_{31}H_{27}N_3O_2$ (473,57) | | | |
|---|---|---|---|
| Ber. | C 78,62 | H 5,74 | N 8,87 |
| Gef:: | 78,30 | 6,03 | 8,52 |

(5) 1- [3- (4-Cyano-phenyl) -propionyl] -6- (N-naphtho-2-yl-methylamino)-1,2,3,4-tetrahydro-chinolin
Hergestellt aus der gemäß Beispiel 8 hergestellten Verbindung und Naphthalin-2-carbonsäurechlorid
Ausbeute: 93 % der Theorie,

| $C_{31}H_{27}N_3O_2$ (473,57) | | | |
|---|---|---|---|
| Ber. | C 78,62 | H 5,74 | N 8,87 |
| Gef:: | 78,62 | 5,88 | 8,19 |

(6) 1- [3- (4-Cyano-phenyl) -propionyl] -6- (N-butyryl-methylamino)-1,2,3,4-tetrahydro-chinolin
Hergestellt aus der gemäß Beispiel 8 hergestellten Verbindung und Buttersäurechlorid
Ausbeute: 98 % der Theorie,

| $C_{24}H_{27}N_3O_2$ (389,49) | | | |
|---|---|---|---|
| Ber. | C 74,00 | H 6,98 | N 10,78 |
| Gef:: | 74,09 | 7,01 | 10,43 |

(7) 1-[3-(4-Cyano-2-acetylamino-phenyl)-propionyl-6-phenylsulfonylamido-1,2,3,4-tetrahydro-chinolin
Hergestellt aus der gemäß Beispiel 2(4) hergestellten Verbindung und Acetylchlorid
Ausbeute: 90 % der Theorie,
$R_f$-Wert: 0,58 (Kieselgel; Methylenchlorid/Methanol = 9:1)

(8) 1-[3-(4-Cyano-2-(2-ethoxycarbonylethylcarbonylamino)-propionyl]-6-phenylsulfonamido-1,2,3,4-tetrahydro-chinolin
Hergestellt aus der gemäß Beispiel 2(4) hergestellten Verbindung und Bernsteinsäureethylesterchlorid
$R_f$-Wert: 0,63 (Kieselgel; Methylenchlorid/Methanol = 9:1)

(9) 1-[3-(4-Cyano-phenyl)-propionyl]-6-(N-benzyl-acetylamino)-1,2,3,4-tetrahydro-chinolin
Hergestellt aus der gemäß Beispiel 14 hergestellten Verbindung und Acetylchlorid
Ausbeute: 91 % der Theorie,
$R_f$-Wert: 0,27 (Kieselgel; Toluol/Essigsäureethylester = 1:1)

(10) 1-[3-(4-Cyano-phenyl)-propionyl]-6-(N-benzyl-N-pentanoylamino)-1,2,3,4-tetrahydro-chinolin
Hergestellt aus der gemäß Beispiel 14 hergestellten Verbindung und Valeriansäurechlorid
Ausbeute: 83 % der Theorie,
$R_f$-Wert: 0,57 (Kieselgel; Toluol/Essigsäureethylester = 1:1)

(11) 1- [3- (4-Cyano-phenyl) -propionyl] -6- [N-benzyl-N- (2-ethoxycarbonylethylcarbonyl)-amino]-1,2,3,4-tetrahydro-chinolin Hergestellt aus der gemäß Beispiel 14 hergestellten Verbindung und Bernsteinsäureethylesterchlorid
Ausbeute: 86 % der Theorie,

$R_f$-Wert: 0,19 (Kieselgel; Methylenchlorid/Essigsäureethyl ester = 8:2)

(12) 1-[3-(4-Cyano-phenyl)-propionyl]-6-(2-oxo-pyrrolidino)-1,2,3,4-tetrahydro-chinolin
Hergestellt aus 6-Amino-1-[3-(4-cyano-phenyl)propionyl]-1,2,3,4-tetrahydro-chinolin und 4-Chlor-buttersäurechlorid
Ausbeute: 77 % der Theorie,
$R_f$-Wert: 0,40 (Kieselgel; Essigsäureethylester)

(13) 1-[3-(4-Cyano-phenyl)-propionyl]-6-(2-oxo-piperidino)-1,2,3,4-tetrahydro-chinolin
Hergestellt aus 6-Amino-1-[3-(4-cyano-phenyl)propionyl]-1,2,3,4-tetrahydro-chinolin und 5-Chlor-pentansäurechlorid
Ausbeute: 69 % der Theorie,
$R_f$-Wert: 0,11 (Kieselgel; Essigsäureethylester)

Beispiel 12

1-[3-(4-Cyano-phenyl)-propionyl]-6-(N-phenyl-butylaminocarbonyl)-1,2,3,4-tetrahydro-chinolin

0, 9 g (4 mMol) 1- [3- (4-Cyano-phenyl) -propionyl] -6-methoxycarbonyl-1,2,3,4-tetrahydro-chinolin (siehe Beispiel 9(39)) werden in 10 ml Dioxan gelöst und mit 10 ml 1 N Natronlauge bei 40°C 4 Stunde gerührt. Dann wird die Lösung mit Salzsäure neutralisiert, eingeengt und der dabei ausfallende Niederschlag wird abgesaugt und getrocknet. Das Produkt wird in 10 ml Thionylchlorid suspendiert und 3 Stunde am Rückfluß gekocht. Dann wird eingeengt und der Rückstand 10 ml Methylenchlorid aufgenommen und mit 0,93 ml Triethylamin und 0,6 g N-Phenylbutylamin in wenig Methylenchlorid versetzt. Nach 20 Minuten ist die Reaktion beendet und der Ansatz wird mit 10 ml 1N Natronlauge und dann 10 ml Wasser gewaschen. Dann wird die Lösung getrocknet und einrotiert und der Rückstand über eine Kieselgelsäule mit Toluol/Essigsäureethylester (8:2) chromatographiert.
Ausbeute: 1,35 g (70 % der Theorie),
$R_f$-Wert: 0,29 (Kieselgel; Toluol:Essigsäureethylester = 7:3)
Analog wurden hergestellt:

(1) 1-[3-(4-Cyano-phenyl)-propianyl]-6-[N-(4-chlor-phenyl)-methylaminocarbonyl]-1,2,3,4-tetrahydro-chinolin
Ausbeute: 75 % der Theorie,
$R_f$-Wert: 0,19 (Kieselgel; Toluol:Essigsäureethylester = 7:3)

(2) 1-[3-(4-Cyano-phenyl)-propionyl]-6-(N-phenyl-ethylaminocarbonyl)-1,2,3,4-tetrahydro-chinolin
Ausbeute: 71 % der Theorie,
$R_f$-Wert: 0,16 (Kieselgel; Toluol:Essigsäureethylester = 7:3)

(3) 1-[3-(4-Cyano-phenyl)-propionyl]-6-(diphenylaminocarbonyl)-1,2,3,4-tetrahydro-chinolin
Ausbeute: 34 % der Theorie,
$R_f$-Wert: 0,37 (Kieselgel; Toluol:Essigsäureethylester = 7:3)

(4) 1-[3-(4-Cyano-phenyl)-propionyl]-6-(N-phenyl-benzylaminocarbonyl)-1,2,3,4-tetrahydro-chinolin
Ausbeute: 29 % der Theorie,
$R_f$-Wert: 0,31 (Kieselgel; Toluol:Essigsäureethylester = 7:3)

(5) 1- [3- (4-Cyano-phenyl) -propionyl] -6- (N-methoxycarbonylmethyl-phenylaminocarbonyl)-1,2,3,4-tetrahydro-chinolin
Ausbeute: 66 % der Theorie,
$R_f$-Wert: 0,18 (Kieselgel; Toluol:Essigsäureethylester = 7:3)

(6) 1-[3-(4-Cyano-phenyl)-propionyl]-6-(N-cyclohexyl-methylaminocarbonyl)-1,2,3,4-tetrahydro-chinolin
Ausbeute: 88 % der Theorie,
$R_f$-Wert: 0,20 (Kieselgel; Toluol:Essigsäureethylester = 7:3)

(7) 1-[3-(4-Cyano-phenyl)-propionyl]-6-(N-ethoxycarbonylmethyl-cyclohexylaminocarbonyl)-1,2,3,4-tetrahydro-chinolin
Ausbeute: 34 % der Theorie,

$R_f$-Wert: 0,13 (Kieselgel; Toluol:Essigsäureethylester = 7:3)

(8) 1-[3-(4-Cyano-phenyl)-propionyl]-6-(2-methoxycarbonyl-pyrrolidinocarbonyl)-1,2,3,4-tetrahydro-chinolin
Ausbeute: 62 % der Theorie,
$R_f$-Wert: 0,12 (Kieselgel; Toluol:Essigsäureethylester = 1:1)

(9) 1-{1-[3-(4-Cyano-phenyl)-propionyl]-6-(2-methoxycarbonylpiperidinocarbonyl)-1,2,3,4-tetrahydro-chinolin
Ausbeute: 64 % der Theorie,
$R_f$-Wert: 0,22 (Kieselgel; Toluol:Essigsäureethylester = 1:1)

(10) 1-{1-[3-(4-Cyano-phenyl)-propionyl]-6-(3-ethoxycarbonylpiperidinocarbonyl)-1,2,3,4-tetrahydro-chinolin
Ausbeute: 75 % der Theorie,
$R_f$-Wert: 0,18 (Kieselgel; Toluol:Essigsäureethylester = 1:1)

(11) 1- [3- (4-Cyano-phenyl) -propionyl] -6- [N- (2-acetylaminoethyl)-phenylamidocarbonyl]-1,2,3,4-tetrahydro-chinolin
Ausbeute: 67 % der Theorie,
$R_f$-Wert: 0,27 (Kieselgel; Essigsäureethylester: Ethanol 19:1)

(12) 1- [3- (4-Cyano-phenyl) -propionyl] -6- [N- (N-benzyloxycarbonyl-N-phenyl-2-aminoethyl)-aminocarbonyl]-1,2,3,4-tetrahydrochinolin
Ausbeute: 73 % der Theorie,
$R_f$-Wert: 0,31 (Kieselgel; Toluol/Essigsäureethylester = 1:1)

Beispiel 13

1- [3- (4-Cyano-phenyl) -propionyl] -6- [N- (N-phenyl-2-aminoethyl) -amidocarbonyl] -1,2,3,4-tetrahydro-chinolin

Hergestellt aus 1-[3-(4-Cyano-phenyl)-propionyl]-6-[N-(N-benzyloxycarbonyl-N-phenyl-2-aminoethyl)-amidocarbonyl]-1,2,3,4-tetrahydro-chinolin (siehe Beispiel 12(12)) durch katalytische Reduktion analog Beispiel 25.
Ausbeute: 75 % der Theorie.

Beispiel 14

1-(3-(4-Cyano-phenyl)-propionyl]-6-benzylamino-1,2,3,4-tetrahydro-chinolin

2, 9 g (9,5 mMol) 6-Amino-1-[3-(4-cyano-phenyl)propionyll-1,2,3,4-tetrahydro-chinolin werden in 80 ml Methanol und 0,6 ml Essigsäure gelöst, mit 1,06 g (10 mMol) Benzaldehyd versetzt und 20 Minuten bei 0°C gerührt. Anschließend gibt man 0,63 g Natriumcyanborhydrid in kleinen Portionen zu, rührt noch eine halbe Stunde und läßt dann auf Raumtemperatur erwärmen. Die Lösung wird dann einrotiert und der Rückstand in wenig Eiswasser aufgenommen. Dann säuert man die Lösung an und gibt Natronlauge bis zur alkalischen Reaktion zu und extrahiert mit Methylenchlorid. Die getrocknete Lösung wird einrotiert und der Rückstand über eine Kieselgelsäule mit Toluol/Essigsäureethylester (1:1) chromatographiert.
Ausbeute: 3,7 g (98 % der Theorie),

| $C_{26}H_{25}N_{30}$ (395,51) | | | |
|---|---|---|---|
| Ber. | C 78,95 | H 6,37 | N 10,62 |
| Gef: | 79,08 | 6,54 | 9,76 |

Analog werden hergestellt:

(1) 1-[3-(4-Cyano-phenyl)-propionyl)-6-[(naphth-1-yl-methyl)-amino]-1,2,3,4-tetrahydro-chinolin
Ausbeute: 78 % der Theorie,

| $C_{30}H_{27}N_3O$ (445,57) | | | |
|---|---|---|---|
| Ber. | C 80,87 | H 6,10 | N 9,43 |

(fortgesetzt)

| $C_{30}H_{27}N_3O$ (445,57) | | | |
|---|---|---|---|
| Gef: | 80,33 | 6,36 | 8,98 |

(2) 1-[3-(4-Cyano-phenyl)-propionyl]-6-[(naphth-2-yl-methyl)-amino]-1,2,3,4-tetrahydro-chinolin
Ausbeute: 90 % der Theorie,

| $C_{30}H_{27}N_3O$ (445,57) | | | |
|---|---|---|---|
| Ber. | C 80,87 | H 6,10 | N 9,43 |
| Gef: | 80,66 | 6,30 | 8,89 |

(3) 1-[3-(4-Cyano-phenyl)-propionyl]-6-(N-methyl-benzylamino)-1,2,3,4-tetrahydro-chinolin
Hergestellt aus 6-Benzylamino-1-[3-(4-cyano-phenyl)-propionyl]-1,2,3,4-tetrahydro-chinolin (siehe Beispiel 14) und Formaldehyd
$R_f$-Wert = 0,42 (Kieselgel; Methylenchlorid/Essigsäureethylester = 19:1)

(4) 1-[3-(4-Cyano-phenyl)-propionyl]-6-(2,2-diphenyl-ethylamino)-1,2,3,4-tetrahydro-chinolin
Hergestellt aus 6-Amino-l-[3-(4-cyano-phenyl)propionyl]-1,2,3,4-tetrahydro-chinolin und Diphenylacetyldehyd
Ausbeute: 80 % der Theorie,
$R_f$-Wert = 0,49 (Kieselgel; Methylenchlorid/Essigsäureethylester = 9:1)

Beispiel 15

1-[3-(4-Cyano-phenyl)-propionyl]-6-(N-ethylaminocarbonylmethyl-benzylamino)-1,2,3,4-tetrahydro-chinolin

Zu einer Lösung von 1,2 g 1-[3-(4-Cyano-phenyl)-propionyl]-6-[N-hydroxycarbonylmethyl-N-benzyl-amino]-1,2,3,4-tetrahydrochinolin [hergestellt analog Beispiel 32 aus 1-[3-(4-Cyanophenyl)-propionyl]-6-[N-ethoxycarbonylmethyl-N-benzyl-amino]-1,2,3,4-tetrahydro-chinolin] (siehe Beispiel 6(1)) in 15 ml Dimethylformamid werden 0,8 g Dicyclohexylcarbodiimid gegeben und 10 Minuten bei 40°C gerührt. Anschließend kühlt man auf 0°C ab und gibt 0,22 g Ethylamin in 5 ml Dimethylformamid zu und läßt über Nacht rühren. Anschließend engt man die Lösung ein und chromatographiert den Rückstand über eine Kieselgelsäule mit Methylenchlorid/Essigsäureethylester (8:2).
Ausbeute: 94 % der Theorie,
$R_f$-Wert: 0,29 (Kieselgel; Methylenchlorid/Essigsäureethylester = 8:2)

Analog werden hergestellt:

(1) 1-[3-(4-Cyano-phenyl)-propionyl]-6-[N-(N,N-dipropylaminocarbonylmethyl) -benzylamino] -1,2,3,4-tetrahydro-chinolin
Ausbeute: 51 % der Theorie,
$R_f$-Wert: 0,43 (Kieselgel; Methylenchlorid/Essigsäureethylester = 9:1)

(2) 1-[3-(4-yano-phenyl)-propionyl]-6-[N-(benzylaminocarbonylmethyl)-benzylamino]-1,2,3,4-tetrahydro-chinolin
Ausbeute: 92 % der Theorie,
$R_f$-Wert: 0,38 (Kieselgel; Methylenchlorid/Essigsäureethylester = 8:2)

(3) 1- [3- (4-Cyano-phenyl) -propionyl] -6- [N- (phenylaminocarbonylmethyl)-benzylamino]-1,2,3,4-tetrahydro-chinolin
Ausbeute: 60 % der Theorie,
$R_f$-Wert: 0,58 (Kieselgel; Methylenchlorid/Essigsäureethylester = 9:1)

Beispiel 16

1-[3-(4-Cyano-phenyl)-propionyl]-6-phenylaminosulfonyl-1,2,3,4-tetrahydro-chinolin

(a) 1-[3-(4-Cyano-phenyl)-propionyl]-1,2,3,4-tetrahydro-chinolin-6-sulfonylchlorid

(0,04 Mol) 1-[3-(4-Cyano-phenyl)-propionyl]-1,2,3,4-tetrahydro-chinolin werden in 16 ml Chlorsulfonsäure bei 0°C gelöst. Dabei erwärmt sich die dickflüssige Masse auf 60°C und es entwickelt sich Salzsäure. Anschließend gibt man den Ansatz vorsichtig auf Eis und extrahiert mit Methylenchlorid, trocknet und rotiert ein.
Ausbeute: 5,9 g (38 % der Theorie),

(b) 1-[3-(4-Cyano-phenyl)-propionyl]-6-phenylaminosulfonyl-1,2,3,4-tetrahydro-chinolin

2,33 g (6 mMol) rohes 1-[3-(4-Cyano-phenyl)-propionyl]-1,2,3,4-tetrahydro-chinolin-6-sulfonylchlorid werden unter Eiskühlung zu einer Lösung von 0,56 g Anilin in 15 ml Pyridin getropft. Anschließend erwärmt man 30 Minuten auf 100°C und engt dann zur Trockene ein. Der Rückstand wird über eine Kieselgelsäule mit Methylenchlorid:Essigsäureethylester chromatographiert.
Ausbeute: 0,5 g (18 % der Theorie).

Analog werden hergestellt:

(1) 1-[3-(4-Cyano-phenyl)-propionyl]-6-benzylaminosulfonyl-1,2,3,4-tetrahydro-chinolin
Ausbeute: 12 % der Theorie

(2) 1-[3-(4-Cyano-phenyl)-propionyl]-6-phenylsulfonyl-1,2,3,4-tetrahydro-chinolin
Hergestellt aus 1-[3-(4-Cyano-phenyl)-propionyl]-1,2,3,4-tetrahydro-chinolin mit Benzolsulfonsäurechlorid und Aluminiumchlorid in Dimethylformamid.
Ausbeute: 38 % der Theorie,
$R_f$-Wert: 0,19 (Toluol/Essigsäureetylester = 8:2)

(3) 1-[3-(4-Cyano-phenyl)-propionyl]-6-benzoyl-1,2,3,4-tetrahydro-chinolin
Hergestellt aus 1-[3-(4-Cyano-phenyl)-propionyl]-1,2,3,4-tetrahydro-chinolin mit Benzoesäurechlorid und Aluminiumchlorid in Dimethylformamid.
Ausbeute: 10 % der Theorie,
$R_f$-Wert: 0,61 (Toluol/Essigsäureetylester = 7:3)

Beispiel 17

1-[N-(4-Cyano-benzyl)-methylaminocarbonyl]-6-phenylsulfonylamino-1,2,3,4-tetrahydro-chinolin

Eine Suspension von 0,87 g (3 mMol) 6-Phenylsulfonamido-1,2,3,4-tetrahydro-chinolin in 8 ml Toluol und 1,55 ml einer 20%igen Lösung von Phosgen in Toluol werden 3 Stunden bei Raumtemperatur gerührt, dann mit 0,44 g p-Cyano-N-methyl-benzylamin versetzt und drei Stunden am Rückfluß gekocht. Anschließend wird das Reaktionsgemisch einrotiert und der Rückstand über eine Kieselgelsäule mit Methylenchlorid/Methanol (39:1) chromatographiert.
Ausbeute: 0,81 g (58 % der Theorie),
$R_f$-Wert: 0,32 (Kieselgel; Methylenchlorid/Essigsäureethylester = 4:1)

Analog wird hergestellt:

(1) 1-[N-(4-Cyano-benzyl)-aminocarbonyl]-6-phenylsulfonylamino-1,2,3,4-tetrahydro-chinolin
Ausbeute: 64 % der Theorie.
$R_f$-Wert: 0,18 (Kieselgel; Methylenchlorid/Methanol = 4:1)

Beispiel 18

1-[3-(4-Cyano-phenyl)propyl]-6-phenylsulfonamido-1,2,3,4-tetrahydro-chinolin

1,73 g 6-Phenylsulfonamido-1,2,3,4-tetrahydro-chinolin werden mit 0,7 g Triethylamin in 10 ml Dimethylformamid gelöst und mit 1,62 g 3-(4-Cyano-phenyl)propyljodid in 15 ml Dimethylformamid versetzt und 5 Stunden bei 40-50°C gerührt. Anschließend wird eingedampft, der Rückstand in Essigester/Wasser gelöst, die organische Phase getrocknet

und im Vakuum eingeengt. Das Produkt wird mit Methylenchlorid/Essigester (19:1) über eine Kieselgelsäule chromatographiert.
Ausbeute: 600 mg (23 % der Theorie),
Schmelzpunkt: 131-132°C

Beispiel 19

1-[3-(4-Cyano-phenyl)thiopropionyl]-6-phenylsulfonamido-1,2,3,4-tetrahydro-chinolin

4,9 g 1-[3-(4-Cyano-phenyl)propionyl]-6-phenylsulfonamido-1,2,3,4-tetrahydro-chinolin werden in 110 ml Toluol gelöst und mit 2,5 g Lawesson-Reagenz [2,4-Bis(4-methoxy-phenyl)-1,3-dithia-2,4-diphosphetan-2,4-disulfid] 1 Stunde bei 110-120°C gekocht. Dann wird zur Trockene eingedampft und der Rückstand über eine Kieselgelsäule mit Methylenchlorid/Essigester = 19:1 chromatographiert.
Ausbeute: 3,5 g (69 % der Theorie),
Schmelzpunkt: 169-171°C

| $C_{25}H_{23}N_3O_2S_2$ (461,61) | | | | |
|---|---|---|---|---|
| Ber. | C 65,08 | H 5,02 | N 9,10 | S 13,89 |
| Gef. | 64,84 | 5,09 | 9,02 | 13,71 |

Beispiel 20

1-[3-(4-Cyano-phenyl)propionyl]-2,3,4,5-tetrahydro-1H-benzo-[b]azepin

Hergestellt analog Beispiel 1 durch Umsetzung von 2,3,4,5-Tetrahydro-1H-benzo[b]azepin und 3-(4-Cyano-phenyl)propionsäurechlorid.
Ausbeute: 3,0 g (82 % der Theorie),
$R_f$-Wert: 0,54 (Kieselgel; Toluol/Essigester = 7:3)

Beipiel 21

1-[3-(4-Cyano-phenyl)propyl]-dihydrocarbostyryl

1,47 g Dihydrocarbostyryl werden in 10 ml Dimethylsulfoxid gelöst und mit 0,49 g Natriumhydrid in Öl bei Raumtemperatur versetzt. Anschließend gibt man 2,71 g 3-(4-Cyano-phenyl)propyljodid in 5 ml Dimethylsulfoxid zu und rührt 1 1/2 Stunden bei Raumtemperatur. Dann wird die Lösung auf 20 ml Wasser gegeben und mit Essigester dreimal extrahiert. Die vereinigten organischen Phasen werden getrocknet und im Vakuum eingeengt. Das erhaltene Öl wird über Kieselgel mit Toluol/Essigester (8:2) chromatographiert.
Ausbeute: 2,5 g (86 % der Theorie),
$R_f$-Wert: 0,63 (Kieselgel; Toluol/Essigester = 8:2).

Beispiel 22

1-[3-(4-Cyano-phenyl)propyl]-6-nitro-dihydrocarbostyryl

Hergestellt analog Beispiel 3 durch Nitrieren von 1-[3-(4-Cyano-phenyl)propyl]-dihydrocarbostyryl.
Ausbeute: 70 % der Theorie,
$R_f$-Wert: 0,30 (Kieselgel; Toluol/Essigester = 8:2).

Beispiel 23

1-[3-(4-Cyano-phenyl)propyl]-6-amino-dihydrocarbostyryl

Hergestellt analog Beispiel 2 durch Reduktion von 1-[3-(4-Cyano-phenyl)propyl]-6-nitro-dihydrocarbostyryl.
Ausbeute: 86 % der Theorie,
$R_f$-Wert: 0,45 (Kieselgel; Essigester).

Beispiel 24

1-[3-(4-Amidino-phenyl)propionyl]-6-phenylsulfonamido-1,2,3,4-tetrahydro-chinolin-hydrochlorid

Bei -10°C gibt man 1,3 g 1-[3-(4-Cyano-phenyl)propionyl]-6-phenylsulfonamido-1,2,3,4-tetrahydro-chinolin (siehe Beispiel 9) in 25 ml mit Chlorwasserstoff gesättigtes Ethanol und läßt weiterhin bei -5°C eine Stunde einen schwachen Chlorwasserstoffstrom durch die Lösung strömen. Dann läßt man auf Raumtemperatur erwärmen und rührt über Nacht. Anschließend wird eingeengt und der Rückstand mit Ethanol gewaschen. Dann suspendiert man den Rückstand in 50 ml Methanol, gibt 2,25 g Ammoniumcarbonat zu und läßt über Nacht stehen. Anschließend wird die Lösung eingeengt und der Rückstand über eine Kieselgelsäule mit Methylenchlorid/Methanol = 8:2 chromatographiert.
Ausbeute: 1,15 g (77 % der Theorie),
Schmelzpunkt: ab 140°C (Zers.)

| $C_{25}H_{26}N_4O_3S$ x HCl x $H_2O$ (517,06) | | | | |
|---|---|---|---|---|
| Ber. | C 58,08 | H 5,65 | N 10,84 | S 6,20 |
| Gef. | 58,39 | 5,73 | 10,54 | 6,07 |

Analog werden hergestellt:

(1) 1-[3-(4-Amidino-phenyl)propionyl]-6-(2-naphthylsulfonamido)-1,2,3,4-tetrahydro-chinolin-hydrochlorid
Hergestellt aus der gemäß Beispiel 9(1) hergestellten Verbindung
Ausbeute: 83 % der Theorie,
Schmelzpunkt: ab 115°C (Zers.)

| $C_{29}H_{28}N_4O_3S$ x 1,25 HCl (558,20) | | | |
|---|---|---|---|
| Ber. | C 62,41 | H 5,30 | N 10,03 |
| Gef. | 61,94 | 5,65 | 9,82 |

(2) 1-[3-(4-Amidino-phenyl)propionyl]-6-(1-naphthyl-sulfonamido)-1,2,3,4-tetrahydro-chinolin-hydrochlorid
Hergestellt aus der gemäß Beispiel 9(2) hergestellten Verbindung
Ausbeute: 55 % der Theorie,
Schmelzpunkt: 100°C (Zers.)

| $C_{29}H_{28}N_4O_3S$ x HCl x 2 $H_2O$ (585,13) | | | |
|---|---|---|---|
| Ber. | C 59,50 | H 5,68 | N 9,75 |
| Gef. | 59,66 | 5,84 | 10,03 |

(3) 1-[3-(4-Amidino-phenyl)propionyl]-6-(4-fluor-phenylsulfonamido)-1,2,3,4-tetrahydro-chinolin-hydrochlorid
Hergestellt aus der gemäß Beispiel 9(3) hergestellten Verbindung
Ausbeute: 86 % der Theorie,
Schmelzpunkt: 135°C (Zers.)

| $C_{25}H_{25}FN_4O_3S$ x 1,25 HCl (526,14) | | | |
|---|---|---|---|
| Ber. | C 57,08 | H 5,03 | N 10,65 |
| Gef. | 57,08 | 5,39 | 10,72 |

(4) 1- [3- (4-Amidino-phenyl)propionyl] -6-butylsulfonamido-1,2,3,4-tetrahydro-chinolin-hydrochlorid
Hergestellt aus der gemäß Beispiel 9(4) hergestellten Verbindung
Ausbeute: 76 % der Theorie,
Schmelzpunkt: 122°C (Zers.)

| $C_{23}H_{30}N_4O_3S$ x HCl x 0,5 $H_2O$ (488,06) | | | |
|---|---|---|---|
| Ber. | C 56,60 | H 6,60 | N 11,48 |
| Gef. | 56,56 | 6,60 | 11,50 |

(5) 1- [ 3 - ( 4 -Amidino-phenyl ) propionyl ] - 5 -phenyl sul f onamido-1,2,3,4-tetrahydro-chinolin-hydrochlorid
Hergestellt aus der gemäß Beispiel 9(5) hergestellten Verbindung
Ausbeute: 56 % der Theorie,
Schmelzpunkt: ab 116°C (sintern ab 95°C)

| $C_{25}H_{26}N_4O_3S$ x HCl x $H_2O$ (517,05) | | | |
|---|---|---|---|
| Ber. | C 58,07 | H 5,65 | N 10,84 |
| Gef. | 58,53 | 5,80 | 10,94 |

(6) 1-[3-(4-Amidino-phenyl)propionyl]-7-phenylsulfonamido-1,2,3,4-tetrahydro-chinolin-hydrochlorid
Hergestellt aus der gemäß Beispiel 9(6) hergestellten Verbindung
Ausbeute: 67 % der Theorie,
Schmelzpunkt: ab -105°C

| $C_{25}H_{26}N_4O_3S$ x HCl x $H_2O$ (517,05) | | | |
|---|---|---|---|
| Ber. | C 58,08 | H 5,65 | N 10,84 |
| Gef. | 58,25 | 5,72 | 10,44 |

(7) 1- [3- (4-Amidino-phenyl) propionyl] -7-benzylsulfonamido-1,2,3,4-tetrahydro-chinolin-hydrochlorid
Hergestellt aus der gemäß Beispiel 9(7) hergestellten Verbindung
Ausbeute: 51 % der Theorie,
Schmelzpunkt: ab 108°C (Zers.)

| $C_{26}H_{28}N_4O_3S$ x HCl x 0,5 $H_2O$ (522,07) | | | |
|---|---|---|---|
| Ber. | C 59,82 | H 5,79 | N 10,73 |
| Gef. | 59,66 | 5,87 | 10,45 |

(8) 1-[3-(4-Amidino-phenyl)propionyl]-6-(4-amino-3,5-dichlorphenylsulfonamido)-1,2,3,4-tetrahydro-chinolin-hydrochlorid
Hergestellt aus der gemäß Beispiel 10 hergestellten Verbindung
Ausbeute: 73 % der Theorie,
Schmelzpunkt: 115°C (Zers.)

| $C_{25}H_{25}Cl_2N_5O_3S$ x HCl (582,44) | | | |
|---|---|---|---|
| Ber. | C 51,51 | H 4,50 | N 12,01 |
| Gef. | 51,52 | 4,71 | 11,94 |

(9) 1-[3-(4-Amidino-phenyl)propionyl]-6-propylsulfonamido-1,2,3,4-tetrahydro-chinolin-hydrochlorid
Hergestellt aus der gemäß Beispiel 10(2) hergestellten Verbindung
Ausbeute: 46 % der Theorie,
Schmelzpunkt: 123°C (Zers.)

| $C_{22}H_{28}N_4O_3S$ x HCl (465,02) | | | |
|---|---|---|---|
| Ber. | C 56,82 | H 6,29 | N 12,05 |
| Gef. | 57,19 | 6,52 | 11,77 |

(10) 1-[3-(4-Amidino-phenyl)propionyl]-6-(5-chlor-thien-2-ylsulfonamido)-1,2,3,4-tetrahydro-chinolin-hydrochlorid

Hergestellt aus der gemäß Beispiel 10(3) hergestellten Verbindung
Ausbeute: 96 % der Theorie,
Schmelzpunkt: 100°C (Zers.)

| $C_{23}H_{23}ClN_4O_3S_2$ × HCl x 0,5 $H_2O$ (548,51) | | | |
|---|---|---|---|
| Ber. | C 50,36 | H 4,50 | N 10,21 |
| Gef. | 50,66 | 4,70 | 10,01 |

(11) 1-[3-(4-Amidino-phenyl)propionyl]-6-isopropylsulfonamido-1,2,3,4-tetrahydro-chinolin-hydrochlorid
Hergestellt aus der gemäß Beispiel 10(4) hergestellten Verbindung
Ausbeute: 62 % der Theorie,
Schmelzpunkt: 110°C (Zers.)

| $C_{22}H_{28}N_4O_3S$ x HCl x 0,5 $H_2O$ (474,02) | | | |
|---|---|---|---|
| Ber. | C 55,74 | H 6,26 | N 11,82 |
| Gef. | 55,62 | 6,62 | 11,07 |

(12) 1- [3- (4-Amidino-phenyl)propionyl] -6- (3-chlorpropylsulfonamido)-1,2,3,4-tetrahydro-chinolin-hydrochlorid
Hergestellt aus der gemäß Beispiel 10(5) hergestellten Verbindung
Ausbeute: 100 % der Theorie,
Schmelzpunkt: 85°C (Zers.)

| $C_{22}H_{27}ClN_4O_3S$ x HCl x $C_2H_5OH$ (545,53) | | | |
|---|---|---|---|
| Ber. | C 52,84 | H 6,28 | N 10,27 |
| Gef. | 54,01 | 6,41 | 9,98 |

(13) 1-[3-(4-Amidino-phenyl)propionyl]-6-benzylsulfonamido-1,2,3,4-tetrahydro-chinolin-hydrochlorid
Ausbeute: 62 % der Theorie,
Schmelzpunkt: ab 80°C

| $C_{26}H_{28}N_4O_3S$ x HCl x $H_2O$ (531,08) | | | | |
|---|---|---|---|---|
| Ber. | C 58,80 | H 5,88 | N 10,55 | S 6,04. |
| Gef. | 58,75 | 5,90 | 10,61 | 5,95 |

(14) 1-[3-(4-Amidino-phenyl)propionyl]-6-phenylacetylamino-1,2,3,4-tetrahydro-chinolin-hydrochlorid
Hergestellt aus der gemäß Beispiel 9(9) hergestellten Verbindung
Ausbeute: 53 % der Theorie,
Schmelzpunkt: ab 150°C

| $C_{27}H_{28}N_4O_2$ x HCl x $H_2O$ (495,03) | | | |
|---|---|---|---|
| Ber. | C 65,51 | H 6,31 | N 11,32 |
| Gef. | 65,58 | 6,38 | 11,13 |

(15) 1-[3-(4-Amidino-phenyl)propionyl]-6-benzoylamino-1,2,3,4-tetrahydro-chinolin-hydrochlorid
Ausbeute: 75 % der Theorie,
Schmelzpunkt: ab 170°C

| $C_{26}H_{26}N_4O_2$ x HCl x $H_2O$ (487,00) | | | |
|---|---|---|---|
| Ber. | C 64,93 | H 6,08 | N 11,65 |
| Gef. | 65,02 | 6,22 | 11,66 |

(16) 1-[3-(4-Amidino-phenyl)propyl]-6-phenylsulfonamido-1,2,3,4-tetrahydro-chinolin-hydrochlorid

Hergestellt aus der gemäß Beispiel 18 hergestellten Verbindung Ausbeute: 26 % der Theorie, Schmelzpunkt: ab 140°C

| $C_{25}H_{28}N_4O_2S$ x HCl x $H_2O$ (503,07); | | | | |
|---|---|---|---|---|
| Ber. | C 59,69 | H 6,21 | N 11,14 | S 6,37 |
| Gef. | 59,72 | 6,08 | 10,97 | 6,38 |

(17) 1-[3-(4-Amidino-phenyl)thiopropionyl]-6-phenylsulfonamido-1,2,3,4-tetrahydro-chinolin-hydrochlorid Hergestellt aus der gemäß Beispiel 19 hergestellten Verbindung Ausbeute: 14 % der Theorie, Schmelzpunkt: ab 140°C (Zers.)

| $C_{25}H_{26}N_4O_2S_2$ x HCl x $H_2O$ (533,12) | | | | | |
|---|---|---|---|---|---|
| Ber. | C 56,33 | H 5,48 | N 10,51 | S 12,03 | Cl 6,65 |
| Gef. | 56,45 | 5,52 | 10,28 | 11,87 | 6,93 |

(18) 1-[3-(4-Amidino-phenyl)propionyl]-5-phenylsulfonamido-2,3-dihydro-indol-hydrochlorid Hergestellt aus der gemäß Beispiel 10(6) hergestellten Verbindung Schmelzpunkt: ab 128°C (Zers.)

| $C_{24}H_{24}N_4O_3S$ x HCl x 1,5 $H_2O$ (512,01) | | | |
|---|---|---|---|
| Ber. | C 56,14 | H 5,31 | N 10,96 |
| Gef. | 56,30 | 5,51 | 10,96 |

(19) 1-[3-(4-Amidino-phenyl)propionyl]-7-phenylsulfonamido-2,3,4,5-tetrahydro-1H-benzo[b]azepin-hydrochlorid Hergestellt aus der gemäß Beispiel 10(7) hergestellten Verbindung Ausbeute: 22,0 % der Theorie, Schmelzpunkt: ab 207°C (Zers.)

| $C_{26}H_{28}N_4O_3S$ x HCl x $C_2H_5OH$ (559,07) | | | |
|---|---|---|---|
| Ber. | C 60,16 | H 6,31 | N 10,02 |
| Gef. | 60,44 | 6,42 | 9,41 |

(20) 1-[3-(4-Amidino-phenyl)propionyl]-3-methyl-6-phenylsulfonamido-1,2,3,4-tetrahydro-chinolin-hydrochlorid Hergestellt aus der gemäß Beispiel 10(8) hergestellten Verbindung Ausbeute: 55% der Theorie, Schmelzpunkt: ab 140°C

| $C_{26}H_{28}N_4O_3S$ x HCl (513,07) | | | |
|---|---|---|---|
| Ber. | C 60,86 | H 5,70 | N 10,92 |
| Gef. | 61,09 | 6,05 | 10,21 |

(21) 1-[3-(4-Amidino-phenyl)propionyl]-4-methyl-6-phenylsulfonamido-1,2,3,4-tetrahydro-chinolin-hydrochlorid Hergestellt aus der gemäß Beispiel 10(9) hergestellten Verbindung Ausbeute: 68 % der Theorie, Schmelzpunkt: ab 133°C (Zers.)

| $C_{26}H_{28}NO_3S$ x HCl (513,07) | | | |
|---|---|---|---|
| Ber. | C 60,86 | H 5,70 | N 10,92 |
| Gef. | 59,82 | 5,79 | 20,73 |

(22) 1-[3-(4-Amidino-phenyl)propionyl]-2-methyl-6-phenylsulfonamido-1,2,3,4-tetrahydro-chinolin-hydrochlorid Hergestellt aus der gemäß Beispiel 9(14) hergestellten Verbindung

Ausbeute: 37 % der Theorie,
Schmelzpunkt: ab 112°C (Zers.)

| $C_{26}H_{29}ClN_4O_3S$ x HCl x 2 $H_2O$ (549,07) | | | |
|---|---|---|---|
| Ber. | C 56,87 | H 6,06 | N 10,02 |
| Gef. | 56,43 | 5,98 | 9,90 |

(23) 1-[3-(4-Amidino-phenyl)propionyl]-6-[(5-dimethylaminonaphth-1-yl)sulfonamido]-1,2,3,4-tetrahydro-chinolin-hydrochlorid
Hergestellt aus der gemäß Beispiel 10(1) hergestellten Verbindung
Ausbeute: 52 % der Theorie,

| $C_{31}H_{33}N_5O_3S$ x 1,25 HCl (601,03) | | | |
|---|---|---|---|
| Ber. | C 61,98 | H 5,87 | N 11,65 |
| Gef. | 61,64 | 6,43 | 10,38 |

(24) 1-[N-(4-Amidino-benzyl)-aminocarbonyl]-6-phenylsulfonylamino-1,2,3,4-tetrahydro-chinolin-hydrochlorid
Ausbeute: 64 % der Theorie,
$C_{24}H_{25}N_5O_3S$ (463,57)
Massenspektrum: FAB-MS: $(M+H)^+$ =464

(25) 1-[3-(4-Amidino-phenyl)propyl]-6-phenylsulfonamido-dihydrocarbostyryl-hydrochlorid
Schmelzpunkt: ab 136°C

| $C_{25}H_{26}N_4O_3S$ x HCl x 2 $H_2O$ (535,07) | | | |
|---|---|---|---|
| Ber. | C 56,22 | H 5,85 | N 10,49 |
| Gef. | 55,15 | 5,58 | 10,58 |

(26) 1- [3- (4-Amidino-phenyl) propionyl] -6- (3-trifluoromethylbenzolsulfonylamino)-1,2,3,4-tetrahydro-chinolin-hydrochlorid
Hergestellt aus der gemäß Beispiel 10(10) hergestellten Verbindung
Ausbeute: 72 % der Theorie,
Schmelzpunkt: 146-147°C

| $C_{26}H_{25}F_3N_4O_3S$ x HCl x $C_2H_5OH$ (613,14) | | | | |
|---|---|---|---|---|
| Ber. | C 54,85 | H 5,26 | N 9,14 | Cl 5,78 |
| Gef: | 54,71 | 5,23 | 9,10 | 6,00 |

(27) 1-[3-(4-Amidino-phenyl)propionyl]-6-(2,5-dichlor-benzolsulfonylamino)-1,2,3,4-tetrahydro-chinolin-hydrochlorid
Hergestellt aus der gemäß Beispiel 10(11) hergestellten Verbindung
Ausbeute: 77 % der Theorie,
Schmelzpunkt: 239°C

| $C_{25}H_{24}Cl_2N_4O_3S$ x HCl (567,93) | | | |
|---|---|---|---|
| Ber. | C 52,67 | H 4,44 | N 9,87 |
| Gef: | 50,33 | 4,86 | 10,39 |

(28) 1-[3-(4-Amidino-phenyl)propionyl]-6-(2,3,5,6-tetramethylbenzolsulfonylamino)-1,2,3,4-tetrahydro-chinolin-hydrochlorid
Hergestellt aus der gemäß Beispiel 10(12) hergestellten Verbindung
Ausbeute: 12 % der Theorie,
Schmelzpunkt: 224-225°C

$R_f$-Wert: 0,28 (Kieselgel; Methylenchlorid/Ethanol = 4:1)

(29) 1-[3-(4-Amidino-phenyl)propionyl]-6-(5-isochinolinylsulfonylamino)-1,2,3,4-tetrahydro-chinolin-hydrochlorid
Hergestellt aus der gemäß Beispiel 10(14) hergestellten Verbindung
Ausbeute: 66 % der Theorie,
Schmelzpunkt: 195°C

| $C_{28}H_{27}N_5O_3S$ x HCl x $H_2O$ (568,1) | | | |
|---|---|---|---|
| Ber. | C 58,45 | H 5,24 | N 12,17 |
| Gef. | 57,49 | 5,64 | 11,97 |

(30) l-[3-(4-Amidino-phenyl)propionyl]-6-(cyclopropyl-sulfonylamino)-1,2,3,4-tetrahydro-chinolin-hydrochlorid
Hergestellt aus der gemäß Beispiel 10(15) hergestellten Verbindung
Ausbeute: 87 % der Theorie,
Schmelzpunkt: ab 85°C

| $C_{22}H_{26}N_4O_3S$ x HCl (463,0) | | | |
|---|---|---|---|
| Ber. | C 57,07 | H 5,88 | N 12,10 |
| Gef. | 56,35 | 6,55 | 11,33 |

(31) 1-[3-(4-Amidino-phenyl)propionyl]-6-(benzimidazol-5-ylsulfonylamino)-1,2,3,4-tetrahydro-chinolin-hydrochlorid
Hergestellt aus der gemäß Beispiel 10(16) hergestellten Verbindung
Ausbeute: 66 % der Theorie,
Schmelzpunkt: ab 220°C

| $C_{26}H_{26}N_6O_3S$ x HCl x $H_2O$ (557,08) | | | |
|---|---|---|---|
| Ber. | C 56,06 | H 5,25 | N 15,09 |
| Gef. | 55,76 | 5,37 | 14,74 |

(32) 1-[3-(3-Amidino-phenyl)propionyl]-6-phenylsulfonylamino-1,2,3,4-tetrahydro-chinolin-hydrochlorid
Hergestellt aus der gemäß Beispiel 9(24) hergestellten Verbindung
Ausbeute: 17 % der Theorie
Schmelzpunkt: ab 134-139°C

| $C_{25}H_{26}N_4O_3S$ x HCl x $H_2O$ (517,05) | | | |
|---|---|---|---|
| Ber. | C 58,08 | H 5,65 | N 10,84 |
| Gef. | 57,52 | 5,83 | 10,04 |

(33) 1-[(4-Amidino-phenyloxy)acetyl]-6-phenylsulfonylamino-1,2,3,4-tetrahydro-chinolin-hydrochlorid
Hergestellt aus der gemäß Beispiel 9(25) hergestellten Verbindung
Ausbeute: 44 % der Theorie,
Schmelzpunkt: 137-143°C

| $C_{24}H_{24}N_4O_4S$ x HCl x $H_2O$ (519,02) | | | |
|---|---|---|---|
| Ber. | C 55.54 | H 5,24 | N 10,79 |
| Gef. | 54,57 | 5,31 | 10,50 |

(34) 1-[2-((4-Amidino-phenyl)-methyl-amino]acetyl]-6-phenylsulfonylamino-1,2,3,4-tetrahydro-chinolin-hydrochlorid
Hergestellt aus der gemäß Beispiel 9(26) hergestellten Verbindung
Ausbeute: 77 % der Theorie,
Schmelzpunkt: sintert ab 180°C

| $C_{25}H_{27}N_5O_3S$ x HCl x $H_2O$ (532,07) | | | | |
|---|---|---|---|---|
| Ber. | C 56,44 | H 5,68 | N 13,16 | S 6,03 |
| Gef. | 55,71 | 5,53 | 13,03 | 5,87 |

(35) 1- [3- (4-Amidino-phenyl) -propionyl] -6- (cyclohexylsulfonylamino)-1,2,3,4-tetrahydro-chinolin-hydrochlorid
Hergestellt aus der gemäß Beispiel 10(17) hergestellten Verbindung
Ausbeute: 62 % der Theorie,
Schmelzpunkt: Zersetzung ab 125°C

| $C_{25}H_{32}N_4O_3S$ x HCl x $H_2O$ (523,10) | | | | |
|---|---|---|---|---|
| Ber. | C 57,40 | H 6,74 | N 10,71 | S 6,13 |
| Gef. | 57,22 | 6,56 | 10,58 | 6,07 |

(36) 1-[3-(4-Amidino-phenyl)propionylj-6-(3-tolyl-sulfonylamino)-1,2,3,4-tetrahydro-chinolin-hydrochlorid
Hergestellt aus der gemäß Beispiel 10(18) hergestellten Verbindung
Ausbeute: 69 % der Theorie,
Schmelzpunkt: ab 260°C

| $C_{26}H_{28}N_4O_3S$ x HCl x $H_2O$ (531,08) | | | |
|---|---|---|---|
| Ber. | C 58,80 | H 5,88 | N 10,54 |
| Gef. | 58,97 | 5,84 | 10,40 |

(37) 1-[3-(4-Amidino-phenyl)propionyl]-6-(4-methoxy-benzolsulfonylamino)-1,2,3,4-tetrahydro-chinolin-hydrochlorid
Hergestellt aus der gemäß Beispiel 10(19) hergestellten Verbindung
Ausbeute: 80 % der Theorie,

| $C_{26}H_{28}N_4O_4S$ x HCl x $H_2O$ (547,08) | | | |
|---|---|---|---|
| Ber. | C 57,08 | H 5,71 | N 10,24 |
| Gef. | 56,89 | 6,19 | 9,27 |

(38) 1-[3-(4-Amidino-phenyl)-propionyl]-6-(3-aminocarbonylbenzol-sulfonylamino)-1,2,3,4-tetrahydro-chinolin-hydrochlorid
Ausbeute: 32 % der Theorie,

| $C_{26}H_{27}N_5O_4S$ x HCl x $H_2O$ (560,08) | | | |
|---|---|---|---|
| Ber. | C 55,76 | H 5,40 | N 12,50 |
| Gef. | 54,15 | 5,74 | 10,75 |

(39) 1- [3- (4-Amidino-phenyl) -propionyl] -6- (N-benzoyl-methylamino)-1,2,3,4-tetrahydro-chinolin-hydrochlorid
Hergestellt aus der gemäß Beispiel 11(2) hergestellten Verbindung
Ausbeute: 65 % der Theorie,
Schmelzpunkt: 90-92°C

| $C_{27}H_{28}N_4O_2$ x HCl x $2H_2O$ (512,03) | | | |
|---|---|---|---|
| Ber. | C 63,34 | H 6,30 | N 10,94 |
| Gef. | 63,21 | 6,48 | 10,92 |

(40) 1-[3-(4-Amidino-phenyl)-propionyl]-6-[N-(4-chlorbenzoyl)-methylamino]-1,2,3,4-tetrahydro-chinolin-hydrochlorid
Hergestellt aus der gemäß Beispiel 11(3) hergestellten Verbindung

Ausbeute: 76 % der Theorie,

| $C_{27}H_{27}N_4O_2Cl$ x HCl x $H_2O$ (529,47) | | | |
|---|---|---|---|
| Ber. | C 61,24 | H 5,71 | N 10,58 |
| Gef. | 61,70 | 5,88 | 10,37 |

(41) 1-[3-(4-Amidino-phenyl)-propionyl]-6-[N-(l-naphthoyl)-methylamino]-1,2,3,4-tetrahydro-chinolin-hydrochlorid
Hergestellt aus der gemäß Beispiel 11(4) hergestellten Verbindung
Ausbeute: 71 % der Theorie,

| $C_{31}H_{30}N_4O_2$ x HCl x 1,5 $H_2O$ (554,09) | | | |
|---|---|---|---|
| Ber. | C 67,19 | H 6,18 | N 10,11 |
| Gef. | 67,22 | 6,12 | 10,19 |

(42) 1- [3- (4-Amidino-phenyl) -propionyl] -6- [N- (2-naphthoyl) -methylamino)-1,2,3,4-tetrahydro-chinolin-hydrochlorid
Hergestellt aus der gemäß Beispiel 11(5) hergestellten Verbindung
Ausbeute: 72 % der Theorie,

| $C_{31}H_{30}N_4O_2$ x HCl x $H_2O$ (545,09) | | | |
|---|---|---|---|
| Ber. | C 68,30 | H 6,10 | N 10,27 |
| Gef. | 68,03 | 6,28 | 10,27 |

(43) 1-[3-(4-Amidino-phenyl)-propionyl]-6-(N-butyryl-methylamino)-1,2,3,4-tetrahydro-chinolin-hydrochlorid
Hergestellt aus der gemäß Beispiel 11(6) hergestellten Verbindung
Ausbeute: 59 % der Theorie,

| $C_{24}H_{30}N_4O_2$ x HCl x $H_2O$ (461,01) | | | |
|---|---|---|---|
| Ber. | C 62,52 | H 7,21 | N 12,15 |
| Gef. | 62,66 | 7,28 | 11,84 |

(44) 1-[3-(4-Amidino-2-nitro-phenyl)-propionyl]-6-phenylsulfonylamino-1,2,3,4-tetrahydro-chinolin-hydrochlorid
Hergestellt aus der gemäß Beispiel 9(28) hergestellten Verbindung
Ausbeute: 35 % der Theorie,
$R_f$-Wert: 0,44 (Kieselgel; Methylenchlorid/Methanol = 4:1)

(45) 1-[3-(4-Amidino-3-amino-phenyl)-propionyl]-6-phenylsulfonylamino-1,2,3,4-tetrahydro-chinolin-hydrochlorid
Ausbeute: 53 % der Theorie,

| $C_{25}H_{27}N_5O_3$ x 2 HCl (514,65) | | | |
|---|---|---|---|
| Ber. | C 54,49 | H 5,30 | N 12,70 |
| Gef. | 54,60 | 5,61 | 12,47 |

(46) 1-[3-(4-Amidino-2-acetylamino-phenyl)-propionyl]-6-phenylsulfonylamino-1,2,3,4-tetrahydro-chinolin-hydrochlorid
Hergestellt aus der gemäß Beispiel 11(7) hergestellten Verbindung
Ausbeute: 59 % der Theorie,

| $C_{27}H_{29}N_5O_4S$ x HCl (556,08) | | | |
|---|---|---|---|
| Ber. | C 53,32 | H 5,44 | S 5,76 |
| Gef. | 52,93 | 5,87 | 5,50 |

(47) 1-[3-(4-Amidino-2-(2-ethoxycarbonylethylcarbonylamino)-phenyl)-propionyl]-6-phenylsulfonylamino-1,2,3,4-tetrahydrochinolin-hydrochlorid
Hergestellt aus der gemäß Beispiel 11(8) hergestellten Verbindung
Ausbeute: 35 % der Theorie,

| $C_{31}H_{35}N_{35}O_6S$ x HCl (642,18) | | | |
|---|---|---|---|
| Ber. | C 57,98 | H 5,49 | N 10,90 |
| Gef. | 55,39 | 5,91 | 10,43 |

(48) 1-(4-Amidino-benzoyl)-6-phenylsulfonylamino-1,2,3,4-tetrahydro-chinolin-hydrochlorid
Hergestellt aus der gemäß Beispiel 9(29) hergestellten Verbindung
Ausbeute: 62 % der Theorie,

| $C_{23}H_{22}N_4O_3S$ x HCl x $H_2O$ (489,00) | | | |
|---|---|---|---|
| Ber. | C 56,49 | H 5,15 | N 11,46 |
| Gef. | 55,80 | 5,04 | 11,15 |

(49) 1-[3-(4-Amidino-3-fluor-phenyl)-propionyl]-6-phenylsulfonylamino-1,2,3,4-tetrahydro-chinolin-hydrochlorid
Hergestellt aus der gemäß Beispiel 9(31) hergestellten Verbindung
Ausbeute: 55 % der Theorie,

| $C_{25}H_{25}FN_4O_3S$ x HCl (517,03) | | | |
|---|---|---|---|
| Ber. | C 58,08 | H 5,07 | N 10,84 |
| Gef. | 57,63 | 5,18 | 10,75 |

(50) 1-[3-(2-Amidino-pyridin-5-yl)-propionyl]-6-phenylsulfonylamino-1,2,3,4-tetrahydro-chinolin-hydrochlorid
Hergestellt aus der gemäß Beispiel 9(32) hergestellten Verbindung
Ausbeute: 68 % der Theorie,

| $C_{24}H_{25}N_5O_3S$ x HCl x $H_2O$ (518,04) | | | |
|---|---|---|---|
| Ber. | C 55,65 | H 5,45 | N 13,52 |
| Gef. | 55,23 | 5,54 | 12,75 |

(51) 1-[3-(4-Amidino-phenyl)-acroyl]-6-phenylsulfonylamino-1,2,3,4-tetrahydro-chinolin-hydrochlorid
Hergestellt aus der gemäß Beispiel 9(33) hergestellten Verbindung
Ausbeute: 18 % der Theorie,

| $C_{25}H_{24}N_4O_3S$ x HCl x $H_2O$ (515,04) | | | | |
|---|---|---|---|---|
| Ber. | C 58,30 | H 5,28 | N 10,88 | S 6,23 |
| Gef. | 56,82 | 5,29 | 10,84 | 6,29 |

(52) 1-[3-(4-Amidino-phenyl)-propionyl]-6-piperidinocarbonyl-1,2,3,4-tetrahydro-chinolin-hydrochlorid
Hergestellt aus der gemäß Beispiel 9(34) hergestellten Verbindung
Ausbeute: 65 % der Theorie,

| $C_{25}H_{30}N_4O_2$ x HCl x 1,5 $H_2O$ (481,99) | | | |
|---|---|---|---|
| Ber. | C 62,29 | H 7,11 | N 11,62 |
| Gef. | 61,78 | 6,94 | 11,40 |

(53) 1-[3-(4-Amidino-phenyl)-propionyl]-6-benzylaminocarbonyl-1,2,3,4-tetrahydro-chinolin-hydrochlorid
Ausbeute: 3 % der Theorie,
$C_{27}H_{28}N_4O_2$ (440,55)

Massenspektrum: $(M+H)^+$ = 442

(54) 1-[3-(4-Amidino-phenyl)-propionyl]-6-(N-methyl-phenylaminocarbonyl)-1,2,3,4-tetrahydro-chinolin-hydrochlorid
Hergestellt aus der gemäß Beispiel 9(36) hergestellten Verbindung
Ausbeute: 5 % der Theorie,

| $C_{27}H_{28}N_4O_2$ x HCl x $H_2O$ (494,99) | | | |
|---|---|---|---|
| Ber. | C 65,51 | H 6,31 | N 11,32 |
| Gef. | 65,60 | 6,26 | 11,23 |

(55) 1-[3-(4-Amidino-phenyl)-propionyl]-6-diethylaminocarbonyl-1,2,3,4-tetrahydro-chinolin-hydrochlorid
Hergestellt aus der gemäß Beispiel 9(37) hergestellten Verbindung
Ausbeute: 7 % der Theorie,

| $C_{24}H_{30}N_4O_2$ x HCl x 2,5 $H_2O$ (487,98) | | | |
|---|---|---|---|
| Ber. | C 59,07 | H 7,44 | N 11,48 |
| Gef. | 59,05 | 7,06 | 11,12 |

(56) 1-[3-(4-Amidino-phenyl)-propionyl)-6-(3,5-dimethyl-piperidinocarbonyl)-1,2,3,4-tetrahydro-chinolin-hydrochlorid
Hergestellt aus der gemäß Beispiel 9(38) hergestellten Verbindung
Ausbeute: 7 % der Theorie,

| $C_{27}H_{34}N_4O_2$ x HCl x 2 $H_2O$ (519,04) | | | |
|---|---|---|---|
| Ber. | C 61,41 | H 7,63 | N 10,61 |
| Gef. | 60,76 | 7,36 | 10,35 |

(57) 1-[3-(4-Amidino-phenyl)-propionyl]-6-(N-phenyl-butylaminocarbonyl)1,2,3,4-tetrahydro-chinolin-hydrochlorid
Hergestellt aus der gemäß Beispiel 12 hergestellten Verbindung
Ausbeute: 15 % der Theorie,

| $C_{30}H_{34}N_4O_2$ x HCl x $H_2O$ (537,07) | | | |
|---|---|---|---|
| Ber. | C 67,08 | H 6,94 | N 10,43 |
| Gef. | 67,07 | 6,85 | 10,23 |

(58) 1-[3-(4-Amidino-phenyl)-propionyl]-6-[N-(4-chlorphenyl)-methylaminocarbonyl)-1,2,3,4-tetrahydro-chinolin-hydrochlorid
Hergestellt aus der gemäß Beispiel 12(1) hergestellten Verbindung
Ausbeute: 33 % der Theorie,

| $C_{27}H_{27}N_4O_2$ x HCl x $H_2O$ (529,43) | | | |
|---|---|---|---|
| Ber. | C 61,25 | H 5,71 | N 10,58 |
| Gef. | 60,74 | 5,70 | 10,24 |

(59) 1-[3-(4-Amidino-phenyl)-propionyl)-6-(N-phenyl-ethylaminocarbonyl)-1,2,3,4-tetrahydro-chinolin-hydrochlorid
Hergestellt aus der gemäß Beispiel 12(2) hergestellten Verbindung
Ausbeute: 64 % der Theorie,

| $C_{28}H_{30}N_4O_2$ x HCl x $H_2O$ (509,02) | | | |
|---|---|---|---|
| Ber. | C 66,06 | H 6,53 | N 11,01 |

(fortgesetzt)

| $C_{28}H_{30}N_4O_2$ x HCl x $H_2O$ (509,02) | | | |
|---|---|---|---|
| Gef. | 66,55 | 6,49 | 10,82 |

(60) 1-[3-(4-Amidino-phenyl)-propionyl]-6-diphenylaminocarbonyl-1,2,3,4-tetrahydro-chinolin-hydrochlorid
Hergestellt aus der gemäß Beispiel 12(3) hergestellten Verbindung
Ausbeute: 41 % der Theorie,

| $C_{32}H_{30}N_4O_2$ x 2 HCl x 2 $H_2O$ (611,57) | | | |
|---|---|---|---|
| Ber. | C 62,85 | H 5,94 | N 9,17 |
| Gef. | 61,57 | 5,99 | 8,61 |

(61) 1-[3-(4-Amidino-phenyl)-propionyl]-6-(N-phenyl-benzylaminocarbonyl)-1,2,3,4-tetrahydro-chinolin-hydrochlorid
Hergestellt aus der gemäß Beispiel 12(4) hergestellten Verbindung
Ausbeute: 33 % der Theorie,
Schmelzpunkt: Zersetzung ab 130°C

(62) 1-[3-(4-Amidino-phenyl)-propionyl]-6-(N-ethoxycarbonylmethyl-phenylaminocarbonyl)-1,2,3,4-tetraydro-chinolin-hydrochlorid
Hergestellt aus der gemäß Beispiel 12(5) hergestellten Verbindung
Ausbeute: 64 % der Theorie,

| $C_{30}H_{32}N_4O_2$ x HCl (549,05) | | | |
|---|---|---|---|
| Ber. | C 65,62 | H 6,06 | N 10,20 |
| Gef. | 66,23 | 6,29 | 10,12 |

(63) 1-[3-(4-Amidino-phenyl)-propionyl]-6-(N-cyclohexyl-methylaminocarbonyl)-1,2,3,4-tetrahydro-chinolin-hydrochlorid
Hergestellt aus der gemäß Beispiel 12(6) hergestellten Verbindung
Ausbeute: 30 % der Theorie,

| $C_{27}H_{34}N_4O_2$ x HCl x 0,5 $H_2O$ (492,04) | | | |
|---|---|---|---|
| Ber. | C 65,90 | H 7,38 | N 11,39 |
| Gef. | 65,88 | 7,41 | 11,14 |

(64) 1-(3-(4-Amidino-phenyl)-propionyl]-6-(4-methyl-piperidinocarbonyl-1,2,3,4-tetrahydro-chinolin-hydrochlorid
Hergestellt aus der gemäß Beispiel 9(22) hergestellten Verbindung
Ausbeute: 38 % der Theorie,

| $C_{26}H_{32}N_4O_2$ x HCl x 2,5 $H_2O$ (514,02) | | | |
|---|---|---|---|
| Ber. | C 60,75 | H 7,45 | N 10,90 |
| Gef. | 60,51 | 7,14 | 10,70 |

(65) 1-[3-(4-Amidino-phenyl)-propionyl]-6-morpholinocarbonyl-1,2,3,4-tetrahydro-chinolin-hydrochlorid
Hergestellt aus der gemäß Beispiel 9(23) hergestellten Verbindung
Ausbeute: 70 % der Theorie,

| $C_{24}H_{28}N_4O_3$ x HCl x 2,5 $H_2O$ (501,96) | | | |
|---|---|---|---|
| Ber. | C 57,42 | H 6,83 | N 11,16 |
| Gef. | 57,86 | 6,63 | 10,73 |

(66) 1-[3-(4-Amidino-phenyl)-propionyl)-6-(N-ethoxycarbonylmethyl-cyclohexylaminocarbonyl)-1,2,3,4-tetrahydro-chinolinhydrochlorid
Hergestellt aus der gemäß Beispiel 12(7) hergestellten Verbindung
Ausbeute: 77 % der Theorie,

| $C_{30}H_{38}N_4O_4$ x HCl x 0,5 $H_2O$ (564,10) | | | |
|---|---|---|---|
| Ber. | C 63,87 | H 7,15 | N 9,93 |
| Gef. | 63,43 | 7,18 | 9,43 |

(67) 1-[3-(4-Amidino-phenyl)-propionyl]-6-(2-ethoxycarbonylpyrrolidinocarbonyl)-1,2,3,4-tetrahydro-chinolin-hydrochlorid
Hergestellt aus der gemäß Beispiel 12(8) hergestellten Verbindung
Ausbeute: 61 % der Theorie,

| $C_{27}H_{32}N_4O_4$ x HCl x $H_2O$ (531,02) | | | |
|---|---|---|---|
| Ber. | C 61,06 | H 6,64 | N 10,55 |
| Gef. | 60,50 | 6,57 | 10,50 |

(68) 1-[3-(4-Amidino-phenyl)-propionyl]-6-(2-ethoxycarbonylpiperidinocarbonyl)-1,2,3,4-tetrahydro-chinolin-hydrochlorid
Hergestellt aus der gemäß Beispiel 12(9) hergestellten Verbindung
Ausbeute: 63 % der Theorie,

| $C_{28}H_{34}N_4O_4$ x HCl x $H_2O$ (545,05) | | | |
|---|---|---|---|
| Ber. | C 61,69 | H 6,84 | N 10,28 |
| Gef. | 61,45 | 6,67 | 9,96 |

(69) 1-[3-(4-Amidino-phenyl)-propionyl]-6-(3-ethoxycarbonylpiperidinocarbonyl)-1,2,3,4-tetrahydro-chinolin-hydrochlorid
Hergestellt aus der gemäß Beispiel 12(10) hergestellten Verbindung
Ausbeute: 70 % der Theorie,

| $C_{28}H_{34}N_4O_4$ x HCl x $H_2O$ (545,05) | | | |
|---|---|---|---|
| Ber. | C 61,69 | H 6,84 | N 10,28 |
| Gef. | 61,69 | 6,82 | 10,21 |

(70) 1- [3- (4-Amidino-phenyl) -propionyl] -6- [N- (2-acetylaminoethyl)-phenylaminocarbonyl]-1,2,3,4-tetrahydro-chinolin-hydrochlorid
Hergestellt aus der gemäß Beispiel 12(11) hergestellten Verbindung
Ausbeute: 22 % der Theorie,

| $C_{30}H_{33}N_5O_3$ x HCl x 1,5 $H_2O$ (575,1) | | | |
|---|---|---|---|
| Ber. | C 62,61 | H 6,44 | N 12,18 |
| Gef. | 62,76 | 6,35 | 12,04 |

(71) 1-[3-(4-Amidino-phenyl)-propionyl]-6-[N-(2-aminoethyl)-phenylaminocarbonyl]-1,2,3,4-tetrahydro-chinolin-hydrochlorid
Hergestellt aus der gemäß Beispiel 13 hergestellten Verbindung
Ausbeute: 36 % der Theorie,
Massenspektrum: FAB-MS $(M+H)^+$ = 470

(72) 1-[3-(4-Amidino-phenyl)-propionyl]-6-benzylamino-1,2,3,4-tetrahydro-chinolin-hydrochlorid
Hergestellt aus der gemäß Beispiel 14 hergestellten Verbindung

Ausbeute: 66 % der Theorie,

| $C_{26}H_{28}N_4O$ x HCl x 1,5 $H_2O$ (476,04) | | | |
|---|---|---|---|
| Ber. | C 65,60 | H 6,77 | N 11,77 |
| Gef. | 65,17 | 6,81 | 11,36 |

(73) 1-[3-(4-Amidino-phenyl)-propionyl]-6-(naphth-1-yl-methylamino)-1,2,3,4-tetrahydro-chinolin-hydrochlorid
Hergestellt aus der gemäß Beispiel 14(1) hergestellten Verbindung
Ausbeute: 45 % der Theorie,

| $C_{30}H_{30}N_4O$ x HCl x 2 $H_2O$ (535,1) | | | |
|---|---|---|---|
| Ber. | C 67,33 | H 6,59 | N 10,47 |
| Gef. | 66,07 | 6,85 | 9,42 |

(74) 1-[3-(4-Amidino-phenyl)-propionyl]-6-(naphthalin-2-ylmethyl-amino)-1,2,3,4-tetrahydro-chinolin-hydrochlorid
Hergestellt aus der gemäß Beispiel 14(2) hergestellten Verbindung
Ausbeute: 72 % der Theorie,

| $C_{30}H_{30}N_4O$ x HCl x 2 $H_2O$ (535,1) | | | |
|---|---|---|---|
| Ber. | C 67,33 | H 6,59 | N 10,47 |
| Gef. | 67,21 | 6,64 | 10,05 |

(75) 1-[3-(4-Amidino-phenyl)-propionyl]-6-(N-methyl-benzylamino)-1,2,3,4-tetrahydro-chinolin-hydrochlorid
Hergestellt aus der gemäß Beispiel 14(3) hergestellten Verbindung
Ausbeute: 59 % der Theorie

| $C_{27}H_{30}N_4O$ x HCl x $H_2O$ (481,06) | | | |
|---|---|---|---|
| Ber. | C 67,41 | H 6,91 | N 11,64 |
| Gef. | 67,44 | 7,03 | 11,28 |

(76) 1-[3-(4-Amidino-phenyl)-propionyl]-6-(N-ethoxycarbonylmethyl-benzylamino)-1,2,3,4-tetrahydro-chinolin-hydrochlorid
Hergestellt aus der gemäß Beispiel 6(1) hergestellten Verbindung
Ausbeute: 52 % der Theorie,

| $C_{30}H_{34}N_4O_3$ x HCl x $H_2O$ (553,13) | | | |
|---|---|---|---|
| Ber. | C 64,10 | H 6,81 | N 9,96 |
| Gef. | 64,17 | 6,76 | 10,04 |

(77) 1-[3-(4-Amidino-phenyl)-propionyl]-6-[N-(ethoxycarbonylmethyl)-N-(naphth-2-yl-methyl)-amino]-1,2,3,4-tetrahydro-chinolin-hydrochlorid
Hergestellt aus der gemäß Beispiel 6(2) hergestellten Verbindung
Ausbeute: 84 % der Theorie,

| $C_{34}H_{36}N_4O_3$ x HCl x 1,5 $H_2O$ (612,19) | | | |
|---|---|---|---|
| Ber. | C 66,70 | H 6,58 | N 9,15 |
| Gef. | 66,81 | 6,40 | 9,46 |

(78) 1-[3-(4-Amidino-phenyl)-propionyl]-6-[N-(ethoxycarbonylmethyl)-N-(naphth-1-yl-methyl)-amino]-1,2,3,4-tetrahydro-chinolin-hydrochlorid
Hergestellt aus der gemäß Beispiel 6(3) hergestellten Verbindung
Ausbeute: 69 % der Theorie,

| $C_{34}H_{36}N_4O_3$ x HCl x 1,5 $H_2O$ (612,19) | | | |
|---|---|---|---|
| Ber. | C 66,70 | H 6,58 | N 9,15 |
| Gef. | 66,83 | 6,44 | 9,14 |

(79) 1-[3-(4-Amidino-phenyl)-propionyl]-6-(N-acetyl-N-benzylamino)-1,2,3,4-tetrahydro-chinolin-hydrochlorid
Hergestellt aus der gemäß Beispiel 11(9) hergestellten Verbindung
Ausbeute: 46 % der Theorie,

| $C_{28}H_{30}N_4O_2$ x HCl x 2 $H_2O$ (527,07) | | | |
|---|---|---|---|
| Ber. | C 63,80 | H 6,69 | N 10,63 |
| Gef. | 63,69 | 6,86 | 10,21 |

(80) 1-[3-(4-Amidino-phenyl)-propionyl]-6-(N-pentanoyl-N-benzylamino)-1,2,3,4-tetrahydro-chinolin-hydrochlorid
Ausbeute: 63 % der Theorie,
Hergestellt aus der gemäß Beispiel 11(10) hergestellten Verbindung

| $C_{31}H_{36}N_4O_2$ x HCl x $H_2O$ (551,15) | | | |
|---|---|---|---|
| Ber. | C 67,55 | H 7,13 | N 10,16 |
| Gef. | 66,99 | 7,30 | 10,08 |

(81) 1- [3- (4-Amidino-phenyl) -propionyl] -6- [N- (2-ethoxycarbonylethylcarbonyl)-benzylamino]-1,2,3,4-tetrahydro-chinolinhydrochlorid
Hergestellt aus der gemäß Beispiel 11(11) hergestellten Verbindung
Ausbeute: 66 % der Theorie,

| $C_{32}H_{36}N_4O_4$ x HCl x 0,5 $H_2O$ (586,16) | | | |
|---|---|---|---|
| Ber. | C 65,57 | H 6,53 | N 9,55 |
| Gef. | 65,07 | 6,34 | 9,79 |

(82) 1-[3-(4-Amidino-phenyl)-propionyl]-6-(N-methanesulfonylbenzylamino)-1,2,3,4-tetrahydro-chinolin-hydrochlorid
Hergestellt aus der gemäß Beispiel 10(22) hergestellten Verbindung
Ausbeute: 60 % der Theorie,

| $C_{27}H_{30}N_4O_3S$ x HCl (527,12) | | | |
|---|---|---|---|
| Ber. | C 61,52 | H 5,92 | N 10,62 |
| Gef. | 61,18 | 6,26 | 10,45 |

(83) 1-(3-(4-Amidino-phenyl)-propionyl]-6-(N-(ethylaminocarbonylmethyl)-benzylamino]-1,2,3,4-tetrahydro-chinolin-hydrochlorid
Hergestellt aus der gemäß Beispiel 14 hergestellten Verbindung Ausbeute: 57 % der Theorie,

| $C_{30}H_{35}N_5O_2$ x HCl x 2 $H_2O$ (570,14) | | | |
|---|---|---|---|
| Ber. | C 63,20 | H 7,00 | N 12,28 |
| Gef. | 63,06 | 7,00 | 11,61 |

(84) 1-[3-(4-Amidino-phenyl)-propionyl]-6-[N-(N,N-dipropylaminocarbonylmethyl)-benzylamino]-1,2,3,4-tetrahydro-chinolin-hydrochlorid
Hergestellt aus der gemäß Beispiel 14(1) hergestellten Verbindung
Ausbeute: 62 % der Theorie,

| $C_{34}H_{43}N_5O_2$ x HCl x $H_2O$ (608,25) | | | |
|---|---|---|---|
| Ber. | C 67,14 | H 7,62 | N 11,51 |
| Gef. | 67,72 | 7,67 | 11,17 |

(85) 1-[3-(4-Amidino-phenyl)-propionyl)-6-[N-(benzylaminocarbonylmethyl)-benzylamino)-1,2,3,4-tetrahydro-chinolinhydrochlorid
Hergestellt aus der gemäß Beispiel 15(2) hergestellten Verbindung
Ausbeute: 50 % der Theorie,

| $C_{35}H_{37}N_5O_2$ x HCl x 2 $H_2O$ (632,21) | | | |
|---|---|---|---|
| Ber. | C 66,49 | H 6,69 | N 11,07 |
| Gef. | 66,65 | 6,78 | 10,56 |

(86) 1-[3-(4-Amidino-phenyl)-propionyl]-6-[N-(phenylaminocarbonylmethyl)-benzylamino]-1,2,3,4-tetrahydro-chinolin-hydrochlorid
Hergestellt aus der gemäß Beispiel 15(3) hergestellten Verbindung
Ausbeute: 78 % der Theorie,

| $C_{34}H_{35}N_5O2$ x HCl x $H_2O$ (600,19) | | | |
|---|---|---|---|
| Ber. | C 68,04 | H 6,38 | N 11,66 |
| Gef. | 67,92 | 6,58 | 11,37 |

(87) 1-[3-(4-Amidino-phenyl)-propionyl]-6-phenylaminosulfonyl-1,2,3,4-tetrahydro-chinolin-hydrochlorid
Hergestellt aus der gemäß Beispiel 16b) hergestellten Verbindung
Ausbeute: 21 % der Theorie,

| $C_{25}H_{26}NO_3S$ x 2 HCl x $H_2O$ (553,51) | | | |
|---|---|---|---|
| Ber. | C 54,25 | H 5,46 | N 10,12 |
| Gef. | 54,52 | 5,54 | 10,16 |

(88) 1-[3-(4-Amidino-phenyl)-propionyl]-6-benzylaminosulfonyl-1,2,3,4-tetrahydro-chinolin-hydrochlorid
Hergestellt aus der gemäß Beispiel 16(1) hergestellten Verbindung
Ausbeute: 28 % der Theorie,

| $C_{26}H_{28}N_4O_3S$ x HCl x 2 $H_2O$ (549,09) | | | |
|---|---|---|---|
| Ber. | C 56,87 | H 6,06 | N 10,20 |
| Gef. | 57,40 | 5,74 | 9,94 |

(89) 1-[3-(4-Amidino-phenyl)-propionyl]-6-phenylsulfonyl-1,2,3,4-tetrahydro-chinolin-hydrochlorid
Hergestellt aus der gemäß Beispiel 16(2) hergestellten Verbindung
Ausbeute: 48 % der Theorie,

| $C_{25}H_{25}N_3O_3S$ x HCl x $H_2O$ (502,01) | | | |
|---|---|---|---|
| Ber. | C 59,81 | H 5,62 | N 8,37 |
| Gef. | 59,94 | 5,59 | 8,26 |

(90) 1-[3-(4-Amidino-phenyl)-propionyl]-6-benzoyl-1,2,3,4-tetrahydro-chinolin-hydrochlorid
Hergestellt aus der gemäß Beispiel 16(3) hergestellten Verbindung
Ausbeute: 56 % der Theorie,

| $C_{26}H_{25}N_3O_2$ x HCl x 1,5 $H_2O$ (474,95) | | | |
|---|---|---|---|
| Ber. | C 65,75 | H 6,15 | N 8,85 |
| Gef. | 66,12 | 5,93 | 9,05 |

(91) 1-[3-(4-Amidino-phenyl)-propionyl]-6-(2,2-diphenyl-ethylamino)-1,2,3,4-tetrahydro-chinolin-hydrochlorid
Hergestellt aus der gemäß Beispiel 14(4) hergestellten Verbindung
Ausbeute: 82 % der Theorie,

| $C_{33}H_{34}N_4O$ x HCl x 2 $H_2O$ (575,16) | | | |
|---|---|---|---|
| Ber. | C 68,91 | H 6,81 | N 9,74 |
| Gef. | 67,62 | 6,82 | 9,26 |

(92) 1- [3- (4-Amidino-phenyl) -propionyl] -6- (N-ethoxycarbonylmethyl-2,2-diphenylethylamino)-1,2,3,4-tetrahydro-chinolin-hydrochlorid
Hergestellt aus der gemäß Beispiel 6(4) hergestellten Verbindung
Ausbeute: 84 % der Theorie,

| $C_{37}H_{40}N_4O_3$ x HCl x $H_2O$ (643,25) | | | |
|---|---|---|---|
| Ber. | C 68,62 | H 6,73. | N 8,71 |
| Gef. | 69,13 | 6,75 | 8,83 |

(93) 1-[3-(4-Amidino-phenyl)-propionyl]-6-(2-oxo-pyrrolidino)-1,2,3,4-tetrahydro-chinolin-hydrochlorid
Hergestellt aus der gemäß Beispiel 11(12) hergestellten Verbindung
Ausbeute: 59 % der Theorie,

| $C_{23}H_{26}N_4O_2$ x HCl x 1,5 $H_2O$ (453,98) | | | |
|---|---|---|---|
| Ber. | C 60,84 | H 6,66 | N 12,34 |
| Gef. | 60,86 | 6,88 | 11,45 |

(94) 1-[3-(4-Amidino-phenyl)-propionyll-6-(2-oxo-piperidino)-1,2,3,4-tetrahydro-chinolin-hydrochlorid
Hergestellt aus der gemäß Beispiel 11(13) hergestellten Verbindung
Ausbeute: 59 % der Theorie,

| $C_{24}H_{28}N_4O_2$ x HCl x 1,5 $H_2O$ (468,01) | | | |
|---|---|---|---|
| Ber. | C 61,59 | H 6,90 | N 11,97 |
| Gef. | 61,78 | 7,02 | 11,46 |

(95) 1-[N-(4-Amidino-benzyl)-methylaminocarbonyl]-6-phenylsulfonylamino-1,2,3,4-tetrahydro-chinolin-hydrochlorid
Hergestellt aus der gemäß Beispiel 17 hergestellten Verbindung Ausbeute: 59 % der Theorie,
$C_{25}H_{27}N_5O_3S$ (477,60)
Massenspektrum: FAB-MS: $(M+H)^+$ = 478

Beispiel 25

1-[3-(4-Amidino-phenyl)-propionyl]-6-[N-(3-methoxycarbonylpropyl)-naphth-1-yl-sulfonylamino]-1,2,3,4-tetrahydro-chinolin

0,89 g 1-[3-(4-Benzyloxycarbonylamidino-phenyl)-propionyl]-6-[N-(3-methoxycarbonylpropyl)-naphth-1-yl-sulfonylamino]-1,2,3,4-tetrahydro-chinolin (siehe Beispiel 7(3)) werden in 30 ml Methanol und 1,1 ml 1 N Salzsäure gelöst und über Palladiumkohle bei Raumtemperatur 3 Stunde mit 3 bar Wasserstoff hydriert. Anschließend filtriert man vom Katalysator ab, engt die Lösung ein und chromatographiert den Rückstand über eine Kieselgelsäule mit

Methylenchlorid/Methanol (8,5:1,5). Die Hauptfraktion wird einrotiert und das erhaltene Material getrocknet. Ausbeute: 0,39 g (53% der Theorie),

| $C_{34}H_{36}N_4O_5S$ x HCl x $H_2O$ (667,23) | | | |
|---|---|---|---|
| Ber. | C 61,21 | H 5,89 | N 8,40 |
| Gef. | 61,50 | 5,98 | 8,52 |

Analog werden hergestellt:

(1) 1-[3-(4-Amidino-phenyl)-propionyl]-6-[N-(1-ethoxycarbonylethyl)-naphth-1-yi-sulfonylamino]-1,2,3,4-tetrahydro-chinolinhydrochlorid
Ausbeute: 63 % der Theorie,

| $C_{34}H_{36}N_4O_5S$ x HCl x $H_2O$ (667,23) | | | |
|---|---|---|---|
| Ber. | C 61,21 | H 5,89 | N 8,40 |
| Gef. | 60,46 | 5,71 | 8,18 |

(2) 1-[3-(4-Amidino-phenyl)-propionyl]-6-[N-(naphth-1-yl-sulfonyl-methylamino]-1,2,3,4-tetrahydro-chinolin-hydrochlorid
Ausbeute: 70 % der Theorie,

| $C_{30}H_{30}N_4O_3S$ x HCl x 1,5 $H_2O$ (590,15) | | | |
|---|---|---|---|
| Ber. | C 61,06 | H 5,81 | N 9,49 |
| Gef. | 60,73 | 5,74 | 9,51 |

(3) 1-[3-(4-Amidino-phenyl)-propionyl]-6-[N-(naphth-1-yl-sulfonyl-benzylamino]-1,2,3,4-tetrahydro-chinolin-hydrochlorid
Ausbeute: 81 % der Theorie,

| $C_{36}H_{34}N_4O_3S$ x HCl x 1,5 $H_2O$ (666,25) | | | |
|---|---|---|---|
| Ber. | C 64,90 | H 5,75 | N 8,41 |
| Gef. | 65,09 | 5,78 | 8,44 |

(4) 1-[3-(4-Amidino-phenyl)-propionyl]-6-[N-(naphth-l-yl-sulfonyl)-N-(2-morpholinoethyl)-amino]-1,2,3,4-tetrahydro-chinolin-hydrochlorid
Hergestellt aus 1-[3-(4-Benzyloxycarbonylamidino-phenyl)-propionyl]-6-[N-(naphth-1-yl-sulfonyl)-N-(2-morpholinoethyl)-amino]-1,2,3,4-tetrahydro-chinolin
Ausbeute: 40 % der Theorie,

| $C_{35}H_{37}N_5O_5S$ x HCl x 2,5 $H_2O$ (721,28) | | | |
|---|---|---|---|
| Ber. | C 58,28 | H 6,01 | N 9,71 |
| Gef. | 58,21 | 5,88 | 9,48 |

(5) 1-[3-(4-Amidino-phenyl)-propionyl]-6-[N-(naphth-1-yl-sulfonyl)-N-(ethoxycarbonylmethyl)-amino]-1,2,3,4-tetrahydro-chinolin-hydrochlorid
Ausbeute: 70 % der Theorie,

| $C_{33}H_{34}N_4O_5S$ x HCl x $H_2O$ (653,20) | | | |
|---|---|---|---|
| Ber. | C 60,68 | H 5,71 | N 8,58 |
| Gef. | 60,01 | 5,69 | 8,54 |

(6) l-[3-(4-Amidino-phenyl)-propionyl]-6-[N-(naphth-l-yl-sulfonyl)-N,N-(di(methoxycarbonylmethyl)-aminocarbonylmethyl)-amino]-1,2,3,4-tetrahydro-chinolin
Hergestellt aus der gemäß Beispiel 7(8) hergestellten Verbindung
Ausbeute: 67 % der Theorie,

| $C_{37}H_{39}N_5O_8S$ x HCl x $H_2O$ (768,29) | | | |
|---|---|---|---|
| Ber. | C 57,84 | H 5,51 | N 9,12 |
| Gef. | 57,49 | 5,52 | 9,07 |

Beispiel 26

1-[3-(4-Amidino-phenyl}-propionyl]-6-(2,4,6-trimethyl-benzolsulfonylamino)-1,2,3,4-tetrahydro-chinolin-hydrojodid

1, 32 g (2,7 mMol) 1- [3- (4-Cyano-phenyl) -propionyl] -6- (2, 4, 6-trimethyl-benzolsulfonylamino)-1,2,3,4-tetrahydro-chinolin (siehe Beispiel 10(13)) werden in 20 ml Pyridin gelöst und mit 2 ml Triethylamin versetzt. Anschließend werden unter Eiskühlung 3,6 g Schwefelwasserstoff eingeleitet und die Lösung über Nacht gerührt. Dann wird 2 Stunden Stickstoff durch die Lösung geblasen und anschließend eingeengt. Den Rückstand nimmt man mit Eis und 3 ml konzentrierter Salzsäure auf und extrahiert 3 mal mit Essigester. Die organsiche Phase wird getrocknet und einrotiert. Das rohe Thioamidderivat wird in 50 ml Aceton gelöst und 2,5 Stunden mit 8 ml Methyljodid bei 45°C gerührt und schließlich einrotiert. Dann wird der Rückstand in 50 ml Methanol oder Ethanol gelöst, mit 3 g Ammoniumacetat versetzt, und 6 Stunden bei 45°C gerührt und dann eingeengt. Das entstehende Amidin wird über eine Kieselgelsäule gereinigt.
Ausbeute: 46 % der Theorie,
Schmelzpunkt: ab 124°C

| $C_{28}H_{32}N_4O_3S$ x HJ (632,57) | | | |
|---|---|---|---|
| Ber. | C 53,17 | H 5,26 | N 8,86 |
| Gef: | 51,05 | 5,44 | 8,57 |

Analog werden hergestellt:

(1) 1-[3-(4-Amidino-phenyl)-propionyl]-6-[N-(chinolin-8-sulfonyl)-N-(ethoxycarbonylmethyl)-amino]-1,2,3,4-tetrahydro-chinolin-hydrojodid
Hergestellt aus 1-[3-(4-Cyano-phenyl)-propionyl]-6-[N-(chinolin-8-sulfonyl)-N-(ethoxycarbonylmethyl)-aminol-1,2,3,4-tetrahydro-chinolin (siehe Beispiel 10(20)).
Ausbeute: 48 % der Theorie,

| $G_{32}H_{33}JN_5O_5S$ x HJ x 0,5 $H_2O$ (736,63) | | | |
|---|---|---|---|
| Ber. | C 52,18 | H 4,79 | N 9,51 |
| Gef: | 52,13 | 4,90 | 9,39 |

(2) 1-[3-(4-Amidino-phenyl)-propionyl]-6-[N-(isochinolin-5-sulfonyl)-N-(ethoxycarbonylmethyl)-amino)-1,2,3,4-tetrahydro-chinolin-hydrojodid
Hergestellt aus l-[3-(4-Cyano-phenyl)-propionyl]-6-[N-(isochinolin-5-sulfonyl)-N-(ethoxycarbonylmethyl)-amino)-1,2,3,4-tetrahydro-chinolin (siehe Beispiel 7)
Ausbeute: 17 % der Theorie,

| $C_{32}H_{33}N_5O_5S$ x HJ x $H_2O$ (745,63) | | | |
|---|---|---|---|
| Ber. | C 51,55 | H 4,87 | N 9,39 |
| Gef: | 51,30 | 4,96 | 8,87 |

(3) 1-[3-(4-Amidino-phenyl)-propionyl]-6-[N-(phenylmethansulfonyl)-N-(ethoxycarbonylmethyl)-amino]-1,2,3,4-tetrahydrochinolin-hydrojodid
Hergestellt aus der gemäß Beispiel 10(24) hergestellten Verbindung
Ausbeute: 57 % der Theorie,

Schmelzpunkt: sintert ab 110°C

| $C_{30}H_{34}N_4O_5S$ x HJ x 0,5 $H_2O$ (699,61) | | | |
|---|---|---|---|
| Ber. | C 51,50 | H 5,19 | N 8,01 |
| Gef: | 51,52 | 5,27 | 7,79 |

(4) 1-[3-(4-Amidino-phenyl)-propionyl]-6-[N-(n-butylsulfonyl)-N-(ethoxycarbonylmethyl)-amino]-1,2,3,4-tetrahydro-chinolinhydrojodid
Hergestellt aus der gemäß Beispiel 7(1) hergestellten Verbindung
Ausbeute: 56 % der Theorie,

| $C_{27}H_{36}N_4O_5S$ x HJ (656,59) | | | |
|---|---|---|---|
| Ber. | C 49,39 | H 5,68 | N 8,53 |
| Gef: | 48,88 | 5,77 | 8,38 |

(5) 1-[(4-Amidino-phenoxy)-acetyl]-6-[N-(1-naphthylsulfonyl)-ethoxycarbonylmethylamino]-1,2,3,4-tetrahydro-chinolin-hydrojodid
Hergestellt aus der gemäß Beispiel 9(27) hergestellten Verbindung
Ausbeute: 48 % der Theorie,

| $C_{32}H_{32}N_4O_6S$ x HJ (728,59) | | | |
|---|---|---|---|
| Ber. | C 52,75 | H 4,56 | N 7,69 |
| Gef: | 52,33 | 4,79 | 7,52 |

(6) 1-[3-(4-Amidino-phenyl)-propionyl]-6-(N-benzoyl-ethoxycarbonylmethylamino]-1,2,3,4-tetrahydro-chinolin-hydrojodid Hergestellt aus der gemäß Beispiel 11 hergestellten Verbindung
Ausbeute: 35 % der Theorie,
Schmelzpunkt: 130°C unter Zersetzung

| $C_{30}H_{32}N_4O_4$ x HJ x $H_2O$ (658,54) | | | |
|---|---|---|---|
| Ber. | C 54,72 | H 5,36 | N 8,51 |
| Gef: | 55,04 | 5,45 | 8,41 |

(7) 1-[3-(4-Amidino-phenyl)-propionyl]-6-[N-(naphtho-1-yl)-ethoxycarbonylmethylamino]-1,2,3,4-tetrahydro-chinolin-hydrojodid
Hergestellt aus der gemäß Beispiel 11(1) hergestellten Verbindung
Ausbeute: 60 % der Theorie,

| $C_{34}H_{34}N_4O_4$ x HJ x $H_2O$ (708,60) | | | |
|---|---|---|---|
| Ber. | C 57,63 | H 5,26 | N 17,81 |
| Gef: | 57,59 | 5,33 | 18,87 |

(8) 1- [3- (4-Amidino-phenyl) -propionyl] -6- [N- (2-naphthylsulfonyl)-ethoxycarbonylmethylamino]-1,2,3,4-tetrahydro-chinolinhydrojodid
Hergestellt aus der gemäß Beispiel 10(21) hergestellten Verbindung
Ausbeute: 51 % der Theorie,
Schmelzpunkt: Zersetzung ab 120°C

| $C_{33}H_{34}N_4O_5S$ x HJ (726,64) | | | |
|---|---|---|---|
| Ber. | C 54,55 | H 4,86 | N 7,71 |
| Gef: | 53,63 | 4,97 | 7,63 |

(9) 1-[3-(4-Amidino-3-methyl-phenyl)-propionyl]-6-phenylsulfonylamino-1,2,3,4-tetrahydro-chinolin-hydrojodid
Hergestellt aus der gemäß Beispiel 9(30) hergestellten Verbindung
Ausbeute: 10 % der Theorie.
$R_f$-Wert: 0,48 (Kieselgel; Methylenchlorid/Ethanol = 9:1)

Beispiel 27

1-[3-[4-(N-Methoxycarbonyl-amidino)phenyll-propionyll-6-phenylsulfonamido-1,2,3,4-tetrahydro-chinolin

630 mg 1-[3-(4-Amidino-phenyl)propionyl)-6-phenylsulfonamido-1,2,3,4-tetrahydro-chinolin-hydrochlorid werden in 10 ml Tetrahydrofuran und 1 ml Wasser gelöst, dann werden 260 mg Natriumcarbonat zugegeben. Schließlich tropft man 120 mg Chlorameisensäuremethylester in 1,5 ml Tetrahydrofuran zu und rührt 4 Stunden weiter. Anschließend versetzt man mit 20 ml Wasser und 30 ml Essigester. Nun wird die organische Phase abgetrennt, mit Wasser gewaschen, getrocknet und eingeengt. Der Rückstand wird mit einem Essigester/Methylenchlorid-Gemisch (7:3) über eine Kieselgelsäule chromatographiert.
Ausbeute: 350 mg (56 % der Theorie),
Schmelzpunkt: Schaum

| $C_{27}H_{29}N_4O_5S$ (520,61) | | | | |
|---|---|---|---|---|
| Ber. | C 62,29 | H 5,42 | N 10,76 | S 6,16 |
| Gef. | 61,85 | 5,61 | 10,00 | 6,40 |

Beispiel 28

1-[3-(4-Amidino-phenyl)propionyl]-6-methylamino-1,2,3,4-tetrahydro-chinolin-hydrochlorid und 1-[3-(4-Amidino-phenyl)propionyl]-6-(N-methyl-phenylsulfonamido)-1,2,3,4-tetrahydro-chinolin-hydrochlorid

Hergestellt analog Beispiel 24 aus 1-[3-(4-Cyano-phenyl)propionyl]-6-(N-methyl-phenylsulfonamido)-1,2,3,4-tetrahydrochinolin (siehe Beispiel 9(8)) und anschließende chromatographische Auftrennung über eine Kieselgelsäule unter Verwendung von Methylenchlorid/Methanol (8:2).

a) 1-[3-(4-Amidino-phenyl)propionyl]-6-methylamino-l,2,3,4-tetrahydro-chinolin-hydrochlorid
Ausbeute: 25 % der Theorie,
Schmelzpunkt: sintert ab 130°C

| $C_{20}H_{24}N_4O_3$ (390,92) x HCl (390,92) | | | |
|---|---|---|---|
| Ber. | C 61,45 | H 6,96 | N 14,33 |
| Gef. | 61,27 | 6,91 | 14,05 |

b) Z-[3-(4-Amidino-phenyl)propionylj-6-(N-methyl-phenylsulfonamido)-1,2,3,4-tetrahydro-chinolin-benzolsulfonat
Ausbeute: 6 % der Theorie,
Schmelzpunkt: sintern ab 90°C

| $C_{26}H_{28}N_4O_3S$ x $C_6H_5SO_3H$ x $H_2O$ (652,79) | | | |
|---|---|---|---|
| Ber. | C 58,88 | H 5,56 | N 8,58 |
| Gef. | 58,53 | 5,37 | 7,94 |

Analog werden hergestellt:

(1a) 1-[3-(4-Amidino-phenyl)propionyl)-6-(2-phenylethylamino)-1,2,3,4-tetrahydro-chinolin-hydrochlorid
Hergestellt aus der gemäß Beispiel 9(11) hergestellten Verbindung
Ausbeute: 22 % der Theorie,
Schmelzpunkt: ab 90°C

| $C_{27}H_{30}N_4O$ x 2,5 HCl x 2 $H_2O$ (553,76) | | | |
|---|---|---|---|
| Ber. | C 58,56 | H 6,64 | N 10,12 |
| Gef. | 56,69 | 6,30 | 9,64 |

(1b) I-[3-(4-Amidino-phenyl)propionyl]-6-[N-(2-phenylethyl)-phenylsulfonamido]-1,2,3,4-tetrahydro-chinolin-hydrochlorid
Ausbeute: 41 % der Theorie,
Schmelzpunkt: ab 105°C

| $C_{33}H_{39}N_4O_3S$ x HCl x 0,5 $H_2O$ (612,20) | | | | |
|---|---|---|---|---|
| Ber. | C 64,75 | H 5,93 | N 9,15 | S 5,24 |
| Gef. | 64,66 | 6,04 | 9,01 | 5,51 |

(2a) 1-[3-(4-Amidino-phenyl)propionyl]-6-(N-ethoxycarbonylmethylamino)-1,2,3,4-tetrahydro-chinolin-hydrochlorid Hergestellt aus der gemäß Beispiel 9(10) hergestellten Verbindung
Ausbeute: 13,0 % der Theorie,
Schmelzpunkt: ab 135°C (Zers.)

| $C_{23}H_{28}N_4O_3$ x HCl x 7,5 $H_2O$ (471,99) | | | |
|---|---|---|---|
| Ber. | C 58,53 | H 6,83 | N 11,87 |
| Gef. | 58,44 | 6,44 | 11,58 |

(2b) 1-[3-(4-Amidino-phenyl)propionyl]-6-(N-ethoxycarbonylmethyl-phenylsulfonamido)-1,2,3,4-tetrahydro-chinolin-benzolsulfonat
Ausbeute: 21 % der Theorie,
Schmelzpunkt: ab 105°C

| $C_{29}H_{32}N_4O_5S$ x 0,5 $C_6H_5SO_3H$ x $H_2O$ x 0,5 HCl (664,00) | | | |
|---|---|---|---|
| Ber. | C 57,83 | H 5,69 | N 8,44 |
| Gef. | 56,96 | 5,60 | 8,47 |

Beispiel 29

1-[3-(4-Amidino-phenyl)propionyl]-6-(N-carboxymethyl-phenylsulfonamido)-1,2,3,4-tetrahydro-chinolin

370 mg 1-[3-(4-Amidino-phenyl)propionyl]-6-(N-ethoxycarbonylmethyl-phenylsulfonamido)-1,2,3,4-tetrahydro-chinolin werden in 5 ml Ethanol gelöst und mit 1,6 ml 1N Natronlauge über Nacht gerührt. Anschließend wird mit Salzsäure neutralisiert, eingeengt und der Rückstand über eine Kieselgelsäule mit Methanol chromatographiert.
Ausbeute: 190 mg (65 % der Theorie),

| $C_{27}H_{28}N_4O_5S$ (520,61) | | | |
|---|---|---|---|
| Ber. | C 62,29 | H 5,42 | N 10,76 |
| Gef. | 61,13 | 5,59 | 10,48 |

Beispiel 30

1-[3-(4-Aminomethyl-phenyl)propionyl]-6-phenylsulfonamido-1,2,3,4-tetrahydro-chinolin

900 mg 1-[3-(4-Cyano-phenyl)propionyl]-6-phenylsulfonamido-1,2,3,4-tetrahydro-chinolin werden in 20 ml methanolischer Ammoniaklösung bei 3 bar 17 Stunden lang über Raney-Nickel hydriert. Anschließend wird vom Katalysator abgesaugt, eingeengt, der erhaltene Rückstand in Wasser gelöst und alkalisch gestellt. Es wird mit Methylenchlorid extrahiert, die organische Phase abgetrennt, getrocknet und im Vakuum eingeengt. Ausbeute: 0,6 g (67 %

der Theorie),
Schmelzpunkt: 130-132°C

| $C_{25}H_{27}N_3O_3S$ (449,58) | | | | |
|---|---|---|---|---|
| Ber. | C 66,79 | H 6,05 | N 9,35 | S 7,13 |
| Gef. | 66,73 | 6,16 | 9,44 | 7,12 |

Analog werden hergestellt:

(1) 1-[3-(4-Aminomethyl-phenyl)propionyl]-6-(1-naphthyl-sulfonamido)-1,2,3,4-tetrahydro-chinolin

(2) 1-[3-(4-Aminomethyl-phenyl)propionyl]-6-(4-fluor-phenylsulfonamido)-1,2,3,4-tetrahydro-chinolin

(3) 1-[3-(4-Aminomethyl-phenyl)propionyl]-6-butylsulfonamido-1,2,3,4-tetrahydro-chinolin

(4) 1-[3-(4-Aminomethyl-phenyl)propionyl]-6-(N-methyl-phenylsulfonamido)-1,2,3,4-tetrahydro-chinolin

(5) 1-[3-(4-Aminomethyl-phenyl)propionyl]-6-benzylsulfon-amido-1,2,3,4-tetrahydro-chinolin

(6) 1-[3-(4-Aminomethyl-phenyl)propionyl]-6-benzoylamino-1,2,3,4-tetrahydro-chinolin

Beispiel 31

1-[3-(4-Amidino-phenyl)-propionyl]-6-[N-(chinolin-8-sulfonyl)-hydroxycarbonylmethylamino]-1,2,3,4-tetrahydro-chinolin

590 mg 1-[3-(4-Amidino-phenyl)-propionylj-6-[N-(chinolin-8-sulfonyl)-N-ethoxycarbonylmethyl-amino]-1,2,3,4-tetrahydrochinolin (siehe Beispiel 26(1)) werden in 15 ml Ethanol und 2,4 ml 1N Natronlauge gelöst und 2,5 Stunden bei Raumtemperatur gerührt. Anschließend wird mit 0,1N Salzsäure neutralisiert und eingeengt. Der Rückstand wird mit Wasser und Ethanol verrührt und getrocknet.
Ausbeute: 200 mg (41 % der Theorie),
Schmelzpunkt: 215-217°C unter Zersetzung

| $C_{30}H_{29}N_5O_5S$ x 1,5 $H_2O$ (598,68) | | | |
|---|---|---|---|
| Ber. | C 60,19 | H 5,39 | N 11,70 |
| Gef. | 59,70 | 5,45 | 11,34 |

Analog werden hergestellt:

(1) 1-[3-(4-Amidino-phenyl)-propionyl]-6-[N-(isochinolin-5-sulfonyl)-hydroxycarbonylmethylamino]-1,2,3,4-tetraydrochinolin
Hergestellt aus der gemäß Beispiel 26(2) hergestellten Verbindung
Ausbeute: 45 % der Theorie,
Schmelzpunkt: 213-215°C unter Zersetzung

| $C_{30}H_{29}N_5O_5S$ x 1,5 $H_2O$ (598,68) | | | |
|---|---|---|---|
| Ber. | C 60,19 | H 5,39 | N 11,70 |
| Gef. | 60,20 | 5,39 | 11,37 |

(2) 1- [3- (4-Amidino-phenyl) -propionyl] -6- [N- (phenylmethan-sulfonyl)-hydroxycarbonylmethylamino]-1,2,3,4-tetrahydro-chinolin
Hergestellt aus der gemäß Beispiel 26(3) hergestellten Verbindung
Ausbeute: 81 % der Theorie,

| $C_{28}H_{30}N_4O_5S$ x $H_2O$ (552,64) | | | |
|---|---|---|---|
| Ber. | C 60,85 | H 5,84 | N 10,14 |
| Gef. | 61,25 | 5,94 | 10,16 |

(3) 1-[3-(4-Amidino-phenyl)-propionyl]-6-[N-(n-butylsulfonyl)-hydroxycarbonylmethylamino]-1,2,3,4-tetrahydro-chinolin
Hergestellt aus der gemäß Beispiel 26(4) hergestellten Verbindung
Ausbeute: 68 % der Theorie,

| $C_{25}H_{32}N_4O_5S$ x 0,5 $H_2O$ (509,63) | | | |
|---|---|---|---|
| Ber. | C 58,92 | H 6,53 | N 10,99 |
| Gef. | 58,82 | 6,61 | 10,96 |

(4) 1-[(4-Amidino-phenoxy)-acetyl]-6-[N-(1-naphthylsulfonyl)-hydroxycarbonylmethylamino]-1,2,3,4-tetrahydro-chinolin-hydrochlorid
Hergestellt aus der gemäß Beispiel 26(5) hergestellten Verbindung
Ausbeute: 59 % der Theorie,

| $C_{30}H_{28}N_4O_6S$ x HCl x 0,5 $H_2O$ (618,14) | | | |
|---|---|---|---|
| Ber. | C 57,17 | H 5,18 | N 8,51 |
| Gef. | 57,45 | 4,82 | 8,93 |

(5) 1-[3-(4-Amidino-phenyl)-propionyl]-6-(N-benzoyl-hydroxycarbonylmethylamino]-1,2,3,4-tetrahydro-chinolin
Hergestellt aus der gemäß Beispiel 26(6) hergestellten Verbindung
Ausbeute: 31 % der Theorie,

| $C_{28}H_{28}N_4O_4$ x $H_2O$ (658,54) | | | |
|---|---|---|---|
| Ber. | C 66,92 | H 6,02 | N 11,15 |
| Gef. | 66,23 | 6,18 | 10,98 |

(6) 1-[3-(4-Amidino-phenyl)-propionyl]-6-[N-(1-naphthoyl)-hydroxycarbonylmethylamino]-1,2,3,4-tetrahydro-chinolin
Hergestellt aus der gemäß Beispiel 26(7) hergestellten Verbindung

| $C_{32}H_{30}N_4O_4$ x $H_2O$ (552,63) | | | |
|---|---|---|---|
| Ber. | C 69,55 | H 5,84 | N 10,14 |
| Gef. | 68,84 | 5,84 | 9,93 |

(7) 1-[3-(4-Amidino-2-(2-hydroxycarbonylethylcarbonylamino)-phenyl)-propionyl]-6-phenylsulfonylamino-1,2,3,4-tetrahydrochinolin-hydrochlorid
Hergestellt aus der gemäß Beispiel 24(47) hergestellten Verbindung
Ausbeute: 87 % der Theorie,

(8) 1- [3- (4-Amidino-phenyl) -propionyl] -6- [N- (naphth-2-yl-sulfonyl)-ethoxycarbonylmethylamino]-1,2,3,4-tetrahydro-chinolin
Ausbeute: 82 % der Theorie,

| $C_{31}H_{30}N_4O_5S$ x 1,5 $H_2O$ (597,70) | | | |
|---|---|---|---|
| Ber. | C 62,30 | H 5,57 | N 9,37 |
| Gef. | 62,15 | 5,47 | 9,39 |

(9) 1-[3-(4-Amidino-phenyl)-propionyl]-6-[N-(naphth-1-yl-sulfonyl)-N-(3-hydroxycarbonylpropyl)-amino]-l,2,3,4-tetrahydrochinolin
Hergestellt aus der gemäß Beispiel 25 hergestellten Verbindung Ausbeute: 48 % der Theorie,
$C_{33}H_{34}N_4O_5S$ (598,72)
Massenspektrum: FAS-MS: $(M+H)^+ = 599$

(10) 1-[3-(4-Amidino-phenyl)-propionyl]-6-[N-(naphth-1-yl-sulfonyl)-N-(1-hydroxycarbonylethyl)-amino]-1,2,3,4-tetrahydrochinolin-hydrochlorid
Hergestellt aus der gemäß Beispiel 25(1) hergestellten Verbindung
Ausbeute: 80 % der Theorie,

| $C_{32}H_{32}N_4O_5S$ x HCl x 1,5 $H_2O$ (611,72) | | | |
|---|---|---|---|
| Ber. | C 62,83 | H 5,77 | N 9,16 |
| Gef. | 62,97 | 5,73 | 9,16 |

(11) 1-[3-(4-Amidino-phenyl)-propionyl]-6-[N-(naphth-1-yl-sulfonyl)-hydroxycarbonylmethylamino]-1,2,3,4-tetrahydro-chinolin-hydrochlorid
Hergestellt aus der gemäß Beispiel 25(2) hergestellten Verbindung
Ausbeute: 77 % der Theorie,

| $C_{31}H_{30}N_4O_5S$ x HCl x $H_2O$ (625,15) | | | |
|---|---|---|---|
| Ber. | C 63,25 | H 5,48 | N 9,52 |
| Gef. | 63,45 | 5,83 | 9,76 |

(12) 1-[3-(4-Amidino-phenyl)-propionyl)-6-[N-(naphth-1-yl-sulfonyl)-N-(N,N-di(hydroxycarbonylmethyl)-aminocarbonyl)-amino]-1,2,3,4-tetrahydro-chinolin-hydrochlorid
Hergestellt aus der gemäß Beispiel 25(6) hergestellten Verbindung
Ausbeute: 91 % der Theorie,

| $C_{35}H_{35}N_5O_8S$ x $H_2O$ (703,78) | | | |
|---|---|---|---|
| Ber. | C 59,73 | H 5,30 | N 9,95 |
| Gef. | 59,92 | 5,25 | 9,97 |

(13) 1-[3-(4-Amidino-phenyl)-propionyl]-6-(N-hydroxycarbonylmethyl-phenylamino)-1,2,3,4-tetrahydro-chinolin-hydrochlorid
Hergestellt aus der gemäß Beispiel 24(62) hergestellten Verbindung
Ausbeute: 26 % der Theorie,

| $C_{28}H_{28}N_4O_4$ x HCl x 1,5 $H_2O$ (548,02) | | | |
|---|---|---|---|
| Ber. | C 61,36 | H 5,89 | N 10,12 |
| Gef. | 61,34 | 5,67 | 9,85 |

(14) 1-[3-(4-Amidino-phenyl)-propionyl]-6-(N-hydroxycarbonylmethyl-cyclohexylaminocarbonyl)-1,2,3,4-tetrahydro-chinolinhydrochlorid
Hergestellt aus der gemäß Beispiel 24(66) hergestellten Verbindung
Ausbeute: 58 % der Theorie,

| $C_{28}H_{34}N_4O_4$ x HCl x 0,5 H20 (536,05) | | | |
|---|---|---|---|
| Ber. | C 62,74 | H 6,77 | N 10,45 |
| Gef. | 63,24 | 6,78 | 9,76 |

(15) 1-[3-(4-Amidino-phenyl)-propionyl]-6-(2-hydroxycarbonylpyrrolidino)-1,2,3,4-tetrahydro-chinolin-hydrochlorid

Hergestellt aus der gemäß Beispiel 24(67) hergestellten Verbindung
Ausbeute: 35 % der Theorie,

| $C_{25}H_{28}N_4O_4$ x HCl x $H_2O$ (502,96) | | | |
|---|---|---|---|
| Ber. | C 59,70 | H 6,21 | N 11,16 |
| Gef. | 60,02 | 6,31 | 10,17 |

(16) 1-[3-(4-Amidino-phenyl)-propionyl]-6-(2-hydroxycarbonylpiperidino)-1,2,3,4-tetrahydro-chinolin-hydrochlorid
Hergestellt aus der gemäß Beispiel 24(68) hergestellten Verbindung
Ausbeute: 46 % der Theorie,

| $C_{26}H_{30}N_4O_4$ x HCl x $H_2O$ (517,03) | | | |
|---|---|---|---|
| Ber. | C 60,40 | H 6,43 | N 10,84 |
| Gef. | 60,73 | 6,35 | 9,94 |

(17) 1-[3-(4-Amidino-phenyl)-propionyl]-6-(3-hydroxycarbonylpiperidino)-1,2,3,4-tetrahydro-chinolin-hydrochlorid
Hergestellt aus der gemäß Beispiel 24(69) hergestellten Verbindung
Ausbeute: 47 % der Theorie,

| $C_{26}H_{30}N_4O_4$ x HCl x $H_2O$ (517,03) | | | |
|---|---|---|---|
| Ber. | C 60,40 | H 6,43 | N 10,84 |
| Gef. | 61,28 | 6,50 | 10,39 |

(18) 1-[3-(4-Amidino-phenyl)-propionyl]-6-[N-(naphthalin-2-ylmethyl)-hydroxycarbonylmethylamino]-1,2,3,4-tetrahydro-chinolin-hydrochlorid
Hergestellt aus der gemäß Beispiel 24(77) hergestellten Verbindung
Ausbeute: 70 % der Theorie,

| $C_{32}H_{32}N_4O_3$ x HCl (557,13) | | | |
|---|---|---|---|
| Ber. | C 66,98 | H 5,96 | N 10,05 |
| Gef. | 67,81 | 6,45 | 8,86 |

(19) 1-[3-(4-Amidino-phenyl)-propionyl]-6-[N-(naphth-1-ylylmethyl)-hydroxycarbonylmethylamino]-1,2,3,4-tetrahydro-chinolin-hydrochlorid
Hergestellt aus der gemäß Beispiel 24(78) hergestellten Verbindung
Ausbeute: 69 % der Theorie,

| $C_{32}H_{32}N_4O_3$ x HCl x $H_2O$ (575,13) | | | |
|---|---|---|---|
| Ber. | C 66,82 | H 6,13 | N 9,74 |
| Gef. | 67,43 | 6,75 | 8,46 |

(20) 1-[3-(4-Amidino-phenyl)-propionyl]-6-[N-(2-hydroxycarbonylethylcarbonyl)-benzylamino]-1,2,3,4-tetrahydro-chinolinhydrochlorid
Hergestellt aus der gemäß Beispiel 24(81) hergestellten Verbindung
Ausbeute: 98 % der Theorie,

| $C_{30}H_{32}N_4O_4$ x HCl x $H_2O$ (567,11) | | | |
|---|---|---|---|
| Ber. | C 63,53 | H 6,22 | N 9,87 |
| Gef. | 63,53 | 6,38 | 9,11 |

(21) 1- [3- (4-Amidino-phenyl) -propionyl] -6- (N-hydroxycarbonylmethyl-benzylamino)-1,2,3,4-tetrahydro-chinolin-hydrochlorid

Hergestellt aus der gemäß Beispiel 24(76) hergestellten Verbindung
Ausbeute: 79 % der Theorie,

| $C_{28}H_{30}N_4O_3$ x HCl x 1,5 $H_2O$ (534,07) | | | |
|---|---|---|---|
| Ber. | C 62,97 | H 6,41 | N 10,49 |
| Gef. | 63,17 | 6,83 | 8,67 |

(22) 1-[3-(4-Amidino-phenyl)-propionyl]-6-[N-hydroxycarbonylmethyl-N-(2,2-diphenyl-ethyl)-amino)-1,2,3,4-tetrahydrochinolin-hydrochlorid
Hergestellt aus der gemäß Beispiel 24(92) hergestellten Verbindung
Ausbeute: 90 % der Theorie,

| $C_{35}H_{36}N_4O_3$ x HCl (597,20) | | | |
|---|---|---|---|
| Ber. | C 69,79 | H 6,04 | N 8,46 |
| Gef. | 70,39 | 6,24 | 9,38 |

Beispiel 32

1-[3-(4-Benzyloxycarbonylamidino-phenyl)-propionyl]-6-(1-naphthyl-sulfonamido)-1,2,3,4-tetrahydro-chinolin

15,5 g 1-[3-(4-Amidino-phenyl)propionyl]-6-(1-naphthyl-sulfonamido)-1,2,3,4-tetrahydro-chinolin-hydrochlorid werden in 250 ml Tetrahydrofuran und 25 ml Wasser gelöst und mit 6,9 g Natriumcarbonat versetzt. Dann wird bei Raumtemperatur 5,8 g (0,032 Mol) Chlorameisensäurebenzylester während 30 Minuten zugetropft und die Lösung über Nacht gerührt. Anschließend wird vom Niederschlag abdekantiert, die Lösung auf ca. 50 ml eingeengt und mit Essigsäureethylester dreimal extrahiert. Die organische Phase wird getrocknet, einrotiert und der Rückstand über eine Kieselgelsäule mit Methylenchlorid/Essigsäureethylester (7:3) filtriert.
Ausbeute: 14,0 g (77 % der Theorie),
Schmelzpunkt: 172-174°C
Analog werden hergestellt:

(1) 1-[3-(4-Benzyloxycarbonylamidino-phenyl)-propionyl]-6-phenylsulfonylamino-1,2,3,4-tetrahydro-chinolin
Hergestellt aus der gemäß Beispiel 22 hergestellten Verbindung Ausbeute: 75 % der Theorie,
Schmelzpunkt: 172-174°C

| $C_{33}H_{32}N_4O_5S$ x 0,5 $H_2O$ (605,72) | | | |
|---|---|---|---|
| Ber. | C 65,44 | H 5,49 | N 9,25 |
| Gef. | 65,58 | 5,60 | 8,93 |

(2) 1-[3-(4-Methoxycarbonylamidino-phenyl)-propionyl]-6-[N-(chinolin-8-sulfonyl)-N-ethoxycarbonylmethyl-amino]-1,2,3,4-tetrahydro-chinolin

(3) 1-[3-(4-Octyloxycarbonylamidino-phenyl)-propionyl]-6-[N-(chinolin-8-sulfonyl)-N-ethoxycarbonylmethyl-amino]-1,2,3,4-tetrahydro-chinolin

(4) 1-[3-(4-Hexyloxycarbonylamidino-phenyl)-propionyl]-6-phenylsulfonylamino-1,2,3,4-tetrahydro-chinolin

(5) 1-[3-(4-Ethyloxycarbonylamidino-phenyl)-propionyl]-6-phenylsulfonylamino-1,2,3,4-tetrahydro-chinolin

(6) 1-[3-(4-Heptyloxycarbonylamidino-phenyl)-propionyl]-6-[N-(naphth-1-yl-sulfonyl)-N-ethoxycarbonylmethyl-amino]-1,2,3,4-tetrahydro-chinolin-6-yl)-()-amino]-essigsäure-ethylester

(7) 1-[3-(4-Ethyloxycarbonylamidino-phenyl)-propionyl]-6-[N-(naphth-1-yl-sulfonyl)-N-ethoxycarbonylmethyl-amino]-1,2,3,4-tetrahydro-chinolin

(8) 1-[3-(4-Octyloxycarbonylamidino-phenyl)-propionyl]-6-(N-phenylsulfonyl-N-ethoxycarbonylmethyl-amino)-1,2,3,4-tetrahydro-chinolin

(9) 1-[3-(4-Methyloxycarbonylamidino-phenyl)-propionyl]-6-(N-phenylsulfonyl-N-ethoxycarbonylmethyl-amino)-1,2,3,4-tetrahydro-chinolin

(10) 1-[3-(4-Ethyloxycarbonylamidino-phenyl)-propionyl]-6-(N benzoyl-N-ethoxycarbonylmethyl-amino)-1,2,3,4-tetrahydro-chi nolin

(11) 1-[3-(4-Octyloxycarbonylamidino-phenyl)-propionyl]-6-(N-ethoxycarbonylmethyl-phenylamino)-1,2,3,4-tetrahydrochinolin

(12) 1-[3-(4-Methyloxycarbonylamidino-phenyl)-propionyl]-6-(N-ethoxycarbonylmethyl-phenylamino)-1,2,3,4-tetrahydrochinolin

Beispiel 33

| Trockenampulle mit 75 mg Wirkstoff pro 10 ml | |
|---|---|
| Zusammensetzung: | |
| Wirkstoff | 75,0 mg |
| Mannitol | 50,0 mg |
| Wasser für Injektionszwecke | ad 10,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 34

| Trockenampulle mit 35 mg Wirkstoff pro 2 ml | |
|---|---|
| Zusammensetzung: | |
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 35

| Tablette mit 50 mg Wirkstoff | |
|---|---|
| Zusammensetzung: | |
| (1) Wirkstoff | 50,0 mg |
| (2) Milchzucker | 98,0 mg |
| (3) Maisstgrke | 50,0 mg |

(fortgesetzt)

| Tablette mit 50 mg Wirkstoff | |
|---|---|
| Zusammensetzung: | |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 36

| Tablette mit 350 mg Wirkstoff | |
|---|---|
| Zusammensetzung: | |
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 37

| Kapseln mit 50 mg Wirkstoff | |
|---|---|
| Zusammensetzung: | |
| (1) Wirkstoff | 50,0 mg |
| (2) Maisstgrke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 38

| Kapseln mit 350 mg Wirkstoff | |
|---|---|
| Zusammensetzung: | |
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

Beispiel 39

| Suppositorien mit 100 mg Wirkstoff | |
|---|---|
| 1 Zäpfchen enthält: | |
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

## Patentansprüche

**1.** Phenylalkylderivate der allgemeinen Formel

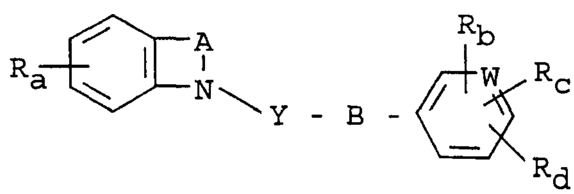

, (I)

in der

$R_a$ ein Wasserstoffatom, eine Carboxy-, $C_{1-3}$-Alkoxycarbonyl-, Benzoyl-, Phenylsulfonyl-, Nitro-, $R_1NR_2$-, $R_1NR_2$-X- oder $(R_3X)NR_1$- Gruppe, in denen

$R_1$ ein Wasserstoffatom, eine $C_{1-5}$-Alkylgruppe, welche durch eine Phenyl-, Carboxy-, $C_{1-4}$-Alkoxycarbonyl- oder Aminocarbonylgruppe substituiert sein kann, wobei die Aminogruppe der Aminocarbonylgruppe zusätzlich durch $C_{1-4}$-Alkyl-, Phenyl-$C_{1-3}$-alkyl-, Phenyl-, Carboxy-$C_{1-3}$-alkyl- oder $C_{1-3}$-Alkoxycarbonyl-$C_{1-3}$-alkylgruppen mono- oder disubstituiert und die Substituenten gleich oder verschieden sein können, oder eine geradkettige $C_{2-3}$-Alkylgruppe, die endständig durch Amino-, $C_{1-3}$-Alkylamino-, Di-($C_{1-3}$-Alkyl)amino-, $C_{1-4}$-Alkanoylamino-, Phenylamino-, N-Benzyloxycarbonylphenylamino-, Pyrrolidino-, Piperidino- oder Morpholinogruppe substituiert ist,

$R_2$ ein Wasserstoffatom, eine gegebenenfalls durch eine oder zwei Phenylgruppen oder durch eine Naphthylgruppe substituierte $C_{1-3}$-Alkylgruppe oder eine Phenylgruppe, die durch ein Fluor-, Chlor- oder Bromatom

oder durch eine geradkettige $C_{2-3}$-Alkylgruppe, die endständig durch eine Amino-, $C_{1-3}$-Alkylamino-, $C_{1-3}$-Alkanoylamino-, Di-($C_{1-3}$-Alkyl)-amino-, Pyrrolidino-, Piperidino- oder Morpholinogruppe substituiert ist, substituiert sein kann,

$R_1$ und $R_2$ zusammen mit dem dazwischen liegenden Stickstoffatom eine gegebenenfalls durch eine $C_{1-3}$-Alkyl-, Carboxyoder $C_{1-3}$-Alkoxycarbonylgruppe substituierte Pyrrolidinooder Piperidinogruppe, eine durch zwei $C_{1-3}$-Alkylgruppen substituierte Pyrrolidino- oder Piperidinogruppe oder eine Morpholinogruppe,

$R_3$ eine geradkettige oder verzweigte $C_{1-7}$-Alkylgruppe, die in 1-, 2- oder 3-Stellung durch eine Phenylgruppe oder in 2-bis 7-Stellung durch ein Fluor-, Chlor- oder Bromatom, durch eine Carboxy- oder $C_{1-3}$-Alkoxycarbonylgruppe substituiert sein kann, eine Trifluormethylgruppe, eine Phenyl-, Naphthyl- oder Chromanylgruppe, die jeweils durch ein Fluor-, Chlor- oder Bromatom, durch eine Trifluormethyl-, $C_{2-3}$-Alkyl-, $C_{1-3}$-Alkoxy-, Amino-, $C_{1-3}$-Alkylamino-, Di-($C_{1-3}$-alkyl)amino- oder Aminocarbonylgruppe substituiert sein können, wobei die vorstehend erwähnten Phenyl-, Naphthyl- oder Chromanylgruppen zusätzlich durch ein bis drei Methylgruppen substituiert sein können, eine durch zwei Chlor- oder Bromatome substituierte Phenyl- oder Aminophenylgruppe, eine gegebenenfalls durch ein Chlor- oder Bromatom oder durch eine Methylgruppe substituierte Thienylgruppe, eine $C_{3-8}$-Cycloalkyl-, $C_{8-12}$-Bicycloalkanon-, Chinolyl-, Isochinolyl- oder Benzimidazolylgruppe oder

$R_1$ und $R_3$ zusammen eine n-Alkylengruppe mit 3 bis 5 Kohlenstoffatomen, in der eine mit der $SO_2$- oder CO-Gruppe verknüpfte Ethylengruppe durch eine 1,2-Phenylengruppe ersetzt sein kann, und

X eine Carbonyl- oder Sulfonylgruppe darstellen,

oder $R_a$ auch eine $C_{2-3}$-Alkanoylgruppe, die im Alkylteil durch eine Carboxy-$C_{1-3}$-alkyl- oder $C_{1-3}$-Alkoxycarbonyl-$C_{1-3}$-alkylgruppe und eine Benzoyl-, Naphthoyl-, Phenylsulfonyl- oder Naphthylsulfonylgruppe substituiert ist,
$R_b$ eine gegebenenfalls durch eine $C_{1-10}$-Alkoxycarbonyl- oder Phenyl-$C_{1-3}$-alkoxycarbonylgruppe substituierte Amidinogruppe, eine Cyano- oder Aminomethylgruppe,
$R_c$ und $R_d$, die gleich oder verschieden sein können, jeweils ein Wasserstoff-, Fluor-, Chlor-, Brom- oder Jodatom, eine Methyl-, Methoxy-, Nitro-, Amino- oder Aminocarbonylgruppe oder eine gegebenenfalls durch eine geradkettige $C_{2-4}$-Alkanoylgruppe substituierte Aminogruppe, in der der Alkanoylteil endständig durch eine Carboxy- oder $C_{1-3}$-Alkoxycarbonylgruppe substituiert sein kann,
A eine gegebenenfalls durch eine oder zwei $C_{1-3}$-Alkylgruppen substituierte Ethylen-, Ethenylen-, n-Propylen- oder n-Butylengruppe, wobei eine Methylengruppe einer gegebenenfalls durch eine oder zwei $C_{1-3}$-Alkylgruppen substituierten Ethylenoder n-Propylengruppe, die

(i) mit dem Stickstofftom verknüpft ist, durch eine Carbonylgruppe, oder

(ii) mit dem Phenylkern verknüpft ist, durch ein Sauerstoffoder Schwefelatom, durch eine Sulfinyl- oder Sulfonylgruppe oder durch eine gegebenenfalls durch eine $C_{1-3}$-Alkylgruppe substituierte Iminogruppe ersetzt sein kann,

B eine Bindung, eine gegebenenfalls durch eine oder zwei $C_{1-3}$-Alkylgruppen substituierte Methylen-, Ethylen-, Ethenylen- oder n-Propylengruppe, wobei

(iii) in den vorstehend erwähnten Methylen-, Ethylen- oder n-Propylengruppen eine Methylengruppe, wenn Y eine Carbonyloder Thiocarbonylgruppe darstellt, durch ein Sauerstoffatom oder durch eine gegebenenfalls durch eine $C_{1-3}$-Alkylgruppe substituierte Iminogruppe oder
(iv) in den vorstehend erwähnten Ethylen- oder n-Propylengruppen, wenn Y eine Methylengruppe darstellt, eine in 3-oder 4-Stellung befindliche Methylengruppe bezogen auf das Stickstoffatom durch ein Sauerstoffatom oder durch eine gegebenenfalls durch eine $C_{1-3}$-Alkylgruppe substituierte Iminogruppe ersetzt sein kann,

W eine Methingruppe oder ein Stickstoffatom und
Y eine Methylen-, Carbonyl- oder Thiocarbonylgruppe bedeuten,
deren Tautomere, deren Stereoisomere, deren Gemische und deren Salze.

**2.** Phenylalkylderivate der allgemeinen Formel I gemäß Anspruch 1, in der
$R_a$, $R_c$, $R_d$, A, B, W und Y wie im Anspruch 1 definiert sind und
$R_b$ eine gegebenenfalls durch eine $C_{1-10}$-Alkoxycarbonyl- oder Phenyl-$C_{1-3}$-alkoxycarbonylgruppe substituierte Amidinogruppe darstellt,
deren optische Antipoden und deren Salze.

**3.** Phenylalkylderivate der allgemeinen Formel I gemäß Anspruch 1, in der
$R_a$ eine $R_1NR_2$-, $R_1'NR_2'$-X- oder $(R_3X)NR_1$-Gruppe, in denen Gruppe, in denen

$R_1$ ein Wasserstoffatom, eine $C_{1-4}$-Alkylgruppe, welche durch eine Phenyl-, Carboxy-, $C_{1-2}$-Alkoxycarbonyl- oder Aminocarbonylgruppe substituiert sein kann, wobei die Aminogruppe der Aminocarbonylgruppe zusätzlich durch $C_{1-4}$-Alkyl-, Phenyl-, Benzyl-, Carboxy-$C_{1-2}$-alkyl- oder $C_{1-2}$-Alkoxycarbonyl-$C_{1-2}$-alkylgruppen mono- oder disubstituiert und die Substituenten gleich oder verschieden sein können, oder eine Ethylgruppe, die endständig durch Amino-, Acetylamino-, Morpholino-, Phenylamino- oder N-Benzyloxycarbonyl-phenylaminogruppe substituiert ist,

$R_2$ ein Wasserstoffatom, eine gegebenenfalls durch eine oder zwei Phenylgruppen oder durch eine Naphthylgruppe substituierte $C_{1-3}$-Alkylgruppe, eine Cyclohexylgruppe, eine gegebenenfalls durch ein Chloratom, durch eine 2-Aminoethyloder 2-Acetylaminogruppe substituierte Phenylgruppe,

$R_1$' und $R_2$' die für $R_1$ und $R_2$ vorstehend erwähnten Bedeutungen besitzen oder zusammen mit dem dazwischen liegenden Stickstoffatom eine gegebenenfalls durch eine Methyl-, Carboxy- oder $C_{1-2}$-Alkoxycarbonylgruppe substituierte Pyrrolidino- oder Piperidinogruppe, eine durch zwei Methylgruppen substituierte Pyrrolidino- oder Piperidinogruppe oder eine Morpholinogruppe,

$R_3$ eine geradkettige oder verzweigte $C_{1-5}$-Alkylgruppe, die in 1-, 2- oder 3-Stellung durch eine Phenyl-, Carboxy- oder $C_{1-3}$-Alkoxycarbonylgruppe oder in 2- oder 3-Stellung durch ein Chloratom substituiert sein kann, eine Trifluormethylgruppe, eine Phenyl- oder Naphthylgruppe, die jeweils durch ein Fluor-, Chlor- oder Bromatom, durch eine Trifluormethyl-, $C_{1-3}$-Alkyl-, $C_{1-3}$-Alkoxy-, Amino-, $C_{1-3}$-Alkylamino-, Di-($C_{1-3}$-alkyl)amino- oder Aminocarbonylgruppe substituiert sein können, wobei die vorstehend erwähnten Phenylgruppen zusätzlich durch ein bis drei Methylgruppen substituiert sein können, eine durch zwei Chlor- oder Bromatome substituierte Phenyl- oder Aminophenylgruppe, eine durch ein Chlor- oder Bromatom substituierte Thienylgruppe, eine $C_{3-7}$-Cycloalkyl-, Chinolyl-, Isochinolyl- oder Benzimidazolylgruppe oder

$R_1$ und $R_3$ zusammen eine n-Alkylengruppe mit 3 bis 5 Kohlenstoffatomen, in der eine mit der $SO_2$ - oder CO-Gruppe verknüpfte Ethylengruppe durch eine 1,2-Phenylengruppe ersetzt sein kann, und

X eine Carbonyl- oder Sulfonylgruppe darstellen,

oder $R_a$ auch eine $C_{2-3}$-Alkanoylgruppe, die durch eine Carboxy-$C_{1-3}$-alkyl- oder $C_{1-3}$-Alkoxycarbonyl-$C_{1-3}$-alkylgruppe und eine Benzoyl-, Naphthoyl-, Phenylsulfonyl- oder Naphthylsulfonylgruppe substituiert ist,

$R_b$ eine gegebenenfalls durch eine $C_{1-10}$-Alkoxycarbonyl- oder Phenyl-$C_{1-3}$-alkoxycarbonylgruppe substituierte Amidinogruppe,

$R_c$ ein Wasserstoff-, Fluor-, Chlor-, Brom- oder Jodatom, eine Methyl-, Methoxy-, Aminocarbonyl-, Amino- oder Nitrogruppe oder eine gegebenenfalls durch eine geradkettige $C_{2-4}$-Alkanoylgruppe substituierte Aminogruppe, in der der Alkanoylteil endständig durch eine Carboxy- oder $C_{1-3}$-Alkoxycarbonylgruppe substituiert sein kann,

$R_d$ ein Wasserstoffatom,

A eine gegebenenfalls durch eine oder zwei Methylgruppen substituierte Ethylen-, n-Propylen- oder n-Butylengruppe, wobei eine Methylengruppe einer gegebenenfalls durch eine oder zwei Methylgruppen substituierten Ethylen- oder n-Propylengruppe, die

(i) mit dem Stickstofftom verknüpft ist, durch eine Carbonylgruppe ersetzt sein kann,

B eine Bindung, eine gegebenenfalls durch eine oder zwei Methylgruppen substituierte Methylen-, Ethylen-, Ethenylen- oder n-Propylengruppe, wobei

(iii) in den vorstehend erwähnten Methylen-, Ethylen- oder n-Propylengruppen eine Methylengruppe, wenn Y eine Carbonyloder Thiocarbonylgruppe darstellt, durch ein Sauerstoffatom oder durch eine gegebenenfalls durch eine Methylgruppe substituierte Iminogruppe oder

(iv) in den vorstehend erwähnten Ethylen- oder n-Propylengruppen, wenn Y eine Methylengruppe darstellt, eine in 3-oder 4-Stellung befindliche Methylengruppe bezogen auf das Stickstoffatom durch ein Sauerstoffatom oder durch eine gegebenenfalls durch eine Methylgruppe substituierte Iminogruppe ersetzt sein kann,

W eine Methingruppe und
Y eine Methylen-, Carbonyl- oder Thiocarbonylgruppe bedeuten,
deren optische Antipoden und Salze.

**4.** Phenylalkylderivate der allgemeinen Formel I gemäß Anspruch 1, in der
$R_a$ eine $(R_3SO_2)NR_1$-Gruppe darstellt,
deren optische Antipoden und deren Salze.

**5.** Phenylalkylderivate der allgemeinen Formel I gemäß Anspruch 1, in der

$R_a$ eine $(R_3SO_2)NR_1$-Gruppe, wobei $R_1$ und $R_3$ wie im Anspruch 4 definiert sind,
$R_b$ eine gegebenenfalls durch eine $C_{1-10}$-Alkoxycarbonyl- oder Phenyl-$C_{1-3}$-alkoxycarbonylgruppe substituierte Amidinogruppe,
$R_c$ und $R_d$ jeweils ein Wasserstoffatom,
A eine gegebenenfalls durch eine Methylgruppe substituierte n-Propylengruppe,
B eine Ethylengruppe,
W eine Methingruppe und
Y eine Carbonylgruppe bedeuten,
deren optische Antipoden und deren Salze.

**6.** Folgende Phenylalkylderivate der allgemeinen Formel I gemäß Anspruch 1:

(a) 1-[3-(4-Amidino-phenyl)propionyl]-6-(4-fluor-phenylsulfonamido)-1,2,3,4-tetrahydro-chinolin,

(b) 1- [3- (4-Amidino-phenyl) propionyl) -6-butylsulfonamido-1,2,3,4-tetrahydro-chinolin,

(c) 1-[3-(4-Amidino-phenyl)propionyl)-5-phenylsulfonamido-1,2,3,4-tetrahydro-chinolin,

(d) 1-[3-(4-Amidino-phenyl)propionyl]-3-methyl-6-phenylsulfonamido-1,2,3,4-tetrahydro-chinolin,

(e) 1-[3-(4-Amidino-phenyl)propionyl]-6-(5-chlor-thien-2-ylsulfonamido)-1,2,3,4-tetrahydro-chinolin,

(f) 1-[3-(4-Amidino-phenyl)propionyl)-6-phenylsulfonamido-1,2,3,4-tetrahydro-chinolin,

(g) 1-[3-(4-Amidino-phenyl)propionyl]-6-(N-methyl-phenylsulfonamido)-1,2,3,4-tetrahydro-chinolin,

(h) 1-[3-(4-Amidino-phenyl)propionyl]-6-(N-ethoxycarbonylmethyl-phenylsulfonamido)-1,2,3,4-tetrahydro-chinolin,

(i) 1-[3-(4-Amidino-phenyl)propionyl]-6-(N-carboxymethyl-phenylsulfonamido)-1,2,3,4-tetrahydro-chinolin,

(j) 1-[3-(4-Aminomethyl-phenyl)propionyl]-6-phenylsulfonamido-1,2,3,4-tetrahydro-chinolin,

(k) 1- [3- (4-Amidino-phenyl)propyl] -6-phenylsulfonamido-1,2,3,4-tetrahydro-chinolin,

(l) 1-[3-(4-Methyloxycarbonyl-amidino-phenyl)propionyl]-6-phenylsulfonamido-1,2,3,4-tetrahydro-chinolin,

(m) 1-[3-(4-Amidino-phenyl)-propionyl]-6-(N-phenyl-methylaminocarbony)-1,2,3,4-tetrahydro-chinolin,

(n) 1-[(4-Amidino-phenoxy)-acetyl]-6-[N-(1-naphthylsulfonyl)-hydroxycarbonylmethylamino]-1,2,3,4-tetrahydro-chinolin,

(o) 1-[3-(4-Amidino-phenyl)-propionyl]-6-diethylaminocarbonyl-1,2,3,4-tetrahydro-chinolin,

(p) 1-[3-(4-Amidino-phenyl)-propionyl]-6-(N-benzoyl-methylamino)-1,2,3,4-tetrahydro-chinolin,

(q) 1- [3- (4-Amidino-phenyl) -propionyl] -6- (N-benzoyl-methylamino)-1,2,3,4-tetrahydro-chinolin,

(r) 1-[3-(4-Amidino-phenyl)-propionyl]-6-[N-(naphth-1-yl-sulfonyl)-hydroxycarbonylmethylamino]-1,2,3,4-tetrahydro-chinolin,

(s) 1-[3-(4-Amidino-phenyl)-propionyl]-6-[N-(1-naphthoyl)-hydroxycarbonylmethylamino]-1,2,3,4-tetrahydro-chinolin,

(t) 1-(3-(4-Amidino-phenyl)-propionyl]-6-(N-benzoyl-hydroxycarbonylmethylamino]-1,2,3,4-tetrahydro-chinolin,

(u) 1-[3-(4-Amidino-phenyl)-propionyl]-6-[N-(chinolin-8-sulfonyl)-hydroxycarbonylmethylaminol-1,2;3,4-tetrahydro-chinolin und

(v) 1-[3-(4-Amidino-phenyl)-propionyl]-6-[N-(n-butylsulfonyl)-hydroxycarbonylmethylamino]-1,2,3,4-tetrahydro-chinolin,

deren optische Antipoden und deren Salze.

**7.** Physiologisch verträgliche Salze der Verbindungen gemäß den Ansprüchen 1 bis 6.

**8.** Arzneimittel, enthaltend eine Verbindung nach mindestens einem der Ansprüche 1 bis 6, in denen $R_b$ mit Ausnahme der Cyanogruppe wie in den Ansprüchen 1 bis 6 definiert ist, oder ein Salz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 6, in denen $R_b$ mit Ausnahme der Cyanogruppe wie in den Ansprüchen 1 bis 6 definiert ist, oder ein Salz gemäß Anspruch 7 zur Herstellung eines Arzneimittels mit einer die Thrombinzeit verlängernder Wirkung, einer thrombinhemmender Wirkung und einer Hemmwirkung auf verwandte Serinproteasen.

**10.** Verfahren zur Herstellung eines Arzneimittels gemäß Anspruch 8, **dadurch gekennzeichnet, daß** auf nichtchemischem Wege eine Verbindung nach mindestens einem der Ansprüche 1 bis 6, in denen $R_b$ mit Ausnahme der Cyanogruppe wie in den Ansprüchen 1 bis 6 definiert ist, oder ein Salz gemäß Anspruch 7 in einen oder mehrere inerte Trägerstoffe und/oder Verdünnungsmittel eingearbeitet wird.

**11.** Verfahren zur Herstellung der Verbindungen gemäß den Ansprüchen 1 bis 7, **dadurch gekennzeichnet, daß**

a) zur Herstellung einer Verbindung der allgemeinen Formel I, in der $R_b$ eine Cyanogruppe und Y eine Methylengruppe darstellen, eine Verbindung der allgemeinen Formel

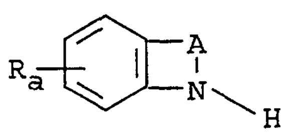
, (II)

in der
A und $R_a$ wie in den Ansprüchen 1 bis 6 definiert sind, mit einer Verbindung der allgemeinen Formel

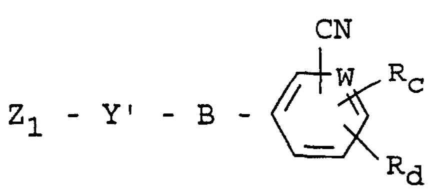
, (III)

in der
B, W, $R_c$ und $R_d$ wie in den Ansprüchen 1 bis 6 definiert sind,
Y' eine Methylengruppe und
$Z_1$ eine Austrittsgruppe darstellen, umgesetzt wird oder

b) zur Herstellung einer Verbindung der allgemeinen Formel I, in der $R_b$ eine Cyanogruppe und Y eine Carbonylgruppe darstellen, eine Verbindung der allgemeinen Formel

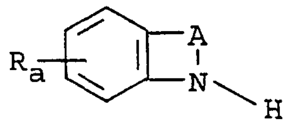

, (II)

in der
A und $R_a$ wie in den Ansprüchen 1 bis 6 definiert sind, mit einer Verbindung der allgemeinen Formel

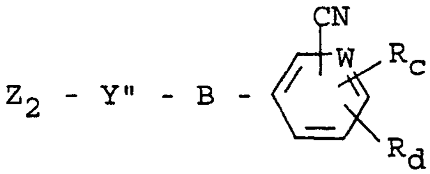

, (IV)

in der
B, W, $R_c$ und $R_d$ wie in den Ansprüchen 1 bis 6 definiert sind,
Y" eine Carbonylgruppe und
$Z_2$ eine Hydroxygruppe oder eine Austrittsgruppe darstellen, umgesetzt wird oder

c) zur Herstellung einer Verbindung der allgemeinen Formel I, in der $R_a$ eine $R_1N(XR_3)$-Gruppe und $R_b$ eine Cyanogruppe darstellen, eine Verbindung der allgemeinen Formel

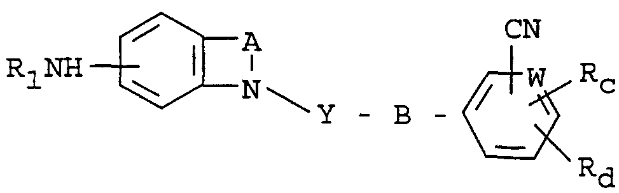

, (V)

in der
A, B, W, Y, $R_c$, $R_d$ und $R_1$ wie in den Ansprüchen 1 bis 6 definiert sind, mit einer Verbindung der allgemeinen Formel

$$Z_3\text{-}X\text{-}R_3 \quad ,(VI)$$

in der
X und $R_3$ wie in den Ansprüchen 1 bis 6 definiert sind und
$Z_3$ eine Hydroxygruppe oder eine Austrittsgruppe darstellt, umgesetzt wird oder

d) zur Herstellung einer Verbindung der allgemeinen Formel I, in der $R_a$ eine $R_1NR_2$- oder $R_1N(XR_3)$-Gruppe, in der $R_1$ mit Ausnahme des Wasserstoffatoms wie in den Ansprüchen 1 bis 6 definiert ist, und $R_b$ eine Cyanogruppe oder eine durch eine $C_{1-10}$-Alkoxycarbonylgruppe oder Phenyl-$C_{1-3}$-alkoxycarbonylgruppe substituierte Amidinogruppe darstellen, eine Verbindung der allgemeinen Formel

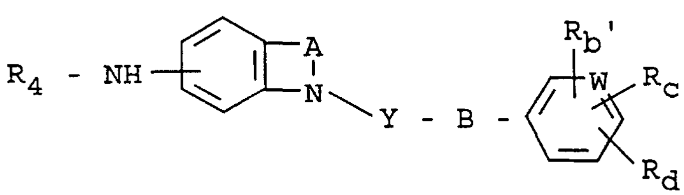

, (VII)

in der
A, B, W, Y, $R_c$ und $R_d$ wie in den Ansprüchen 1 bis 6 definiert sind,
$R_4$ die für $R_2$ in den Ansprüchen 1 bis 6 erwähnten Bedeutungen aufweist oder eine $R_3$-X-Gruppe darstellt, wobei $R_3$ und X wie in den Ansprüchen 1 bis 6 erwähnt definiert sind, und
$R_b$' eine Cyanogruppe oder eine durch eine $C_{1-10}$-Alkoxycarbonylgruppe oder Phenyl-$C_{1-3}$-alkoxycarbonylgruppe substituierte Amidinogruppe darstellt, mit einer Verbindung der allgemeinen Formel

$$Z_4\text{-}R_1' \qquad ,(VIII),$$

in der
$R_1$' eine $C_{1-5}$-Alkylgruppe, welche durch eine Phenyl-, Carboxy-, $C_{1-4}$-Alkoxycarbonyl- oder Aminocarbonylgruppe substituiert sein kann, wobei die Aminogruppe der Aminocarbonylgruppe zusätzlich durch $C_{1-4}$-Alkyl-, Phenyl-$C_{1-3}$-alkyl-, Phenyl-, Carboxy-$C_{1-3}$-alkyl- oder $C_{1-3}$-Alkoxycarbonyl-$C_{1-3}$-alkylgruppen mono- oder disubstituiert und die Substituenten gleich oder verschieden sein können, oder eine geradkettige $C_{2-3}$-Alkylgruppe, die endständig durch eine Di-($C_{1-3}$-Alkyl)amino-, Pyrrolidino-, Piperidino- oder Morpholinogruppe substituiert ist, und
$Z_4$ eine Austrittsgruppe darstellen, umgesetzt wird oder

e) zur Herstellung einer Verbindung der allgemeinen Formel I, in der $R_a$ eine Nitrogruppe und $R_b$ eine Cyanogruppe darstellen, eine Verbindung der allgemeinen Formel

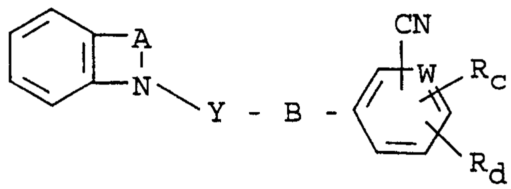

, (IX)

in der
A, B, W, Y, $R_c$ und $R_d$ wie in den Ansprüchen 1 bis 6 definiert sind, nitriert wird oder

f) zur Herstellung einer Verbindung der allgemeinen Formel I, in der $R_a$ eine Aminogruppe und $R_b$ eine Cyanogruppe darstellen, eine Verbindung der allgemeinen Formel

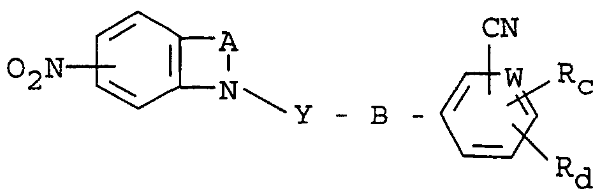

, (X)

in der
A, B, W, Y, $R_c$ und $R_d$ wie in den Ansprüchen 1 bis 6 definiert sind, reduziert wird oder

g) zur Herstellung einer Verbindung der allgemeinen Formel I, in der $R_b$ eine Amidinogruppe darstellt, eine gegebenenfalls im Reaktionsgemisch gebildete Verbindung der allgemeinen Formel

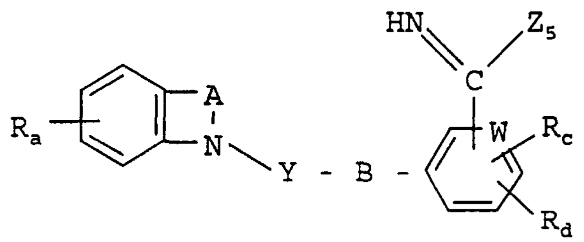

, (XI)

in der
A, B, W, Y, $R_a$, $R_c$ und $R_d$ wie in den Ansprüchen 1 bis 6 definiert sind und
$Z_5$ eine Alkoxy-, Aralkoxy-, Alkylthio- oder Aralkylthiogruppe darstellt, mit Ammoniak oder mit dessen Säureadditionssalzen umgesetzt wird oder

h) zur Herstellung einer Verbindung der allgemeinen Formel I, in der $R_b$ eine durch eine $C_{1-10}$-Alkoxycarbonylgruppe oder Phenyl-$C_{1-3}$-alkoxycarbonylgruppe substituierte Amidinogruppe darstellt, eine Verbindung der allgemeinen Formel

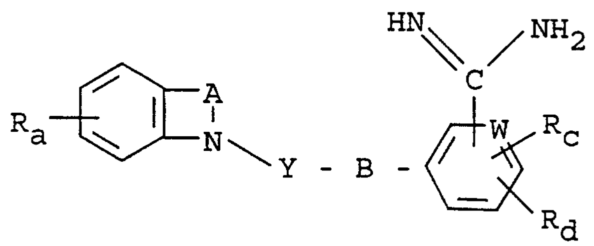

, (XII)

in der
A, B, W, Y, $R_a$, $R_c$ und $R_d$ wie in den Ansprüchen 1 bis 6 definiert sind, mit einer Verbindung der allgemeinen Formel

$$Z_6\text{-CO-OR}_4 \qquad ,\text{(XIII)}$$

in der
$R_4$ eine $C_{1-10}$-Alkyl- oder Phenyl-$C_{1-3}$-alkylgruppe und
$Z_6$ eine Austrittsgruppe darstellen, umgesetzt wird oder

i) zur Herstellung einer Verbindung der allgemeinen Formel I, in der $R_b$ eine Aminomethylgruppe darstellt, eine Verbindung der allgemeinen Formel

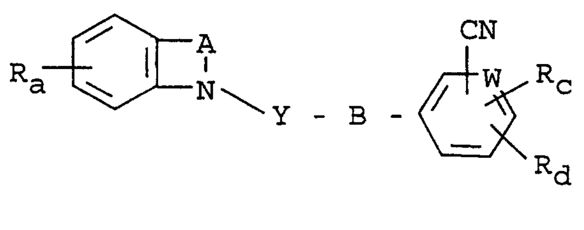

, (XIV)

in der
A, B, W, Y, $R_a$, $R_c$ und $R_d$ wie in den Ansprüchen 1 bis 6 definiert sind, reduziert wird oder

j) zur Herstellung einer Verbindung der allgemeinen Formel I, in der $R_a$ eine $R_1NR_2$-Gruppe, in der $R_1$ mit Ausnahme des Wasserstoffatoms wie in den Ansprüchen 1 bis 6 definiert ist, und $R_b$ eine Cyanogruppe oder

eine durch eine $C_{1-10}$-Alkoxycarbonylgruppe oder Phenyl-$C_{1-3}$-alkoxycarbonylgruppe substituierte Amidinogruppe darstellen, eine Verbindung der allgemeinen Formel

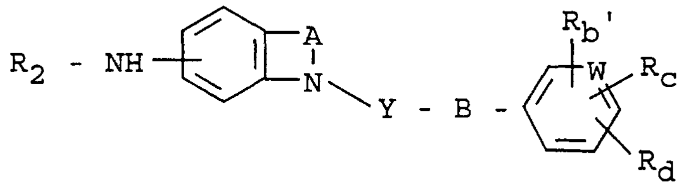

, (XV)

in der
A, B, W, Y, $R_2$, $R_c$ und $R_d$ wie in den Ansprüchen 1 bis 6 definiert sind und
$R_b$' eine Cyanogruppe oder eine durch eine $C_{1-10}$-Alkoxycarbonylgruppe oder Phenyl-$C_{1-3}$-alkoxycarbonylgruppe substituierte Amidinogruppe darstellt, mit einer Verbindung der allgemeinen Formel

$$Z_7\text{-}R_1' \quad ,\text{(XVI)},$$

in der
$R_1$' wie vorstehend erwähnt definiert ist und
$Z_7$ eine Austrittsgruppe oder zusammen mit einem Wasserstoffatom des benachbarten Kohlenstoffatoms ein Sauerstoffatom darstellen, umgestzt wird oder

k) zur Herstellung einer Verbindung der allgemeinen Formel I, in der $R_b$ eine Cyanogruppe und Y eine Carbonylgruppe darstellen, eine Verbindung der allgemeinen Formel

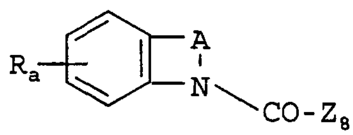

, (XVII)

in der
A und $R_a$ wie in den Ansprüchen 1 bis 6 definiert sind und
$Z_8$ eine Austrittsgruppe darstellt, mit einer Verbindung der allgemeinen Formel

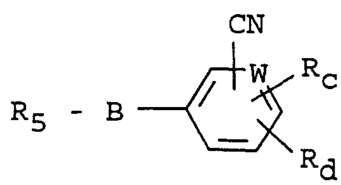

, (XVIII)

in der
B, W, $R_c$ und $R_d$ wie in den Ansprüchen 1 bis 6 definiert sind und
$R_5$ eine gegenbenenfalls durch eine $C_{1-3}$-Alkylgruppe substituierte Aminogruppe darstellt, umgesetzt wird und

erforderlichenfalls ein während den Umsetzungen zum Schutze von reaktiven Gruppen verwendeter Schutzrest abgespalten wird und/oder
gewünschtenfalls anschließend eine so erhaltene Verbindung der allgemeinen Formel I, in der X eine Carbonylgruppe darstellt, mittels eines schwefeleinführenden Mittels in eine entsprechende Thiocarbonylverbindung übergeführt wird und/oder
eine so erhaltene Verbindung der allgemeinen Formel I, in der $R_a$ einen Acylrest enthält, mittels Hydrolyse in eine Verbindung der allgemeinen Formel I, in der $R_a$ eine $R_1$NH-Gruppe darstellt oder in der $R_a$ eine Carboxygruppe

enthält, übergeführt wird und/oder

eine so erhaltene Verbindung der allgemeinen Formel I, in der $R_a$ eine Carboxy- oder Sulfonsäuregruppe darstellt oder enthält, mittels Amidierung in eine entsprechende Amidverbindung der allgemeinen Formel I übergeführt wird und/oder

gewünschtenfalls anschließend eine so erhaltene Verbindung der allgemeinen Formel I in ihre Stereoisomere aufgetrennt wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

**1.** Phenylalkyl derivatives of general formula

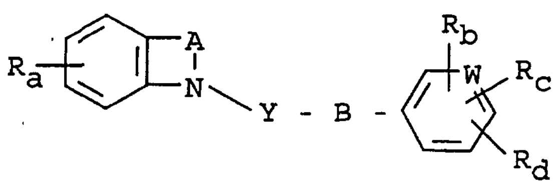 , (I)

wherein

$R_a$ denotes a hydrogen atom, a carboxy, $C_{1-3}$-alkoxycarbonyl, benzoyl, phenylsulphonyl, nitro, $R_1NR_2^-$; $R_1NR_2$-X- or $(R_3X)NR_1$-group wherein

$R_1$ denotes a hydrogen atom, a $C_{1-5}$-alkyl group, which may be substituted by a phenyl, carboxy, $C_{1-4}$-alkoxycarbonyl or aminocarbonyl group, whilst the amino group of the aminocarbonyl group may additionally be mono- or disubstituted by $C_{1-4}$-alkyl, phenyl-$C_{1-3}$-alkyl, phenyl, carboxy-$C_{1-3}$-alkyl- or $C_{1-3}$-alkoxycarbonyl-$C_{1-3}$-alkyl groups and the substituents may be identical or different, or a straight-chained $C_{2-3}$-alkyl group, which is terminally substituted by amino, $C_{1-3}$-alkylamino, di-($C_{1-3}$-alkyl)amino, $C_{1-4}$-alkanoylamino, phenylamino, N-benzyloxycarbonyl-phenylamino, pyrrolidino, piperidino or morpholino group,

$R_2$ denotes a hydrogen atom, a $C_{1-3}$-alkyl group optionally substituted by one or two phenyl groups or by a naphthyl group or a phenyl group which may be substituted by a fluorine, chlorine or bromine atom or by a straight-chained $C_{2-3}$-alkyl group, which is terminally substituted by an amino, $C_{1-3}$-alkylamino, $C_{1-3}$-alkanoylamino, di-($C_{1-3}$-alkyl)amino, pyrrolidino, piperidino or morpholino group,

$R_1$ and $R_2$ together with the nitrogen atom between them denote a pyrrolidino or piperidino group optionally substituted by a $C_{1-3}$-alkyl, carboxy or $C_{1-3}$-alkoxycarbonyl group, a pyrrolidino or piperidino group substituted by two $C_{1-3}$-alkyl groups or a morpholino group,

$R_3$ denotes a straight-chained or branched $C_{1-7}$-alkyl group, which may be substituted in the 1, 2 or 3 position by a phenyl group or in the 2 to 7 position by a fluorine, chlorine or bromine atom, by a carboxy or $C_{1-3}$-alkoxycarbonyl group, a trifluoromethyl group, a phenyl, naphthyl or chromanyl group which may be substituted in each case by a fluorine, chlorine or bromine atom, by a trifluoromethyl; $C_{1-3}$-alkyl, $C_{1-3}$-alkoxy, amino, $C_{1-3}$-alkylamino, di-($C_{1-3}$-alkyl)amino or aminocarbonyl group, whilst the abovementioned phenyl, naphthyl or chromanyl groups may additionally be substituted by one to three methyl groups, a phenyl or aminophenyl group substituted by two chlorine or bromine atoms, a thienyl group optionally substituted by a chlorine or bromine atom or by a methyl group, a $C_{3-8}$-cycloalkyl, $C_{8-12}$-bicycloalkanone, quinolyl, isoquinolyl or benzimidazolyl group or

$R_1$ and $R_3$ together denote an n-alkylene group with 3 to 5 carbon atoms, wherein an ethylene group linked to the $SO_2$- or CO- group may be replaced by a 1,2-phenylene group, and

X denotes a carbonyl or sulphonyl group,

or $R_a$ may also denote a $C_{2-3}$-alkanoyl group, which is substituted in the alkyl moiety by a carboxy-$C_{1-3}$-alkyl or $C_{1-3}$-alkoxycarbonyl-$C_{1-3}$-alkyl group and a benzoyl, naphthoyl, phenylsulphonyl or naphthylsulphonyl group,

$R_b$ denotes an amidino group optionally substituted by a $C_{1-10}$-alkoxycarbonyl or phenyl-$C_{1-3}$-alkoxycarbonyl group, a cyano or aminomethyl group,

$R_c$ and $R_d$, which may be identical or different, each denote a hydrogen, fluorine, chlorine, bromine or iodine atom, a methyl, methoxy, nitro, amino or aminocarbonyl group or an amino group optionally substituted by a straight-chained $C_{2-4}$-alkanoyl group, wherein the alkanoyl moiety may be terminally substituted by a carboxy or $C_{1-3}$-alkoxycarbonyl group,

A denotes an ethylene, ethenylene, n-propylene or n-butylene group optionally substituted by one or two $C_{1-3}$-alkyl groups, whilst a methylene group of an ethylene or n-propylene group optionally substituted by one or two $C_{1-3}$-alkyl groups, which is

(i) linked to the nitrogen atom, may be replaced by a carbonyl group, or

(ii) linked to the phenyl nucleus, may be replaced by an oxygen or sulphur atom, by a sulphinyl or sulphonyl group or by an imino group optionally substituted by a $C_{1-3}$-alkyl group,

B denotes a bond, a methylene, ethylene, ethenylene or n-propylene group optionally substituted by one or two $C_{1-3}$-alkyl groups, whilst

(iii) in the abovementioned methylene, ethylene or n-propylene groups, if Y denotes a carbonyl or thiocarbonyl group, a methylene group may be replaced by an oxygen atom or by an imino group optionally substituted by a $C_{1-3}$-alkyl group, or

(iv) in the abovementioned ethylene or n-propylene groups, if Y denotes a methylene group, a methylene group in the 3 or 4 position relative to the nitrogen atom may be replaced by an oxygen atom or by an imino group optionally substituted by a $C_{1-3}$-alkyl group,

W denotes a methine group or a nitrogen atom and
Y denotes a methylene, carbonyl or thiocarbonyl group,
the tautomers, the stereoisomers and mixtures thereof and the salts thereof.

**2.** Phenylalkyl derivatives of general formula I according to claim 1, wherein
$R_a$, $R_c$, $R_d$, A, B, W and Y are defined as in claim 1 and
$R_b$ denotes an amidino group optionally substituted by a $C_{1-10}$-alkoxycarbonyl or phenyl-$C_{1-3}$-alkoxycarbonyl group,
the optical antipodes and the salts thereof.

**3.** Phenylalkyl derivatives of general formula I according to claim 1, wherein
$R_a$ denotes an $R_1NR_2$, $R_1'NR_2'$-X or $(R_3X)NR_1$-group wherein

$R_1$ denotes a hydrogen atom, a $C_{1-4}$-alkyl group, which may be substituted by a phenyl, carboxy, $C_{1-2}$-alkoxycarbonyl or aminocarbonyl group, whilst the amino group of the aminocarbonyl group may additionally be mono or disubstituted by $C_{1-4}$-alkyl, phenyl, benzyl, carboxy-$C_{1-2}$-alkyl or $C_{1-2}$-alkoxycarbonyl-$C_{1-2}$-alkyl groups and the substituents may be identical or different, or an ethyl group, which is terminally substituted by an amino, acetylamino, morpholino, phenylamino or N-benzyloxycarbonyl-phenylamino group,

$R_2$ denotes a hydrogen atom, a $C_{1-3}$-alkyl group optionally substituted by one or two phenyl groups or by a naphthyl group, a cyclohexyl group, or a phenyl group optionally substituted by a chlorine atom, by a 2-aminoethyl or 2-acetylamino group,

$R_1'$ and $R_2'$ have the meanings given hereinbefore for $R_1$ and $R_2$ or together with the nitrogen atom between them denote a pyrrolidino or piperidino group optionally substituted by a methyl, carboxy or $C_{1-2}$-alkoxycarbonyl group, a pyrrolidino or piperidino group substituted by two methyl groups, or a morpholino group,

$R_3$ denotes a straight-chained or branched $C_{1-5}$-alkyl group, which may be substituted in the 1, 2 or 3 position by a phenyl, carboxy or $C_{1-3}$-alkoxycarbonyl group or in the 2 or 3 position by a chlorine atom, a trifluoromethyl group, a phenyl or naphthyl group, which may be substituted in each case by a fluorine, chlorine or bromine atom, by a trifluoromethyl, $C_{1-3}$-alkyl, $C_{1-3}$-alkoxy, amino, $C_{1-3}$-alkylamino, di($C_{1-3}$-alkyl)amino or aminocarbonyl group, whilst the abovementioned phenyl groups may additionally be substituted by one to three methyl groups, a phenyl or aminophenyl group substituted by two chlorine or bromine atoms, a thienyl group substituted by a chlorine or bromine atom, a $C_{3-7}$-cycloalkyl, quinolyl, isoquinolyl or benzimidazolyl group or

$R_1$ and $R_3$ together denote an n-alkylene group with 3 to 5 carbon atoms, wherein an ethylene group linked to the $SO_2$ or CO- group may be replaced by a 1,2-phenylene group, and

X denotes a carbonyl or sulphonyl group,

or $R_a$ also denotes a $C_{2-3}$-alkanoyl group which is substituted by a carboxy-$C_{1-3}$-alkyl or $C_{1-3}$-alkoxycarbonyl-$C_{1-3}$-alkyl group and a benzoyl, naphthoyl, phenylsulphonyl or naphthylsulphonyl group,
$R_b$ denotes an amidino group optionally substituted by a $C_{1-10}$-alkoxycarbonyl or phenyl-$C_{1-3}$-alkoxycarbonyl group,
$R_c$ denotes a hydrogen, fluorine, chlorine, bromine or iodine atom, a methyl, methoxy, aminocarbonyl, amino or

nitro group or an amino group optionally substituted by a straight-chained $C_{2-4}$-alkanoyl group, wherein the alkanoyl moiety may be terminally substituted by a carboxy or $C_{1-3}$-alkoxycarbonyl group,
$R_d$ denotes a hydrogen atom,
A denotes an ethylene, n-propylene or n-butylene group optionally substituted by one or two methyl groups, whilst a methylene group of an ethylene or n-propylene group optionally substituted by one or two methyl groups, which is

(i) linked to the nitrogen atom, may be replaced by a carbonyl group,

B denotes a bond, a methylene, ethylene, ethenylene or n-propylene group optionally substituted by one or two methyl groups, whilst

(iii) in the abovementioned methylene, ethylene or n-propylene groups, if Y denotes a carbonyl or thiocarbonyl group, a methylene group may be replaced by an oxygen atom or by an imino group optionally substituted by a methyl group, or

(iv) in the abovementioned ethylene or n-propylene groups, if Y denotes a methylene group, a methylene group in the 3 or 4 position relative to the nitrogen atom may be replaced by an oxygen atom or by an imino group optionally substituted by a methyl group,

W denotes a methine group and
Y denotes a methylene, carbonyl or thiocarbonyl group,
the optical antipodes and salts thereof.

**4.** Phenylalkyl derivatives of general formula I according to claim 1, wherein
$R_a$ denotes a $(R_3SO_2)NR_1$-group,
the optical antipodes and the salts thereof.

**5.** Phenylalkyl derivatives of general formula I according to claim 1, wherein
$R_a$ denotes a $(R_3SO_2)NR_1$-group, whilst $R_1$ and $R_3$ are defined as in claim 4,
$R_b$ denotes an amidino group optionally substituted by a $C_{1-10}$-alkoxycarbonyl or phenyl-$C_{1-3}$-alkoxycarbonyl group,
$R_c$ and $R_d$ each denote a hydrogen atom,
A denotes an n-propylene group optionally substituted by a methyl group,
B denotes an ethylene group,
W denotes a methine group and
Y denotes a carbonyl group,
the optical antipodes and the salts thereof.

**6.** The following phenylalkyl derivatives of general formula I according to claim 1:

(a) 1-[3-(4-amidino-phenyl)propionyl]-6-(4-fluoro-phenylsulphonamido)-1,2,3,4-tetrahydro-quinoline,

(b) 1-[3-(4-amidino-phenyl)propionyl]-6-butylsulphonamido-1,2,3,4-tetrahydro-quinoline,

(c) 1-[3-(4-amidino-phenyl)propionyl]-5-phenylsulphonamido-1,2,3,4-tetrahydro-quinoline,

(d) 1-[3-(4-amidino-phenyl)propionyl]-3-methyl-6-phenylsulphonamido-1,2,3,4-tetrahydro-quinoline,

(e) 1-[3-(4-amidino-phenyl)propionyl]-6-(5-chloro-thien-2-yl-sulphonamido)-1,2,3,4-tetrahydro-quinoline,

(f) 1-[3-(4-amidino-phenyl)propionyl]-6-phenylsulphonamido-1,2,3,4-tetrahydro-quinoline,

(g) 1-[3-(4-amidino-phenyl)propionyl]-6-(N-methyl-phenylsulphonamido)-1,2,3,4-tetrahydro-quinoline,

(h) 1-[3-(4-amidino-phenyl)propionyl]-6-(N-ethoxycarbonylmethyl-phenylsulphonamido)-1,2,3,4-tetrahydro-quinoline,

(i) 1-[3-(4-amidino-phenyl)propionyl]-6-(N-carboxymethylphenylsulphonamido)-1,2,3,4-tetrahydro-quinoline,

(j) 1-[3-(4-aminomethyl-phenyl)propionyl]-6-phenylsulphonamido-1,2,3,4-tetrahydro-quinoline,

(k) 1-[3-(4-amidino-phenyl)propyl]-6-phenylsulphonamido-1,2,3,4-tetrahydro-quinoline,

(l) 1-[3-(4-methyloxycarbonyl-amidino-phenyl)propionyl]-6-phenylsulphonamido-1,2,3,4-tetrahydro-quinoline,

(m) 1-[3-(4-amidino-phenyl)-propionyl]-6-(N-phenyl-methylaminocarbony)-1,2,3,4-tetrahydro-quinoline,

(n) 1- [ (4-amidino-phenoxy) -acetyl] -6- [N- (1-naphthylsulphonyl)hydroxycarbonylmethylamino]-1,2,3,4-tetrahydro-quinoline,

(o) 1-[3-(4-amidino-phenyl)-propionyl]-6-diethylaminocarbonyl-1,2,3,4-tetrahydro-quinoline,

(p) 1-[3-(4-amidino-phenyl)-propionyl]-6-(N-benzoyl-methylamino)-1,2,3,4-tetrahydro-quinoline,

(q) 1-[3-(4-amidino-phenyl)-propionyl)-6-(N-benzoyl-methylamino)-1,2,3,4-tetrahydro-quinoline,

(r) 1-[3-(4-amidino-phenyl)-propionyl]-6-[N-(naphth-1-yl-sulphonyl)-hydroxycarbonylmethylamino)-1,2,3,4-tetrahydro-quinoline,

(s) 1-[3-(4-amidino-phenyl)-propionyl]-6-[N-(1-naphthoyl)-hydroxycarbonylmethylamino]-1,2,3,4-tetrahydro-quinoline,

(t) 1-[3-(4-amidino-phenyl)-propionyl]-6-(N-benzoyl-hydroxycarbonylmethylamino]-1,2,3,4-tetrahydro-quinoline,

(u) 1- [3- (4-amidino-phenyl) -propionyl] -6- [N- (quinoline-8-sulphonyl)-hydroxycarbonylmethylamino]-1,2,3,4-tetrahydroquinoline and

(v) 1-[3-(4-amidino-phenyl)-propionyl]-6-[N-(n-butylsulphonyl)-hydroxycarbonylmethylamino]-1,2,3,4-tetrahydro-quinoline,

the optical antipodes and the salts thereof.

**7.** Physiologically acceptable salts of the compounds according to claims 1 to 6.

**8.** Pharmaceutical compositions containing a compound according to at least one of claims 1 to 6, wherein $R_b$ is as defined in claims 1 to 6 with the exception of the cyano group, or a salt according to claim 7 optionally together with one or more inert carriers and/or diluents.

**9.** Use of a compound according to at least one of claims 1 to 6, wherein $R_b$ is as defined in claims 1 to 6 with the exception of the cyano group, or a salt according to claim 7 for preparing a pharmaceutical composition having the effect of prolonging the thrombin time, having a thrombin-inhibiting effect and an inhibitory effect on related serine proteases.

**10.** Process for preparing a pharmaceutical composition according to claim 8, **characterised in that** a compound according to at least one of claims 1 to 6, wherein $R_b$ is as defined in claims 1 to 6 with the exception of the cyano group, or a salt according to claim 7 is incorporated in one or more inert carriers and/or diluents by a non-chemical method.

**11.** Process for preparing the compounds according to claims 1 to 7, **characterised in that**

a) in order to prepare a compound of general formula I wherein $R_b$ denotes a cyano group and Y denotes a methylene group, a compound of general formula

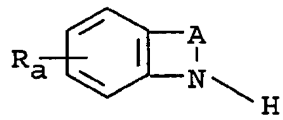 , (II)

wherein
A and $R_a$ are as defined in claims 1 to 6, is reacted with a compound of general formula

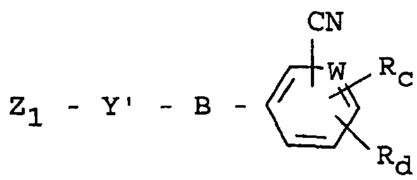 , (III)

wherein
B, W, $R_c$ and $R_d$ are as defined in claims 1 to 6,
Y' denotes a methylene group and
$Z_1$ denotes a leaving group, or

b) in order to prepare a compound of general formula I wherein $R_b$ denotes a cyano group and Y denotes a carbonyl group, a compound of general formula

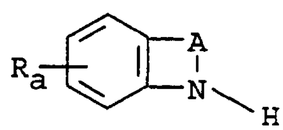 , (II)

wherein
A and $R_a$ are as defined in claims 1 to 6, is reacted with a compound of general formula

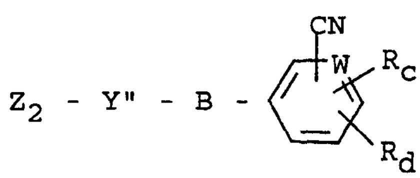 , (IV)

wherein
B, W, $R_c$ and $R_d$ are as defined in claims 1 to 6,
Y" denotes a carbonyl group and
$Z_2$ denotes a hydroxy group or a leaving group, or

c) in order to prepare a compound of general formula I wherein $R_a$ denotes an $R_1N(XR_3)$-group and $R_b$ denotes a cyano group, a compound of general formula

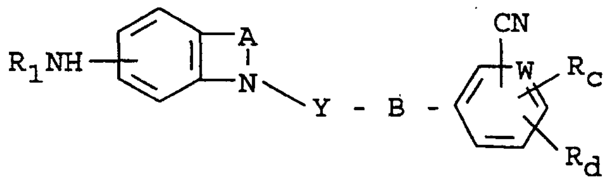

, (V)

wherein
A, B, W, Y, $R_c$, $R_d$ and $R_1$ are as defined in claims 1 to 6, is reacted with a compound of general formula

$$Z_3\text{-}X\text{-}R_3 \quad ,(VI)$$

wherein
X and $R_3$ are as defined in claims 1 to 6 and
$Z_3$ denotes a hydroxy group or a leaving group, or

d) in order to prepare a compound of general formula I wherein $R_a$ denotes an $R_1NR_2$ or $R_1N(XR_3)$-group, wherein $R_1$ is defined as in claims 1 to 6 with the exception of the hydrogen atom, and $R_b$ denotes a cyano group or an amidino group substituted by a $C_{1-10}$-alkoxycarbonyl group or phenyl-$C_{1-3}$-alkoxycarbonyl group, a compound of general formula

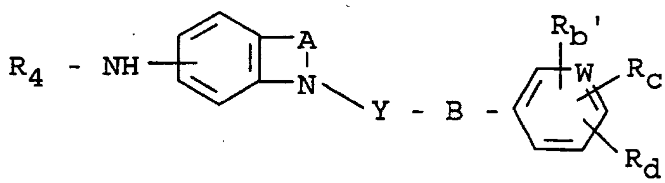

, (VII)

wherein
A, B, W, Y, $R_c$ and $R_d$ are as defined in claims 1 to 6,
$R_4$ has the.meanings given for $R_2$ in claims 1 to 6 or denotes an $R_3$-X- group, whilst $R_3$ and X are defined as in claims 1 to 6, and
$R_b$' denotes a cyano group or an amidino group substituted by a $C_{1-10}$-alkoxycarbonyl group or phenyl-$C_{1-3}$-alkoxycarbonyl group, is reacted with a compound of general formula

$$Z_4\text{-}R_1' \quad ,(VIII),$$

wherein
$R_1$' denotes a $C_{1-5}$-alkyl group, which may be substituted by a phenyl, carboxy, $C_{1-4}$-alkoxycarbonyl or aminocarbonyl group, whilst the amino group of the aminocarbonyl group is additionally mono- or disubstituted by $C_{1-4}$-alkyl, phenyl-$C_{1-3}$-alkyl, phenyl, carboxy-$C_{1-3}$-alkyl or $C_{1-3}$-alkoxycarbonyl-$C_{1-3}$-alkyl groups and the substituents may be identical or different, or a straight-chained $C_{2-3}$-alkyl group, which is terminally substituted by a di-($C_{1-3}$-alkyl)amino, pyrrolidino, piperidino or morpholino group, and
$Z_4$ denotes a leaving group, or

e) in order to prepare a compound of general formula I wherein $R_a$ denotes a nitro group and $R_b$ denotes a cyano group, a compound of general formula

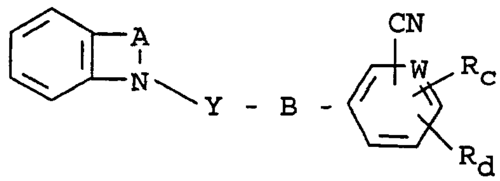

, (IX)

wherein
A, B, W, Y, $R_c$ and $R_d$ are as defined in claims 1 to 6, is nitrated or

f) in order to prepare a compound of general formula I wherein $R_a$ denotes an amino group and $R_b$ denotes a cyano group, a compound of general formula

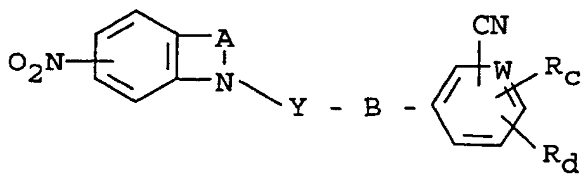

, (X)

wherein
A, B, W, Y, $R_c$ and $R_d$ are as defined in claims 1 to 6, is reduced or

g) in order to prepare a compound of general formula I,
wherein $R_b$ denotes an amidino group, a compound of general formula

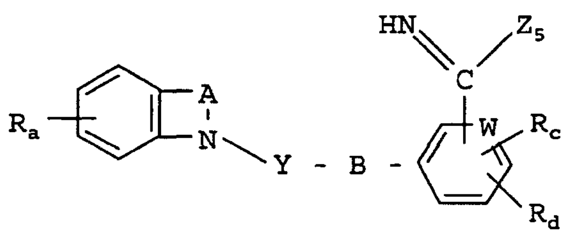

, (XI)

optionally formed in the reaction mixture,
wherein
A, B, W, Y, $R_a$, $R_c$ and $R_d$ are as defined in claims 1 to 6 and $Z_5$ denotes a alkoxy, aralkoxy, alkylthio or aralkylthio group, is reacted with ammonia or with the acid addition salts thereof or

h) in order to prepare a compound of general formula I wherein $R_b$ denotes an amidino group substituted by a $C_{1-10}$-alkoxycarbonyl group or phenyl-$C_{1-3}$-alkoxycarbonyl group, a compound of general formula

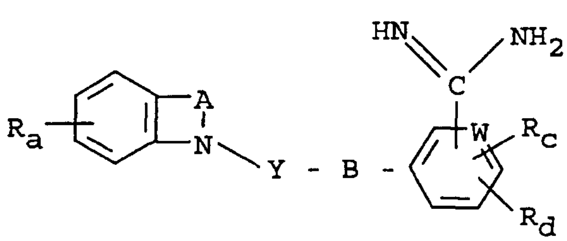

, (XII)

wherein
A, B, W, Y, $R_a$, $R_c$ and $R_d$ are as defined in claims 1 to 6, is reacted with a compound of general formula

$$Z_6\text{-CO-OR}_4 \quad ,\text{(XIII)}$$

wherein
$R_4$ denotes a $C_{1-10}$-alkyl or phenyl-$C_{1-3}$-alkyl group and
$Z_6$ denotes a leaving group, or

i) in order to prepare a compound of general formula I wherein $R_b$ denotes an aminomethyl group, a compound of general formula

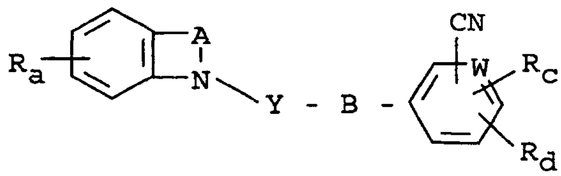
, (XIV)

wherein
A, B, W, Y, $R_a$, $R_c$ and $R_d$ are as defined in claims 1 to 6, is reduced or

j) in order to prepare a compound of general formula I wherein $R_a$ denotes an $R_1NR_2$-group, wherein $R_1$ is defined as in claims 1 to 6 with the exception of the hydrogen atom, and $R_b$ denotes a cyano group or an amidino group substituted by a $C_{1-10}$-alkoxycarbonyl group or phenyl-$C_{1-3}$-alkoxycarbonyl group, a compound of general formula

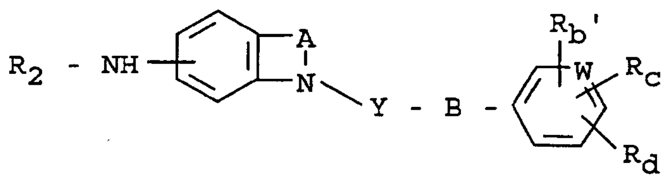
, (XV)

wherein
A, B, W, Y, $R_2$, $R_c$ and $R_d$ are as defined in claims 1 to 6 and $R_{b'}$ denotes a cyano group or an amidino group substituted by a $C_{1-10}$-alkoxycarbonyl group or phenyl-$C_{1-3}$-alkoxycarbonyl group, is reacted with a compound of general formula

$$Z_7\text{-}R_1' \quad ,\text{(XVI)},$$

wherein
$R_1$' is as hereinbefore defined and
$Z_7$ denotes a leaving group or together with a hydrogen atom of the adjacent carbon atom denotes an oxygen atom, or

k) in order to prepare a compound of general formula I wherein $R_b$ denotes a cyano group and Y denotes a carbonyl group, a compound of general formula

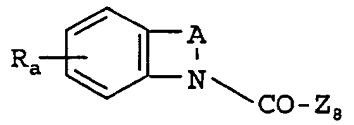

, (XVII)

wherein
A and $R_a$ are as defined in claims 1 to 6 and
$Z_8$ denotes a leaving group, is reacted with a compound of general formula

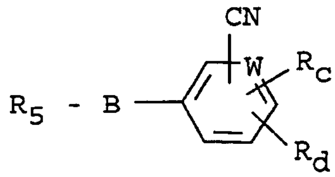

, (XVIII)

wherein
B, W, $R_c$ and $R_d$ are as defined in claims 1 to 6 and
$R_5$ denotes an amino group optionally substituted by a $C_{1-3}$-alkyl group, and

if necessary any protecting group used during the reactions to protect reactive groups is cleaved and/or subsequently, if desired, a compound of.general formula I thus obtained wherein X denotes a carbonyl group may be converted by means of a sulphurising agent into a corresponding thiocarbonyl compound, and/or
a compound of general formula I thus obtained wherein $R_a$ contains an acyl group may be converted by hydrolysis into a compound of general formula I wherein $R_a$ denotes an $R_1NH$-group or wherein $R_a$ contains a carboxy group, and/or
a compound of general formula I thus obtained wherein $R_a$ denotes or contains a carboxy or sulphonic acid group may be converted by amidation into a corresponding amide compound of general formula I, and/or
if desired a compound of general formula I thus obtained is resolved into its stereoisomers and/or
a compound of general formula I thus obtained is converted into the salts thereof, particularly for pharmaceutical use into the physiologically acceptable salts thereof with an inorganic or organic acid or base.

**Revendications**

1. Dérivés phénylalkylés de formule générale

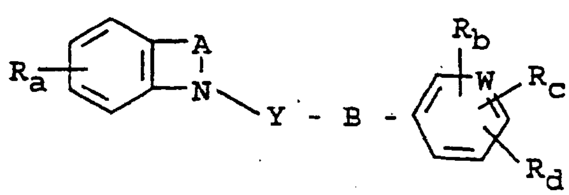

, (I)

où
$R_a$ représente un atome d'hydrogène, un groupe carboxyle, alcoxy en $C_{1-3}$-carbonyle, benzoyle, phénylsulfonyle, nitro, $R_1NR_2$, $R_1NR_2$-X ou $(R_3X)$ $NR_1$, dans lesquels
$R_1$ représente un atome d'hydrogène, un groupe alkyle en $C_{1-5}$, qui peut être substitué par un groupe phényle, carboxyle, alcoxy en $C_{1-4}$-carbonyle ou aminocarbonyle, où le groupe amino du groupe aminocarbonyle peut être mono- ou disubstitué en outre par des groupes alkyle en $C_{1-4}$, phényl-alkyle en $C_{1-3}$, phényle, carboxy-alkyle en $C_{1-3}$ ou alcoxy en $C_{1-3}$-carbonyl-alkyle en $C_{1-3}$ et les substituants peuvent être identiques ou différents, ou bien un groupe alkyle en $C_{2-3}$ linéaire qui est substitué en position terminale par un groupe amino, alkylamino en $C_{1-3}$, di-(alkyle en $C_{1-3}$) amino, alcanoylamino en $C_{1-4}$, phénylamino, N-benzyloxycarbonylphénylamino, pyrrolidino, pipéridino ou morpholino,

$R_2$ représente un atome d'hydrogène, un groupe alkyle en $C_{1-3}$ éventuellement substitué par un ou deux groupes phényle ou par un groupe naphtyle, ou un groupe phényle qui peut être substitué par un atome de fluor, de chlore ou de brome ou par un groupe alkyle en $C_{2-3}$ linéaire qui est substitué en position terminale par un groupe amino, alkylamino en $C_{1-3}$, alcanoylamino en $C_{1-3}$, di-(alkyle en $C_{1-3}$)amino, pyrrolidino, pipéridino ou morpholino,
$R_1$ et $R_2$ représentent avec l'atome d'azote situé entre eux un groupe pyrrolidino ou pipéridino éventuellement substitué par un groupe alkyle en $C_{1-3}$, carboxyle ou alcoxy en $C_{1-3}$-carbonyle, un groupe pyrrolidino ou pipéridino substitué par deux groupes alkyle en $C_{1-3}$ ou un groupe morpholino,
$R_3$ représente un groupe alkyle en $C_{1-7}$ linéaire ou ramifié qui peut être substitué en position 1, 2 ou 3 par un groupe phényle ou en position 2 à 7 par un atome de fluor, de chlore ou de brome, par un groupe carboxyle ou alcoxy en $C_{1-3}$-carbonyle, un groupe trifluorométhyle, un groupe phényle, naphtyle ou chromanyle qui peuvent être substitués dans chaque cas par un atome de fluor, de chlore ou de brome, par un groupe trifluorométhyle, alkyle en $C_{1-3}$, alcoxy en $C_{1-3}$, amino, alkylamino en $C_{1-3}$, di-(alkyle en $C_{1-3}$) amino ou aminocarbonyle, où les groupes phényle, naphtyle ou chromanyle cités précédemment peuvent être substitués en outre par un à trois groupes méthyle, un groupe phényle ou aminophényle substitué par deux atomes de chlore ou de brome, un groupe thiényle éventuellement substitué par un atome de chlore ou de brome ou par un groupe méthyle, un groupe cycloalkyle en $C_{3-8}$, bicycloalcanone en $C_{8-12}$, quinolyle, isoquinolyle ou benzimidazolyle ou
$R_1$ et $R_3$ représentent ensemble un groupe n-alkylène ayant 3 à 5 atomes de carbone dans lequel un groupe éthylène lié au groupe $SO_2$ ou CO peut être remplacé par un groupe 1,2-phénylène, et
X représente un groupe carbonyle ou sulfonyle,
ou $R_a$ représente aussi un groupe alcanoyle en $C_{2-3}$ qui est substitué dans la partie alkyle par un groupe carboxy-alkyle en $C_{1-3}$ ou alcoxy en $C_{1-3}$-carbonyl-alkyle en $C_{1-3}$ et un groupe benzoyle, naphtoyle, phénylsulfonyle ou naphtylsulfonyle,
$R_b$ représente un groupe amidino éventuellement substitué par un groupe alcoxy en $C_{1-10}$-carbonyle ou phényl-alcoxy en $C_{1-3}$-carbonyle; un groupe cyano ou aminométhyle,
$R_c$ et $R_d$, qui peuvent être identiques ou différents, représentent chacun un atome d'hydrogène, de fluor, de chlore, de brome ou d'iode, un groupe méthyle, méthoxy, nitro, amino ou aminocarbonyle ou un groupe amino éventuellement substitué par un groupe alcanoyle en $C_{2-4}$ linéaire dans lequel la partie alcanoyle peut être substituée en position terminale par un groupe carboxyle ou alcoxy en $C_{1-3}$-carbonyle,
A représente un groupe éthylène, éthénylène, n-propylène ou n-butylène éventuellement substitué par un ou deux groupes alkyle en $C_{1-3}$, où un groupe méthylène d'un groupe éthylène ou n-propylène éventuellement substitué par un ou deux groupes alkyle en $C_{1-3}$, qui

(i) est lié à l'atome d'azote peut être remplacé par un groupe carbonyle, ou qui
(ii) est lié au noyau phényle peut être remplacé par un atome d'oxygène ou de soufre, par un groupe sulfinyle ou sulfonyle ou par un groupe imino éventuellement substitué par un groupe alkyle en $C_{1-3}$,

B représente une liaison, un groupe méthylène, éthylène, éthénylène ou n-propylène éventuellement substitué par un ou deux groupes alkyle en $C_{1-3}$, où

(iii) dans les groupes méthylène, éthylène ou n-propylène cités précédemment, un groupe méthylène, quand Y représente un groupe carbonyle ou thiocarbonyle, peut être remplacé par un atome d'oxygène ou par un groupe imino éventuellement substitué par un groupe alkyle en $C_{1-3}$, ou
(iv),dans les groupes éthylène ou n-propylène cités précédemment, quand Y représente un groupe méthylène, un groupe méthylène situé en position 3 ou 4 par rapport à l'atome d'azote peut être remplacé par un atome d'oxygène ou par un groupe imino éventuellement substitué par un groupe alkyle en $C_{1-3}$,

W représente un groupe méthyne ou un atome d'azote et
Y représente un groupe méthylène, carbonyle ou thiocarbonyle,
leurs tautomères, leurs stéréoisomères, leurs mélanges et leurs sels.

**2.** Dérivés phénylalkylés de formule générale I selon la revendication 1 où
$R_a$, $R_c$, $R_d$, A, B, W et Y sont définis comme dans la revendication 1 et
$R_b$ représente un groupe amidino éventuellement substitué par un groupe alcoxy en $C_{1-10}$-carbonyle ou phényl-alcoxy en $C_{1-3}$-carbonyle,
leurs antipodes optiques et leurs sels.

**3.** Dérivés phénylalkylés de formule générale I selon la revendication 1 où
$R_a$ représente un groupe $R_1NR_2$, $R_1'NR_2'$-X ou ($R_3$X) $NR_1$, groupes dans lesquels

$R_1$ représente un atome d'hydrogène, un groupe alkyle en $C_{1-4}$, qui peut être substitué par un groupe phényle, carboxyle, alcoxy en $C_{1-2}$-carbonyle ou aminocarbonyle, où le groupe amino du groupe aminocarbonyle peut être mono- ou disubstitué en outre par des groupes alkyle en $C_{1-4}$, phényle, benzyle, carboxy-alkyle en $C_{1-2}$ ou alcoxy en $C_{1-2}$-carbonyl-alkyle en $C_{1-2}$ et les substituants peuvent être identiques ou différents, ou bien un groupe éthyle qui est substitué en position terminale par un groupe amino, acétylamino, morpholino, phénylamino ou N-benzyloxycarbonylphénylamino,
$R_2$ représente un atome d'hydrogène, un groupe alkyle en $C_{1-3}$ éventuellement substitué par un ou deux groupes phényle ou par un groupe naphtyle, un groupe cyclohexyle, un groupe phényle éventuellement substitué par un atome de chlore, par un groupe 2-aminoéthyle ou 2-acétylamino,
$R_1$' et $R_2$' possèdent les significations indiqués précédemment pour $R_1$ et $R_2$ ou représentent avec l'atome d'azote situé entre eux un groupe pyrrolidino ou pipéridino éventuellement substitué par un groupe méthyle, carboxyle ou alcoxy en $C_{1-2}$-carbonyle, un groupe pyrrolidino ou pipéridino substitué par deux groupes méthyle ou un groupe morpholino,
$R_3$ représente un groupe alkyle en $C_{1-5}$ linéaire ou ramifié qui peut être substitué en position 1, 2 ou 3 par un groupe phényle, carboxyle ou alcoxy en $C_{1-3}$-carbonyle ou en position 2 ou 3 par un atome de chlore, un groupe trifluorométhyle, un groupe phényle ou naphtyle qui peuvent être substitués dans chaque cas par un atome de fluor, de chlore ou de brome, par un groupe trifluorométhyle, alkyle en $C_{1-3}$, alcoxy en $C_{1-3,}$ amino, alkylamino en $C_{1-3,}$ di- (alkyle en $C_{1-3}$) amino ou aminocarbonyle, où les groupes phényle cités précédemment peuvent être substitués en outre par un à trois groupes méthyle, un groupe phényle ou aminophényle substitué par deux atomes de chlore ou de brome, un groupe thiényle substitué par un atome de chlore ou de brome, un groupe cycloalkyle en $C_{3-7}$, quinolyle, isoquinolyle ou benzimidazolyle ou
$R_1$ et $R_3$ représentent ensemble un groupe n-alkylène ayant 3 à 5 atomes de carbone dans lequel un groupe éthylène lié au groupe $SO_2$ ou CO peut être remplacé par un groupe 1,2-phénylène, et
X représente un groupe carbonyle ou sulfonyle,
ou $R_a$ représente aussi un groupe alcanoyle en $C_{2-3}$ qui est substitué par un groupe carboxy-alkyle en $C_{1-3}$ ou alcoxy en $C_{1-3}$-carbonyl-alkyle en $C_{1-3}$ et un groupe benzoyle, naphtoyle, phénylsulfonyle ou naphtylsulfonyle,
$R_b$ représente un groupe amidino éventuellement substitué par un groupe alcoxy en $C_{1-10}$-carbonyle ou phénylalcoxy en $C_{1-3}$-carbonyle,
$R_c$ représente un atome d'hydrogène, de fluor, de chlore, de brome ou d'iode, un groupe méthyle, méthoxy, aminocarbonyle, amino ou nitro ou un groupe amino éventuellement substitué par un groupe alcanoyle en $C_{2-4}$ linéaire dans lequel la partie alcanoyle peut être substituée en position terminale par un groupe carboxyle ou alcoxy en $C_{1-3}$-carbonyle,
$R_d$ représente un atome d'hydrogène,
A représente un groupe éthylène, n-propylène ou n-butylène éventuellement substitué par un ou deux groupes méthyle où un groupe méthylène d'un groupe éthylène ou n-propylène éventuellement substitué par un ou deux groupes méthyle, qui

(i) est lié à l'atome d'azote peut être remplacé par un groupe carbonyle,

B représente une liaison, un groupe méthylène, éthylène, éthénylène ou n-propylène éventuellement substitué par un ou deux groupes méthyle, où

(iii) dans les groupes méthylène, éthylène ou n-propylène cités précédemment, un groupe méthylène, quand Y représente un groupe carbonyle ou thiocarbonyle, peut être remplacé par un atome d'oxygène ou par un groupe imino éventuellement substitué par un groupe méthyle, ou
(iv) dans les groupes éthylène ou n-propylène cités précédemment, quand Y représente un groupe méthylène, un groupe méthylène situé en position 3 ou 4 par rapport à l'atome d'azote peut être remplacé par un atome d'oxygène ou par un groupe imino éventuellement substitué par un groupe méthyle,

W représente un groupe méthyne et
Y représente un groupe méthylène, carbonyle ou thiocarbonyle,
leurs antipodes optiques et leurs sels.

**4.** Dérivés phénylalkylés de formule générale I selon la revendication 1 où
$R_a$ représente un groupe $(R_3SO_2)NR_1$,
leurs antipodes optiques et leurs sels.

**5.** Dérivés phénylalkylés de formule générale I selon la revendication 1 où

$R_a$ représente un groupe $(R_3SO_2)$ $NR_1$, où $R_1$ et $R_3$ sont définis comme dans la revendication 4,
$R_b$ représente un groupe amidino éventuellement substitué par un groupe alcoxy en $C_{1-10}$-carbonyle ou phénylalcoxy en $C_{1-3}$-carbonyle,
$R_c$ et $R_d$ représentent chacun un atome d'hydrogène,
A représente un groupe n-propylène éventuellement substitué par un groupe méthyle,
B représente un groupe éthylène,
W représente un groupe méthyne et
Y représente un groupe carbonyle,
leurs antipodes optiques et leurs sels.

**6.** Dérivés phénylalkylés de formule générale I selon la revendication 1 suivants :

(a) 1-[3-(4-amidino-phényl)propionyl]-6-(4-fluorophénylsulfonamido)-1,2,3,4-tétrahydro-quinoléine,

(b) 1-[3-(4-amidino-phényl)propionyl]-6-butylsulfonamido-1,2,3,4-tétrahydro-quinoléiné,

(c) 1-[3-(4-amidino-phényl)propionyl)-5-phénylsulfonamido-1,2,3,4-tétrahydro-quinoléine,

(d) 1-[3-(4-amidino-phényl)propionyl]-3-méthyl-6-phénylsulfonamido-1,2,3,4-tétrahydro-quinoléine,

(e) 1-[3-(4-amidinb-phényl)propionyl]-6-(5-chlorothién-2-ylsulfonamido)-1,2,3,4-tétrahydro-quinoléine,

(f) 1-[3-(4-amidino-phényl)propionyl]-6-phénylsulfonamido-1,2,3,4-tétrahydro-quinoléine,

(g) 1-[3-(4-amidino-phényl)propionyl]-6-(N-méthylphénylsulfonamido)-1,2,3,4-tétrahydro-quinoléine,

(h) 1-[3-(4-amidino-phényl)propionyl]-6-(N-éthoxycarbonylméthyl-phénylsulfonamido)-1,2,3,4-tétrahydroquinoléine,

(i) 1-[3-(4-amidino-phényl)propionyl]-6-(N-carboxyméthyl-phénylsulfonamido)-1,2,3,4-tétrahydroquinoléine,

(j) 1-[3-(4-aminométhyl-phényl)propionyl]-6-phénylsulfonamido-1,2,3,4-tétrahydro-quinoléine,

(k) 1-[3-(4-amidino-phényl)propyl]-6-phénylsulfonamido-1,2,3,4-tétrahydro-quinoléine,

(l) 1-[3-(4-méthyloxycarbonyl-amidino-phényl)-propionyl]-6-phénylsulfonamido-1,2,3,4-tétrahydroquinoléine,

(m) 1-[3-(4-amidino-phényl)-propionyl]-6-(N-phénylméthylaminocarbon)-1,2,3,4-tétrahydro-quinoléine,

(n) 1-[(4-amidino-phénoxy)-acétyl]-6-[N-(1-naphtylsulfonyl)hydroxycarbonylméthylamino]-1,2,3,4-tétrahydroquinoléine,

(o) 1-[3-(4-amidino-phényl)-propionyl]-6-diéthylaminocarbonyl-1,2,3,4-tétrahydro-quinoléine,

(p) 1-[3-(4-amidino-phényl)-propionyl]-6-(N-benzoylméthylamino)-1,2,3,4-tétrahydro-quinoléine,

(q) 1-[3-(4-amidino-phényl)-propionyl]-6-(N-benzoylméthylamino)-1,2,3,4-tétrahydro-quinoléine,

(r) 1-[3-(4-amidinophényl)-propionyl]-6-(N-(napht-1-yl-sulfonyl)-hydroxycarbonylméthylamino]-1,2,3,4-tétrahydro-quinoléine,

(s) 1-[3-(4-amidino-phényl)-propionyl]-6-[N-(1-naphtoyl)hydroxycarbonylméthylamino]-1,2,3,4-tétrahydroquinoléine,

(t) 1-[3-(4-amidino-phényl)-propionyl]-6-(N-benzoylhydroxycarbonylméthylamino]-1,2,3,4-tétrahydroquinoléine,

(u) 1-[3-(4-amidino-phényl)-propionyl]-6-[N-(quinoléine-8-sulfonyl)-hydroxycarbonylméthylamino]-1,2,3,4-tétrahydro-quinoléine et

(v) 1-[3-(4-amidino-phényl)-propionyl]-6-[N-(n-butylsulfonyl)hydroxycarbonylméthylamino]-1,2,3,4-tétrahydro-quinoléine.

**7.** Sels physiologiquement acceptables des composés selon les revendications 1 à 6.

**8.** Médicament contenant un composé selon au moins l'une des revendications 1 à 6 où $R_b$ est défini comme dans les revendications 1 à 6, à l'exception du groupe cyano, ou un sel selon la revendication 7 outre éventuellement un ou plusieurs supports et/ou diluants inertes.

**9.** Utilisation d'un composé selon au moins l'une des revendications 1 à 6 où $R_b$ est défini comme dans les revendications 1 à 6, à l'exception du groupe cyano, ou d'un sel selon la revendication 7 pour la production d'un médicament ayant un effet prolongeant le temps.de thrombine, un effet inhibant la thrombine et un effet inhibiteur sur les protéases à sérine apparentées.

**10.** Procédé de production d'un médicament selon la revendication 8 **caractérisé en ce que**, par voie non chimique, un composé selon au moins l'une des revendications 1 à 6 où $R_b$ est défini comme dans les revendications 1 à 6, à l'exception du groupe cyano, ou un sel selon la revendication 7 est incorporé dans un ou plusieurs supports et/ou diluants inertes.

**11.** Procédé de production des composés selon les revendications 1 à 7 **caractérisé en ce que**

a) pour la production d'un composé de formule générale I où $R_b$ représente un groupe cyano et Y représente un groupe méthylène, un composé de formule générale

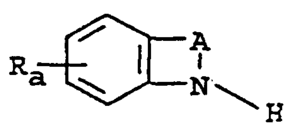

, (II)

où
A et $R_a$ sont définis comme dans les revendications 1 à 6 est mis à réagir avec un composé de formule générale

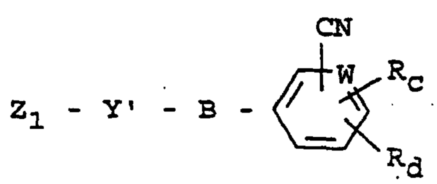

, (III)

où
B, W, $R_c$ et $R_d$ sont définis comme dans les revendications 1 à 6,
Y' représente un groupe méthylène et
$Z_1$ représente un groupe partant, ou
b) pour la production d'un composé de formule générale I où $R_b$ représente un groupe cyano et Y représente un groupe carbonyle, un composé de formule générale

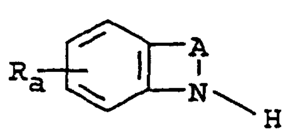

, (II)

où
A et $R_a$ sont définis comme dans les revendications 1 à 6, est mis à réagir avec un composé de formule générale

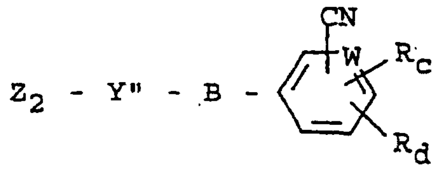

, (IV)

où
B, W, $R_c$ et $R_d$ sont définis comme dans les revendications 1 à 6,
Y" représente un groupe carbonyle et
$Z_2$ représente un groupe hydroxyle ou un groupe partant, ou
c) pour la production d'un composé de formule générale I où $R_a$ représente un groupe $R_1N$ $(XR_3)$ et $R_b$ représente un groupe cyano, un composé de formule générale

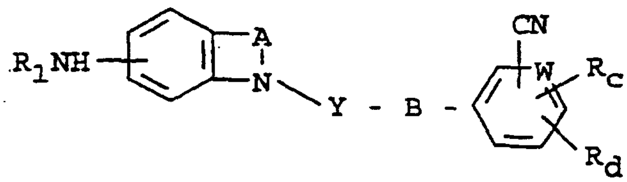

, (V)

où
A, B, W, Y, $R_c$, $R_d$ et $R_1$ sont définis comme dans les revendications 1 à 6, est mis à réagir avec un composé de formule générale

$$Z_3\text{-}X\text{-}R_3 \qquad (VI)$$

où
X et $R_3$ sont définis comme dans les revendications 1 à 6 et $Z_3$ représente un groupe hydroxyle ou un groupe partant, ou
d) pour la production d'un composé de formule générale I où $R_a$ représente un groupe $R_1NR_2$ ou $R_1N$ $(XR_3)$ où $R_1$ est défini comme dans les revendications 1 à 6, à l'exception de l'atome d'hydrogène, et $R_b$ représente un groupe cyano ou un groupe amidino substitué par un groupe alcoxy en $C_{1-10}$-carbonyle ou un groupe phényl-alcoxy en $C_{1-3}$-carbonyle, un composé de formule générale

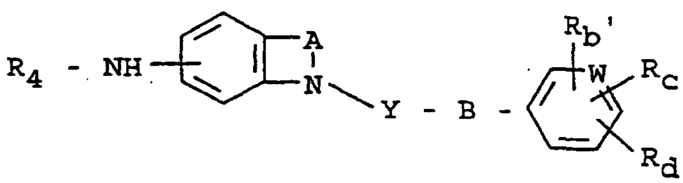

, (VII)

où
A, B, W, Y, $R_c$ et $R_d$ sont définis comme dans les revendications 1 à 6,
$R_4$ présente les significations citées pour $R_2$ dans les revendications 1 à 6 ou représente un groupe $R_3$-X, où $R_3$ et X sont définis comme indiqué dans les revendications 1 à 6, et
$R_b$' représente un groupe cyano ou un groupe amidino substitué par un groupe alcoxy en $C_{1-10}$-carbonyle ou un groupe phényl-alcoxy en $C_{1-3}$-carbonyle, est mis à réagir avec un composé de formule générale

$Z_4$-$R_1$' (VIII)

où
$R_1$' représente un groupe alkyle en $C_{1-5}$, qui peut être substitué par un groupe phényle, carboxyle, alcoxy en $C_{1-4}$-carbonyle ou aminocarbonyle, où le groupe amino du groupe aminocarbonyle peut en outre être mono- ou disubstitué par un groupe alkyle en $C_{1-4}$, phényl-alkyle en $C_{1-3}$, phényle, carboxy-alkyle en $C_{1-3}$ ou alcoxy en $C_{1-3}$-carbonyl-alkyle en $C_{1-3}$, et les substituants peuvent être identiques ou différents, ou un groupe alkyle en $C_{2-3}$ linéaire, qui est substitué en position terminale par un groupe di-(alkyle en $C_{1-3}$)amino, pyrrolidino, pipéridino ou morpholino, et
$Z_4$ représente un groupe partant, ou
e) pour la production d'un composé de formule générale I où $R_a$ représente un groupe nitro et $R_b$ représente un groupe cyano, un composé de formule générale

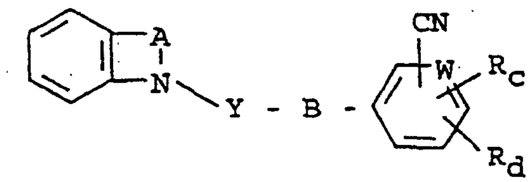
, (IX)

où
A, B, W, Y, $R_c$ et $R_d$ sont définis comme dans les revendications 1 à 6, est nitré ou
f) pour la production d'un composé de formule générale I où $R_a$ représente un groupe amino et $R_b$ représente un groupe cyano, un composé de formule générale

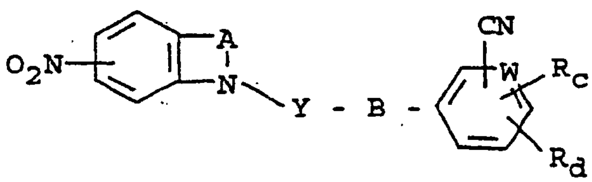
, (X)

où
A, B, W, Y, $R_c$ et $R_d$ sont définis comme dans les revendications 1 à 6, est réduit ou
g) pour la production d'un composé de formule générale I où $R_b$ représente un groupe amidino, un composé éventuellement formé dans le mélange réactionnel, de formule générale

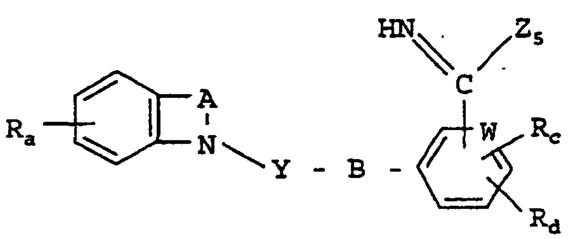
, (XI)

où
A, B, W, Y, $R_a$, $R_c$ et $R_d$ sont définis comme dans les revendications 1 à 6 et
$Z_5$ représente un groupe alcoxy, aralcoxy, alkylthio ou aralkylthio est mis à réagir avec l'ammoniac ou avec ses sels d'addition d'acide ou
h) pour la production d'un composé de formule générale I où $R_b$ représente un groupe amidino substitué par

un groupe alcoxy en $C_{1-10}$-carbonyle ou phényl-alcoxy en $C_{1-3}$-carbonyle, un composé de formule générale

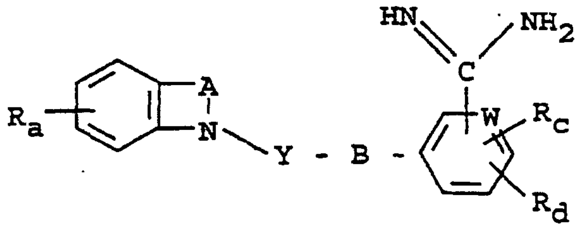

, (XII)

où
A, B, W, Y, $R_a$, $R_c$ et $R_d$ sont définis comme dans les revendications 1 à 6, est mis à réagir avec un composé de formule générale

$$Z_6\text{-CO-OR}_4 \qquad (XIII)$$

où $R_4$ représente un groupe alkyle en $C_{1-10}$ ou phényl-alkyle en $C_{1-3}$ et
$Z_6$ représente un groupe partant, ou
i) pour la production d'un composé de formule générale I où $R_b$ représente un groupe aminométhyle, un composé de formule générale

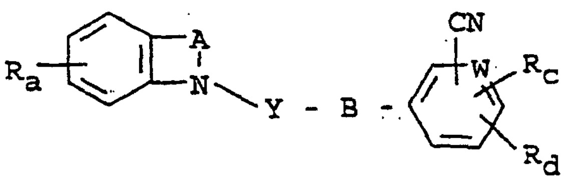

, (XIV)

où
A, B, W, Y, $R_a$, $R_c$ et $R_d$ sont définis comme dans les revendications 1 à 6, est réduit, ou
j) pour la production d'un composé de formule générale I où $R_a$ est un groupe $R_1NR_2$ où $R_1$ est défini comme dans les revendications 1 à 6 à l'exception de l'atome d'hydrogène, et $R_b$ représente un groupe cyano ou un groupe amidino substitué par un groupe alcoxy en $C_{1-10}$-carbonyle ou phényl-alcoxy en $C_{1-3}$-carbonyle, un composé de formule générale

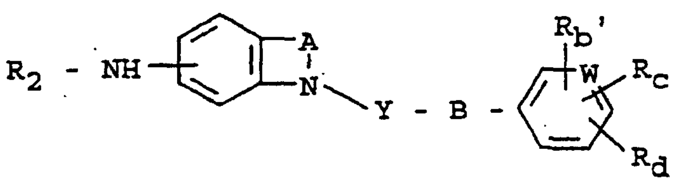

, (XV)

où
A, B, W, Y, $R_2$, $R_c$ et $R_d$ sont définis comme dans les revendications 1 à 6 et
$R_b$' représente un groupe cyano ou un groupe amidino substitué par un groupe alcoxy en $C_{1-10}$-carbonyle ou phényl-alcoxy en $C_{1-3}$-carbonyle, est mis à réagir avec un composé de formule générale

$$Z_7\text{-}R_1' \qquad (XVI)$$

où
$R_1'$ est défini comme indiqué précédemment et
$Z_7$ représente un groupe partant ou représente un atome d'oxygène avec un atome d'hydrogène de l'atome de carbone voisin, ou
k) pour la production d'un composé de formule générale I où $R_b$ représente un groupe cyano et Y représente un groupe carbonyle, un composé de formule générale

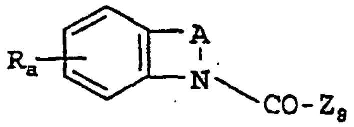
, (XVII)

où
A et $R_a$ sont définis comme dans les revendications 1 à 6 et $Z_8$ représente un groupe partant, est mis à réagir avec un composé de formule générale

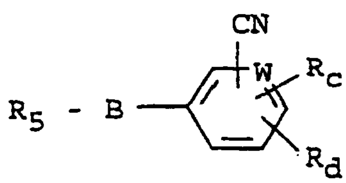
, (XVIII)

où
B, W, $R_c$ et $R_d$ sont définis comme dans les revendications 1 à 6 et
$R_5$ représente un groupe amino éventuellement substitué par un groupe alkyle en $C_{1-3}$, et

si nécessaire, un reste protecteur utilisé pendant les réactions pour la protection de groupes réactifs est clivé et/ou si on le souhaite, un composé de formule générale I ainsi obtenu, où X représente un groupe carbonyle, est converti en un composé thiocarbonylé correspondant au moyen d'un agent introduisant du soufre et/ou
un composé de formule générale I ainsi obtenu, où $R_a$ contient un reste acyle, est converti par hydrolyse en un composé de formule générale I dans laquelle $R_a$ représente un groupe $R_1NH$ ou dans laquelle $R_a$ contient un groupe carboxyle et/ou
un composé ainsi obtenu de formule générale I où $R_a$ représente ou contient un groupe carboxyle ou acide sulfonique est converti par amidation en un composé amide de formule générale I correspondant et/ou
si on le souhaite, un composé de formule générale I ainsi obtenu est résolu en ses stéréoisomères et/ou
un composé de formule générale I ainsi obtenu est converti en ses sels, en particulier pour l'utilisation pharmaceutique en ses sels physiologiquement acceptables avec un acide ou une base inorganique ou organique.